# EUROPEAN PATENT APPLICATION

(11) **EP 4 578 876 A1**
(43) Date of publication of application: **02.07.2025**
(21) Application number: 23857336.4
(22) Date of filing: 22.08.2023
(51) Int. Cl.: C07K 16/28, A61K 39/395, A61P 35/00, C07K 16/46, C12N 15/13

(54) **BISPECIFIC ANTIBODY**

(30) Priority: 23.08.2022 JP 2022132264
(71) Applicant: ONO Pharmaceutical Co., Ltd., Osaka-shi, Osaka 541-8526 (JP)
(72) Inventor: SHIBAYAMA, Shiro, Tsukuba-shi, Ibaraki 300-4247 (JP); ARIMA, Hiroshi, Tsukuba-shi, Ibaraki 300-4247 (JP); SAITO, Akina, Tsukuba-shi, Ibaraki 300-4247 (JP); NAKAMURA, Masatoshi, Tsukuba-shi, Ibaraki 300-4247 (JP); GUNDE, Tea, 8810 Horgen (CH); HESS, Christian, 8810 Horgen (CH); SIMONIN, Alexandre, 8810 Horgen (CH); WARMUTH, Stefan, 8810 Horgen (CH); JOHANSSON, Maria, 8810 Horgen (CH); BROCK, Matthias, 8810 Horgen (CH); WEINERT, Christopher, 8810 Horgen (CH); SPIGA, Fabio Mario, 8810 Horgen (CH)
(74) Representative: Grünecker Patent- und Rechtsanwälte PartG mbB
(86) International application number: PCT/JP2023/030111
(87) International publication number: WO 2024/043227

(57) **Abstract**

The present invention addresses the problem of providing a novel drug for preventing cancer, suppressing the progression of a symptom of cancer, suppressing the recurrence of cancer, or treating cancer.

As the results of intensive studies, the present inventors focused attention on Allergin-1 that is a target molecule involved in the regulation of cancer immunity, and found a bispecific antibody capable of binding specifically respectively to two different epitopes on the S1 and S2 domains, said domains being the extracellular domains of Allergin-1, as a substance that can solve this problem. The present inventors confirmed that this antibody is useful for preventing cancer, suppressing the progression of a symptom of cancer, suppressing the recurrence of cancer, or treating cancer.

## Description

### TECHNICAL FIELD

The present invention relates to a bispecific antibody to Allergin-1 or an antibody fragment thereof, and a pharmaceutical composition containing the bispecific antibody as an active ingredient, as well as pharmaceutical uses thereof.

### BACKGROUND ART

Cancer immunotherapy is a therapy for suppressing or treating the progression of cancer by strengthening immunity against cancer by acting on the immune surveillance mechanism inherent in cancer patients, unlike conventional treatments such as surgery, radiation therapy and drug therapy using anti-cancer drugs or molecular targeting drugs. Recent researches on cancer immunity have revealed that the progression of cancer is related to the immunosuppressive environment, mainly in local area of cancer, and that cancer itself utilizes a system to evade the immune surveillance mechanism. So-called immune checkpoint molecules, such as CTLA-4 and PD-1 or its ligand PD-L1, are known to be used in such evasion systems, and these inhibitors have already achieved remarkable results in clinical practice.

However, it is a fact that there are still cancer patients for whom these immune checkpoint inhibitors do not produce sufficient therapeutic effects, and there is an urgent need to provide new therapeutic methods for these cancer patients and to elucidate the functions of target molecules which are essential for establishing them.

Allergin-1 is a membrane-type receptor with ITIM domains in its intracellular region and an immunoglobulin-like structure in its extracellular region, and is strongly expressed in mast cells. It is known to function as a molecule suppressing allergic reactions, such as suppressing anaphylaxis by suppressing degranulation mediated by IgE receptor signaling and suppressing asthmatic reactions induced by mite antigens, based on analyses using mice deficient in the same molecular (non-patent literatures 1, 2 and 3).

On the other hand, Allergin-1 is also involved in cancer immunity, and cancer treatment by its inhibition has been reported (patent literature 1).

### CITATION LIST

### PATENT LITERATURE

Patent Literature 1: WO2017/038997

### NON-PATENT LITERATURE

Non-Patent Literature 1: Nature Immunology, 2010, Vol. 11, No. 7, p.601-608
Non-Patent Literature 2: PLOS ONE, 2013, Vol. 8, No. 10, e76160
Non-Patent Literature 3: Allergy, 2011, Vol. 18, No. 4, p.506-514

### SUMMARY OF INVENTION

### TECHNICAL PROBLEM

The problem of the present invention is to provide a novel drug enhancing cancer immunity which is effective in cancer therapy.

### SOLUTION TO PROBLEM

The inventors of the present invention, as a result of intensive investigation, found that a bispecific antibody capable of specifically binding to two different epitopes on extracellular domain of Allergin-1 (hereinafter, may be abbreviated as a "bispecific antibody to Allergin-1" or an "anti-Allergin-1 bispecific antibody"), respectively, resolves the above problem, and completed the present invention.

In other words, the present invention mainly provides the following embodiments:
<1> a bispecific antibody or an antibody fragment thereof having antigen-binding sites specifically binding to two different epitopes on extracellular domain of Allergin-1 (hereinafter, may be collectively abbreviated as an "anti-Allergin-1 bispecific antibody or the like"), respectively,
<2> a pharmaceutical composition containing the bispecific antibody to Allergin-1 as an active ingredient;
<3> an agent for suppressing the progression of symptoms of, suppressing the recurrence of, and/or treating cancer, comprising the bispecific antibody to Allergin-1 as an active ingredient,
<4> an antibody or an antibody fragment thereof specifically binding to the S1 domain of Allergin-1, and an antibody or an antibody fragment thereof specifically binding to the S2 domain of Allergin-1, and
<5> polynucleotides coding the heavy chain and light chain of the bispecific antibody described in the preceding item <1>, respectively.

### ADVANTAGE EFFECTS OF INVENTION

The present invention provides the anti-Allergin-1 bispecific antibody for suppressing the progression of symptoms of, suppressing the recurrence of and/or treating cancer.

### BRIEF DESCRIPTION OF DRAWINGS

[Figure 1] It shows the correspondence relations between the respective amino acid sequences of the heavy chain variable region (hereinafter, may be abbreviated as "VH") and light chain variable region (hereinafter, may be abbreviated as "VL") constituting the S1-A binding site and SEQ ID Nos thereof. As it will be clear to those skilled in the art, alphabets in the figure represent one-letter abbreviation corresponding to each amino acid.
[Figure 2] It shows the correspondence relations between the respective amino acid sequences of the VH and VL constituting the S1-B binding site and SEQ ID Nos thereof.
[Figure 3] It shows the correspondence relations between the respective amino acid sequences of multiple CDRs contained in the VH and VL constituting the S1-A binding site, respectively, and SEQ ID Nos thereof.
[Figure 4] It shows the correspondence relations between the respective amino acid sequences of multiple CDRs contained in the VH and VL constituting the S1-B binding site, respectively, and SEQ ID Nos thereof.
[Figure 5] It shows the correspondence relations between the respective amino acid sequences of the VH and VL constituting the S2-C binding site and the S2-D binding site, respectively, and SEQ ID Nos thereof.
[Figure 6] It shows the correspondence relations between the respective amino acid sequences of multiple CDRs contained in the VH and VL, respectively, constituting the S2-C binding site and the S2-D binding site, respectively, and SEQ ID Nos thereof.
[Figure 7] It shows the Morrison form of the anti-Allergin-1 bispecific antibody of the present invention.
[Figure 8] It shows the correspondence relations between the respective amino acid sequences of the heavy chain constant region and the light chain constant region constructing the anti-Allergin-1 bispecific antibody of the present invention and SEQ ID Nos thereof.

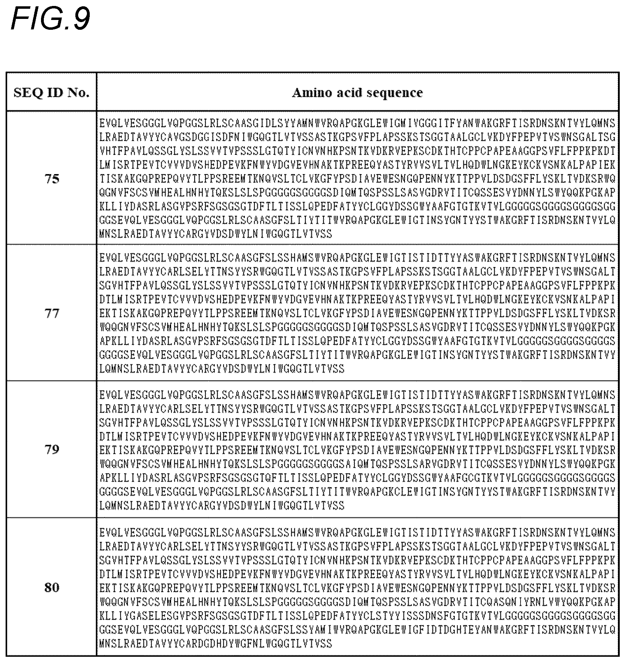

[Figure 9] It shows the correspondence relations between the respective amino acid sequences of the heavy chains constructing the anti-Allergin-1 bispecific antibody of the present invention and SEQ ID Nos thereof.

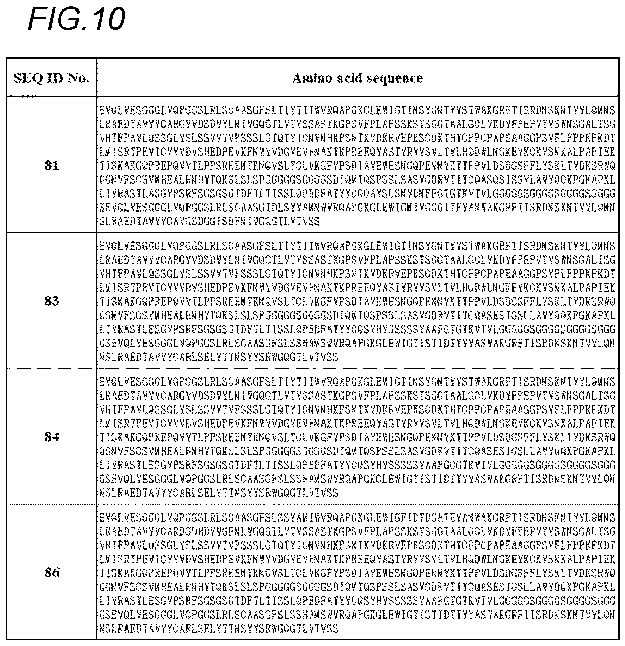

[Figure 10] It shows the correspondence relations between the respective amino acid sequences of the heavy chains constructing the anti-Allergin-1 bispecific antibody of the present invention and SEQ ID Nos thereof.

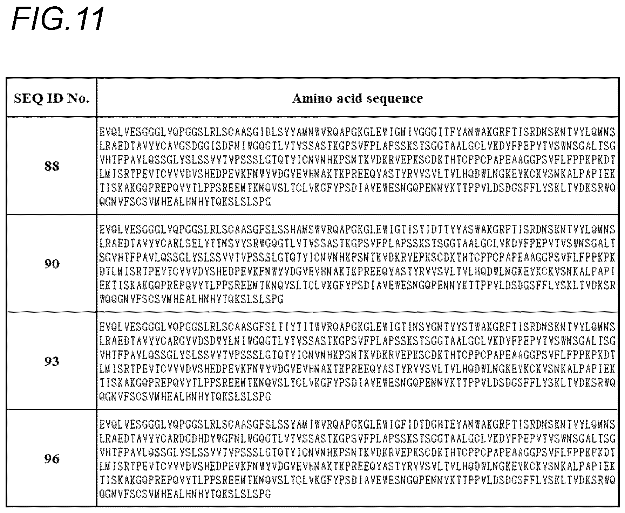

[Figure 11] It shows the correspondence relations between the respective amino acid sequences of the heavy chains constructing the anti-Allergin-1 bispecific antibody of the present invention and SEQ ID Nos thereof.

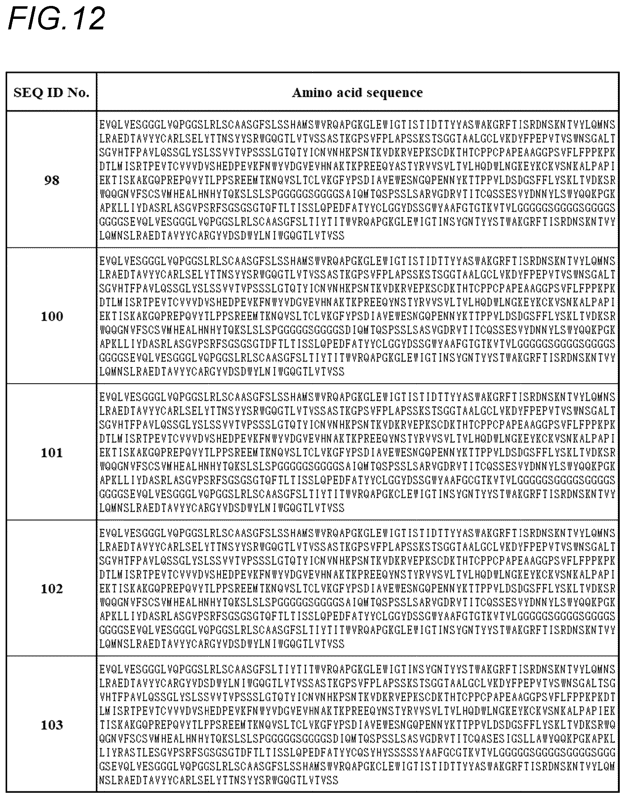

[Figure 12] It shows the correspondence relations between the respective amino acid sequences of the heavy chains constructing the anti-Allergin-1 bispecific antibody of the present invention and SEQ ID Nos thereof.

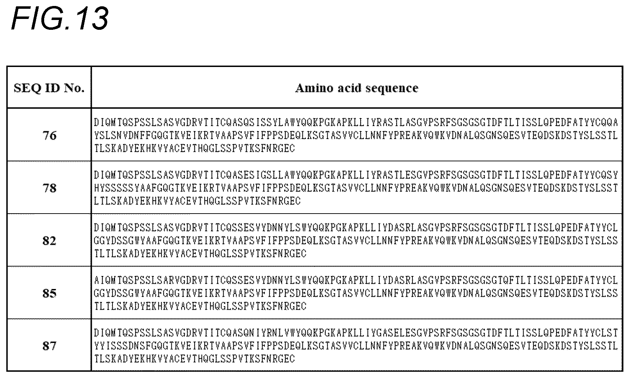

[Figure 13] It shows the correspondence relations between the respective amino acid sequences of the light chains constructing the anti-Allergin-1 bispecific antibody of the present invention and SEQ ID Nos thereof.

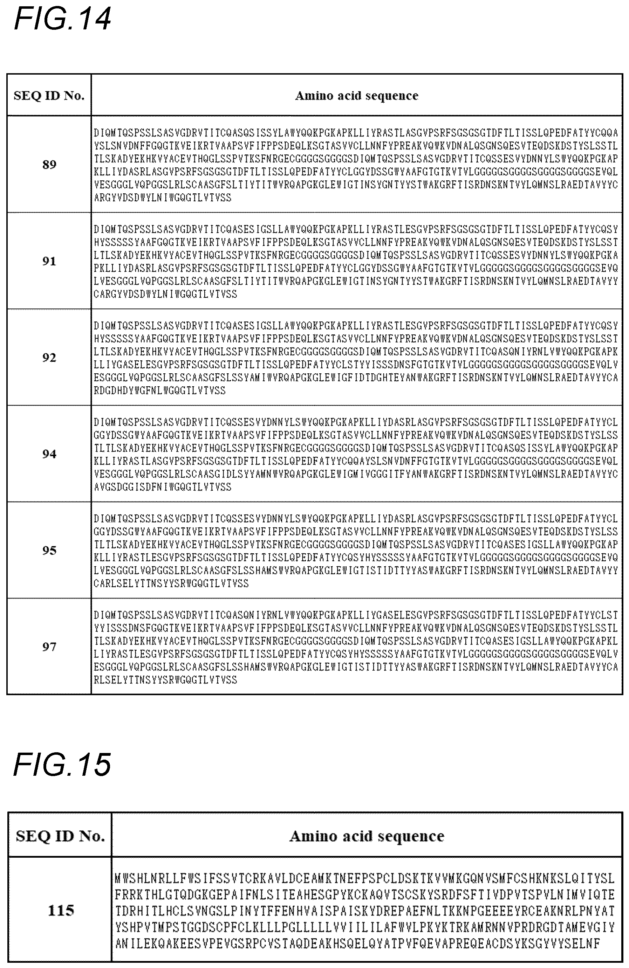

[Figure 14] It shows the correspondence relations between the respective amino acid sequences of the light chains constructing the anti-Allergin-1 bispecific antibody of the present invention and SEQ ID Nos thereof.
[Figure 15] It shows the amino acid sequences of human Allergin-1L (GenBank Registered Number: NP_001078892.1).

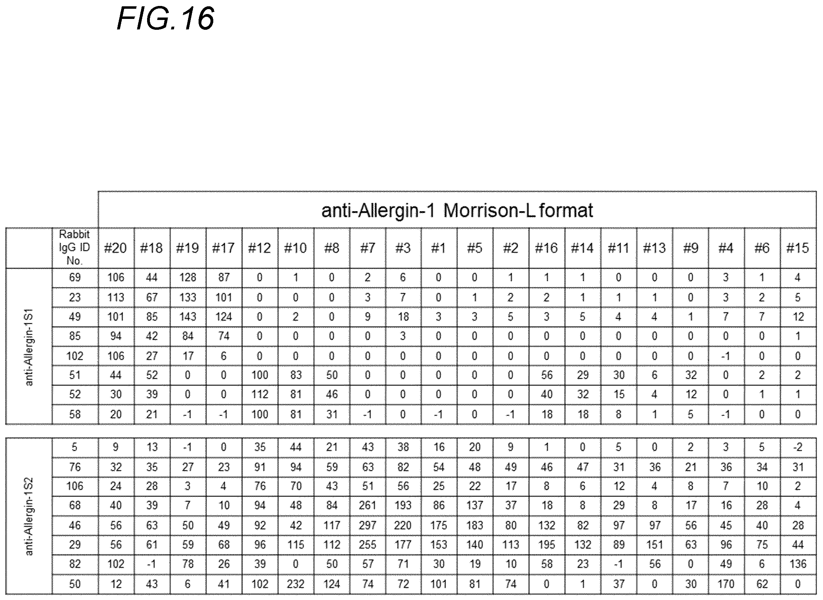

[Figure 16] It shows results of competitive assay representing the shielding degree by binding of the anti-Allergin-1 bispecific antibody of the present invention to Allergin-1. The multiple numbers in the table represent the binding rates of the anti-Allergin-1 antibody to Allergin-1L in the present of the anti-Allergin-1 bispecific antibody of the present invention to that of the same anti-Allergin-1 antibody to Allergin-1L in the absence of the same anti-Allergin-1 bispecific antibody, respectively. The serial numbers in the vertical column of "Rabbit IgG ID No." represent clones of anti-Allergin-1 antibody used in evaluation, respectively, "anti-Allergin-1S1" indicates that the anti-Allergin-1 antibody is specific for the S1 domain, and "anti-Allergin-1S2" indicates that the antibody is specific for the S2 domain. The serial numbers in the horizontal columns to the right represent the respective clones of the evaluated anti-Allergin-1 bispecific antibody.
[Figure 17] It shows results of evaluation on binding of anti-Allergin-1 bispecific antibody #15_h_opt clone to the S1 domain and S2 domain of Allergin-1. The "referenced OD_{450-690 nm}" on the vertical axis represents the binding amount of anti-Allergin-1 bispecific antibody to Allergin-1L and the horizontal axis represents the concentration of added rabbit anti-Allergin-1 antibody. In the figure, "rabbit IgG #50" and "rabbit IgG #85" represent rabbit anti-Allergin-1S2 IgG antibody #50 clone and rabbit anti-Allergin-1S1 IgG antibody #85 clone, respectively.
[Figure 18] It shows results of Biacore analysis regarding the inhibitory effect of anti-Allergin-1 bispecific antibody #15_h_opt clone against binding of TLR9 ligand ODN M362 to Allergin-1. The "Response (RU)" on the vertical axis represents the binding amount of ODN M362 to Allergin-1L.
[Figure 19] It shows the respective results of the anti-tumor effects of anti-Allergin-1 bispecific antibodies (#11_L, #13_L, #15_L, #16_L and #15_h) of the present invention in combination with anti-mouse PD-1 antibody 4H2 in human Allergin-1L-knock-in C57BL/6 mice bearing tumor (mouse colorectal cancer cell line MC38). The vertical axis represents tumor volume, and the horizontal axis represents the days since transplantation of the cancer cell line.
[Figure 20] It shows a result of the anti-tumor effect of anti-Allergin-1 bispecific antibody #15_h of the present invention in combination with anti-mouse PD-1 antibody 4H2 in human Allergin-1L-knock-in C57BL/6 mice bearing tumor (mouse lung cancer cell line 3LL). The vertical axis represents tumor volume, and the horizontal axis represents the days since transplantation of the cancer cell line.
[Figure 21] It shows a result of the antitumor effect of only anti-Allergin-1 bispecific antibody #15_h_opt clone of the present invention in the human Allergin-1L-knock-in C57BL/6 mice bearing tumor (human PD-L1-forced expressing mouse colorectal cancer cell line MC38). The vertical axis represents tumor volume, and the horizontal axis represents the days since transplantation of the cancer cell line.
[Figure 22] It shows a result of evaluation on the effect of the anti-Allergin-1 bispecific antibody of the present invention on the release of cytokines from human peripheral blood mononuclear cells.

### DESCRIPTION OF EMBODIMENTS

Allergin-1, also called as MILR1, and in human, there are at least three splicing variants, Allergin-1L, Allergin-1S1 and Allergin-1S2 (see Nature Immunology (2010), Vol. 11, No. 7, p. 601-608), each of which is a membrane-type protein consisting of the amino acid sequence identified by GenBank Reg. Nos. NP_001078892.1 (SEQ ID No. 115), BAJ08252.1 and BAJ08253.1, respectively. Its mouse homologue is also called as MCA32, which has the amino acid sequence identified by GenBank Reg. No. BAJ08254.1. Herein, Allergin-1L has the S1 domain and the S2 domain in its extracellular domain, and Allergin-1S1 and Allergin-1S2 have only the S1 domain and only the S2 domain, respectively, in their extracellular domains. The S1 domain of Allergin-1L is located in the region covering position 37 to 121 from its N-terminus, and the S2 domain is located in the region covering position 128 to 200 from its N-terminus, while the S1 domain of Allergin-1S1 is located in the region covering position 37 to 121 from its N-terminus, as well, and the S2 domain of Allergin-1S2 is located in the region covering position 38 to 110 from its N-terminus. In the present specification, the term "Allergin-1", unless otherwise limited, may be used as those including all of splicing variants or isoforms thereof. In the present invention, Allergin-1 is preferably human Allergin-1.

In the present specification, the term "isolate" means becoming a single substantially pure component by being identified, separated and/or purified from impurities containing a plurality of or myriad number of components extracted from host cells.

In the present specification, the term "monoclonal antibody" means an antibody obtained from a substantially homogeneous antibody group binding to the same specific antigen.

In the present specification, the term "bispecific antibody" means an antibody having the binding specificity to two different antigen molecules or epitopes on one molecule.

In the present specification, the term "antigen molecule" or "antigen" means a chemical substance, peptide or protein which induces the body's immune responses and is recognized by antibodies produced as a part of such immune responses, and the antigen in the present invention corresponds to the extracellular domain of Allergin-1 or a portion thereof.

In the present specification, the term "epitope" means a part on antigen which is recognized as the binding site when an antibody binds to its antigen, and the epitope in the present invention is a portion of the extracellular domain of Allergin-1 to which each antigen-binding site in the bispecific antibody or antibody fragment thereof of the present invention specifically binds.

In the present specification, the term "antigen-binding site" means the smallest unit comprising a portion of antibody capable of binding to antigen or epitope thereof, which is usually formed by association of the heavy chain and light chain having the VH and VL of antibody constituting it, respectively. The VH and VL consist of (1) three complementarity determining regions (hereinafter, may be abbreviated as "CDR")(complementarity determining region 1 in the VH (hereinafter, may be abbreviated as "VH-CDR1"), complementarity determining region 2 in the VH (hereinafter, may be abbreviated as "VH-CDR2") and complementarity determining region 3 in the VH (hereinafter, may be abbreviated as "VH-CDR3"), and complementarity determining region 1 in the VL (hereinafter, may be abbreviated as "VL-CDR1"), complementarity-determining region 2 in the VL (hereinafter, may be abbreviated as "VL-CDR2") and complementarity-determining region 3 in the VL (hereinafter, may be abbreviated as "VL-CDR3")) and (2) framework regions (hereinafter, may be abbreviated as "FW"), which arrange those CDRs so that a target antigen or epitope can be recognized by combination of those CDRs, respectively.

In the present specification, the term "complex of heavy chain/light chain" means a complex consisting of a heavy chain of antibody and a light chain corresponding thereto, herein the term "corresponding" means the correspondence between the heavy chain and light chain in a relationship that its VH and VL form an antigen binding site.

In the present specification, the term "scFv" is an abbreviation for single chain Fv, which is a low molecular weight antibody modified to be produced in a single chain form so that the VH and VL can form an antigen binding site.

In the present specification, the term "cross-competition" means mutually inhibiting or suppressing the binding to an epitope which is commonly recognized by multiple antibodies, regardless of the degree of binding. Cross-competition can be assessed by competitive binding assays, but there are several evaluation methods using Biacore analysis, ELISA assays, flow cytometry, enzyme-linked immunosorbent assay (ELISA), fluorescence energy transfer assay (FRET), fluorescence microscopy technology (FMAT) and the like.

### [Antigen binding sites of anti-Allergin-1 bispecific antibody]

The anti-Allergin-1 bispecific antibody of the present invention is a bispecific antibody having antigen-binding sites specifically binding to two different epitopes on the extracellular domains of Allergin-1, respectively. Herein, the two antigen-binding sites do not mutually compete for Allergin-1, and are selected from any two different ones among four types consisting of:
(a) an antigen-binding site specifically binding to a specific epitope on the S1 domain of Allergin-1 (in the present specification, may be abbreviated as "S1-A binding site"),
(b) an antigen-binding site specifically binding to another epitope on the S1 domain of Allergin-1, different from the epitope on the S1 domain described in the preceding item (a) (in the present specification, may be abbreviated as "S1-B binding site"),
(c) an antigen-binding site specifically binding to a specific epitope on the S2 domain of Allergin-1 (in the present specification, may be abbreviated as "S2-C binding site"), and
(d) an antigen-binding site specifically binding to another epitope on the S2 domain of Allergin-1, different from the epitope on the S2 domain described in the preceding item (c) (in the present specification, may be abbreviated as "S2-D binding site"). In other words, the S1-A binding site, the S1-B binding site, the S2-C binding site and the S2-D binding site all are antigen binding sites which do not mutually compete for Allergin-1.

Herein, the epitope to which the S1-A binding site specifically binds (hereinafter, may be abbreviated as "S1-A epitope") is constituted by at least any one or more, or all of phenylalanine at position 70, arginine at position 71, arginine at position 72, lysine at position 73, histidine at position 75, leucine at position 76, histidine at position 96, glutamic acid at position 97, lysine at position 102, lysine at position 104 and tyrosine at position 113 in Allergin-1 comprising the amino acid sequence set forth in SEQ ID No. 115, and the epitope to which the S2-D binding site specifically binds (hereinafter, may be abbreviated as "S2-D epitope") is constituted by at least any one or more, or all of isoleucine at position 132, methionine at position 133, valine at position 134, isoleucine at position 135, glutamine at position 136, threonine at position 137, glutamic acid at position 138, histidine at position 146 and proline at position 215 in the same Allergin-1. On the other hand, the epitope to which the S1-B binding site specifically binds (hereinafter, may be abbreviated as "S1-B epitope") is an epitope different from the S1-A epitope represented by at least the amino acids above, and the epitope to which the S2-C binding site specifically binds (hereinafter, may be abbreviated as "S2-C epitope") is an epitope different from the S2-D epitope represented by at least the amino acids above. The term "specifically binding" means a feature that it can directly bind to the S1 domain or S2 domain of Allergin-1 with the binding activity of at least 1 x 10⁻⁵ M, preferably 1 x 10⁻⁷ M and more preferably more than 1 x 10⁻⁹ M affinity (dissociation constant (Kd value)), and does not substantially bind to other domains of Allergin-1 and any other proteins, and the term "not mutually compete" means, for example, that the S1-B binding site does not substantially inhibit the binding of the S1-A binding site to epitopes thereof or that the S1-A binding site does not substantially inhibit the binding of the S1-B binding site to epitopes thereof, or that the S2-D binding site does not substantially inhibit the binding of the S2-C binding site to epitopes thereof or that the S2-C binding site does not substantially inhibit the binding of the S2-D binding site to epitopes thereof.

### [S1-A binding site and S1-B binding site]

Examples of the respective embodiments of the "S 1-A binding site" and "S1-B binding site" of the present invention include, as to the S1-A binding site,
(A1) an antigen-binding site comprising the VH having
   (a) the VH-CDR1 comprising the amino acid sequence set forth in SEQ ID No. 25,
   (b) the VH-CDR2 comprising the amino acid sequence set forth in SEQ ID No. 26, and
   (c) the VH-CDR3 comprising the amino acid sequence set forth in SEQ ID No. 27, and
      the VL having
   (d) the VL-CDR1 comprising the amino acid sequence set forth in SEQ ID No. 49,
   (e) the VL-CDR2 comprising the amino acid sequence set forth in SEQ ID No. 50, and
   (f) the VL-CDR3 comprising the amino acid sequence set forth in SEQ ID No. 51,
(A2) an antigen-binding site comprising the VH having
   (a) the VH-CDR1 comprising the amino acid sequence set forth in SEQ ID No. 28,
   (b) the VH-CDR2 comprising the amino acid sequence set forth in SEQ ID No. 29, and
   (c) the VH-CDR3 comprising the amino acid sequence set forth in SEQ ID No. 30, and
   the VL having:
   (d) the VL-CDR1 comprising the amino acid sequence set forth in SEQ ID No. 52,
   (e) the VL-CDR2 comprising the amino acid sequence set forth in SEQ ID No. 53, and
   (f) the VL-CDR3 comprising the amino acid sequence set forth in SEQ ID No. 54,
(A3) an antigen-binding site comprising the VH having
   (a) the VH-CDR1 comprising the amino acid sequence set forth in SEQ ID No. 31,
   (b) the VH-CDR2 comprising the amino acid sequence set forth in SEQ ID No. 32, and
   (c) the VH-CDR3 comprising the amino acid sequence set forth in SEQ ID No. 33, and
   the VL having:
   (d) the VL-CDR1 comprising the amino acid sequence set forth in SEQ ID No. 55,
   (e) the VL-CDR2 comprising the amino acid sequence set forth in SEQ ID No. 56, and
   (f) the VL-CDR3 comprising the amino acid sequence set forth in SEQ ID No. 57, and
(A4) an antigen-binding site comprising the VH having
   (a) the VH-CDR1 comprising the amino acid sequence set forth in SEQ ID No. 34,
   (b) the VH-CDR2 comprising the amino acid sequence set forth in SEQ ID No. 35, and
   (c) the VH-CDR3 comprising the amino acid sequence set forth in SEQ ID No. 36, and
   the VL having:
   (d) the VL-CDR1 comprising the amino acid sequence set forth in SEQ ID No. 58,
   (e) the VL-CDR2 comprising the amino acid sequence set forth in SEQ ID No. 59, and
   (f) the VL-CDR3 comprising the amino acid sequence set forth in SEQ ID No. 60, and
on the other hand, as to the S1-B binding site,
(B1) an antigen-binding site comprising the VH having
   (a) the VH-CDR1 comprising the amino acid sequence set forth in SEQ ID No. 37,
   (b) the VH-CDR2 comprising the amino acid sequence set forth in SEQ ID No. 38, and
   (c) the VH-CDR3 comprising the amino acid sequence set forth in SEQ ID No. 39, and
   the VL having:
   (d) the VL-CDR1 comprising the amino acid sequence set forth in SEQ ID No. 61,
   (e) the VL-CDR2 comprising the amino acid sequence set forth in SEQ ID No. 62, and
   (f) the VL-CDR3 comprising the amino acid sequence set forth in SEQ ID No. 63, and
(B2) an antigen-binding site comprising the VH having
   (a) the VH-CDR1 comprising the amino acid sequence set forth in SEQ ID No. 40,
   (b) the VH-CDR2 comprising the amino acid sequence set forth in SEQ ID No. 41, and
   (c) the VH-CDR3 comprising the amino acid sequence set forth in SEQ ID No. 42, and
   the VL having:
   (d) the VL-CDR1 comprising the amino acid sequence set forth in SEQ ID No. 64,
   (e) the VL-CDR2 comprising the amino acid sequence set forth in SEQ ID No. 65, and
   (f) the VL-CDR3 comprising the amino acid sequence set forth in SEQ ID No. 66.

Furthermore, the respective examples of the "S1-A binding site" and "S1-B binding site" of the present invention include those of which one to five arbitrary amino acid residues are substituted with other amino acids (preferably, conservative amino acids thereof) in each CDR constituting the respective VH and VL in the above-mentioned (A1) to (A4) and (B1) and (B2), respectively, and which have substantially the same binding activity as that of each original antigen-binding site without any substitutions with the same amino acids to each epitope on the S1 domain. Examples thereof include those of which one amino acid residue in the VH-CDR1 of the S1-A binding site is substituted with a conservative amino acid thereof, and those of which one or two amino acid residues in the VH-CDR2 or VH-CDR3 were substituted with conservative amino acids thereof. Herein, the substitution with a "conservative amino acid" means the exchangeability with a residue having a similar side-chain. For example, a group of amino acids having an aliphatic side-chain includes glycine, alanine, valine, leucine, and isoleucine, a group of amino acids having an aliphatic hydroxyl side-chain includes serine and threonine, a group of amino acids having a side-chain containing amide includes asparagine and glutamine, a group of amino acids having an aromatic side-chain includes phenylalanine, tyrosine and tryptophan, a group of amino acids having a basic side-chain includes lysine, arginine and histidine, and a group of amino acids having a sulfur-containing side-chain includes cysteine and methionine. Preferable examples of the substitution with a conservative amino acid include substitutions among valine, leucine and isoleucine, substitutions between phenylalanine and tyrosine, substitutions between lysine and arginine, substitutions between alanine and valine, as well as substitutions between asparagine and glutamine. Herein, the sentence "which have substantially the same binding activity as that of each original antigen-binding site without any substitutions with the same amino acids to each epitope on the S1 domain" mentioned above means that the binding activity to each epitope on the S1 domain of the S1-A binding site or S1-B binding site substituted with the same amino acids is 95% or more, preferably 98% or more, and more preferably 99% or more to that of the original S1-A binding site or S1-B binding site without any substitutions with the same amino acids.

Furthermore, the respective examples of the "S1-A binding site" and "S1-B binding site" of the present invention include those which contain the respective CDRs having the above-mentioned specific amino acid sequences in the VH and VL constituting them, respectively, and of which the FW is humanized so that it is encoded by a human specific germ-line gene or gene thereof with one or more somatic mutations and/or back mutations. For example, the FWs encoded by human germ-line VH gene IGHV3-66 and VL gene IGKV1-39 or genes thereof with one or more somatic mutations and/or back mutations, respectively, can be applied to each CDR in the VH and VL represented by the above-mentioned (A1) to (A4) and (B1) and (B2), respectively. Herein, the "somatic mutation" means a mutation in an antibody gene introduced by the action of AID (activation-induced cytidine deaminase), and the "back mutation" means the replacement of one or more amino acid residues in the FW of a humanized antibody with the corresponding amino acid residue in the original non-human (e.g. rabbit or mouse) antibody. Examples of the S1-A binding site having the VH and VL humanized like that, respectively, include the antigen-binding site constituted by the VH and VL comprising any one pair of the amino acid sequences selected from
(a) SEQ ID No. 1 and SEQ ID No. 12 or 104,
(b) SEQ ID No. 2 and SEQ ID No. 13 or 105,
(c) SEQ ID No. 3 and SEQ ID No. 14 or 106,
(d) SEQ ID No. 4 and SEQ ID No. 15 or 107,
(e) SEQ ID No. 5 and SEQ ID No. 16 or 108, and
(f) SEQ ID No. 6 and SEQ ID No. 17 or 109, and examples of the S1-B binding site include the antigen-binding site constituted by the VH and VL comprising a pair of the amino acid sequences set forth in
   (a) SEQ ID No. 7 and SEQ ID No. 18 or 110 or
   (b) SEQ ID No. 8 and SEQ ID No. 19 or 111.

Furthermore, examples of the "S1-A binding site" of the present invention also include those which have a VH and VL comprising the respective amino acid sequences having at least 80%, preferably at least 90%, more preferably at least 95%, furthermore preferably at least 98%, or further more preferably at least 99% identity, each independently, to any one pair of the amino acid sequences (excluding three CDRs, respectively) selected from
(a) SEQ ID No. 1 and SEQ ID No. 12 or 104,
(b) SEQ ID No. 2 and SEQ ID No. 13 or 105,
(c) SEQ ID No. 3 and SEQ ID No. 14 or 106,
(d) SEQ ID No. 4 and SEQ ID No. 15 or 107,
(e) SEQ ID No. 5 and SEQ ID No. 16 or 108, and
(f) SEQ ID No. 6 and SEQ ID No. 17 or 109, and in which the differences from the respective amino acid sequences of the original VH and VL have no substantial effect on the binding activity to the same epitope on the S1 domain (hereinafter, may be abbreviated as "homologous S1-A binding site"). Examples of the homologous S1-A binding sites include the S1-A binding site having the VH in which the respective cysteines at position 45 or 44 (corresponding to position 44 according to the Kabat numbering system) in the VH comprising the amino acid sequence set forth in SEQ ID No. 1 or 5 was substituted by glycine, and the S1-A binding site having the VH in which glycine at position 44 (corresponding to position 44 according to the Kabat numbering system) in the VH comprising the amino acid sequence selected from SEQ ID No. 2, 3, 4 and 6 was substituted by cysteine. Herein, the term "% identity" used in comparison of the identity of amino acid sequences is defined as the percentage of the amino acid sequence identical to the reference amino acid sequence (herein, the reference amino acid sequence in which the gap has been introduced when being needed to maximize the sequence identity) when two sequences are aligned. Furthermore, herein, the sentence "the differences from the respective amino acid sequences of the original VH and VL have no substantial effect on the binding activity to the same epitope on the S1 domain" means that the binding activity to the same epitope on the S1 domain of the homologous S1-A binding site is 95% or more, preferably 98% or more, and more preferably 99% or more to that of the S1-A binding site comprising the original VH and VL.

Examples of the "S1-B binding site" of the present invention also include those which have a VH and VL comprising the respective amino acid sequences having at least 80%, preferably at least 90%, more preferably at least 95%, furthermore preferably at least 98%, or further more preferably at least 99% identity, each independently, to a pair of the amino acid sequences (excluding three CDRs, respectively) set forth in
(a) SEQ ID No. 7 and SEQ ID No. 18 or 110, or
(b) SEQ ID No. 8 and SEQ ID No. 19 or 111, and in which the differences from the respective amino acid sequences of the original VH and VL have no substantial effect on the binding activity to the same epitope on the S1 domain (hereinafter, may be abbreviated as "homologous S1-B binding site"). Examples of the homologous S1-B binding sites include the S1-B binding site having the VH in which cysteine at position 44 in the VH comprising the amino acid sequence set forth in SEQ ID No. 7 (corresponding to position 44 according to the Kabat numbering system) was substituted by glycine, and the S1-B binding site having the VH in which glycine at position 44 in the VH comprising the amino acid sequence set forth in SEQ ID No. 8 (corresponding to position 44 according to the Kabat numbering system) was substituted by cysteine.

In the present invention, the VH comprising the amino acid sequence set forth in SEQ ID No. 1 or 5 and the VL comprising the amino acid sequence selected from SEQ ID No. 12 to 17 preferably constitute scFvs, respectively, and the VH comprising the amino acid sequence selected from SEQ ID No. 2, 3, 4 and 6, and the VL comprising the amino acid sequence selected from SEQ ID No. 104 to 109 preferably constitute parts of the heavy chain/light chain complex, respectively. And, in the present invention, the VH comprising the amino acid sequence set forth in SEQ ID No. 7 and the VL comprising the amino acid sequence set forth in SEQ ID No. 18 or 19 preferably constitute scFvs, respectively, whereas the VH comprising the amino acid sequence set forth in SEQ ID No. 8, and the VL comprising the amino acid sequence set forth in SEQ ID No. 110 or 111 preferably constitute parts of the heavy chain/light chain complex, respectively.

In a yet another embodiment, examples of the "S1-A binding site" of the present invention also include an antigen-binding site cross-competing for binding to the epitope on the S1 domain with an antibody or an antigen-binding site thereof having the VH and VL comprising any one pair of the amino acid sequences selected from
(a) SEQ ID No. 1 and SEQ ID No. 104,
(b) SEQ ID No. 2 and SEQ ID No. 105,
(c) SEQ ID No. 3 and SEQ ID No. 106,
(d) SEQ ID No. 4 and SEQ ID No. 107,
(e) SEQ ID No. 5 and SEQ ID No. 108, and
(f) SEQ ID No. 6 and SEQ ID No. 109, and
an antigen-binding site of which the binding to the same epitope on the S1 domain is cross-competed by an antibody or an antigen-binding site thereof having the VH and VL comprising any one pair of the amino acid sequences selected from
(a) SEQ ID No. 1 and SEQ ID No. 104,
(b) SEQ ID No. 2 and SEQ ID No. 105,
(c) SEQ ID No. 3 and SEQ ID No. 106,
(d) SEQ ID No. 4 and SEQ ID No. 107,
(e) SEQ ID No. 5 and SEQ ID No. 108, and
(f) SEQ ID No. 6 and SEQ ID No. 109.

Examples of the "S1-B binding site" of the present invention also include an antigen-binding site cross-competing for binding to the epitope on the S1 domain with an antibody or an antigen-binding site thereof having the VH and VL comprising a pair of the amino acid sequences set forth in
(a) SEQ ID No. 7 and SEQ ID No. 110, or
(b) SEQ ID No. 8 and SEQ ID No. 111, and
an antigen-binding site of which the binding to the same epitope on the S1 domain is cross-competed by an antibody or an antigen-binding site thereof having the VH and VL comprising a pair of the amino acid sequences set forth in
(a) SEQ ID No. 7 and SEQ ID No. 110, or
(b) SEQ ID No. 8 and SEQ ID No. 111.

Examples thereof cross-competing for binding to the same epitope on the S1 domain with the S1-A binding site having the VH and VL comprising the amino acid sequences set forth in SEQ ID No. 1 and SEQ ID No. 12 or 104, respectively, include an antigen-binding site having the VH and VL comprising any one pair of the amino acid sequences selected from:
(a) SEQ ID No. 2 and SEQ ID No. 13 or 105,
(b) SEQ ID No. 3 and SEQ ID No. 14 or 106,
(c) SEQ ID No. 4 and SEQ ID No. 15 or 107,
(d) SEQ ID No. 5 and SEQ ID No. 16 or 108, and
(e) SEQ ID No. 6 and SEQ ID No. 17 or 109.

Examples thereof cross-competing for binding to the same epitope on the S1 domain with the S1-B binding site having the VH and VL comprising the amino acid sequences set forth in SEQ ID No. 7 and SEQ ID No. 18 or 110, respectively, include an antigen-binding site having the VH and VL comprising the amino acid sequences set forth in SEQ ID No. 8 and SEQ ID No. 19 or 111, respectively.

Herein, the "S1-A binding site" of the present invention is preferably an antigen-binding site constituted by the VH and VL comprising any one pair of the amino acid sequences selected from:
(a) SEQ ID No. 1 and 12,
(b) SEQ ID No. 2 and 13,
(c) SEQ ID No. 3 and SEQ ID No. 14 or 106,
(d) SEQ ID No. 4 and SEQ ID No. 15 or 107,
(e) SEQ ID No. 5 and 16, and
(f) SEQ ID No. 6 and SEQ ID No. 17 or 109, more preferably an antigen-binding site constituted by the VH and VL comprising any one pair of the amino acid sequences selected from:
   (a) SEQ ID No. 3 and SEQ ID No. 14 or 106,
   (b) SEQ ID No. 4 and SEQ ID No. 15 or 107,
   (c) SEQ ID No. 5 and 16, and
   (d) SEQ ID No. 6 and SEQ ID No. 17 or 109, furthermore preferably an antigen-binding site constituted by the VH and VL comprising any one pair of the amino acid sequences selected from:
      (a) SEQ ID No. 3 and SEQ ID No. 14 or 106,
      (b) SEQ ID No. 4 and SEQ ID No. 15 or 107, and
      (c) SEQ ID No. 5 and 16, and
the "S1-B binding site" of the present invention is preferably an antigen-binding site constituted by the VH and VL comprising a pair of the amino acid sequences set forth in
(a) SEQ ID No. 7 and SEQ ID No. 18 or 110, or
(b) SEQ ID No. 8 and SEQ ID No. 19 or 111.

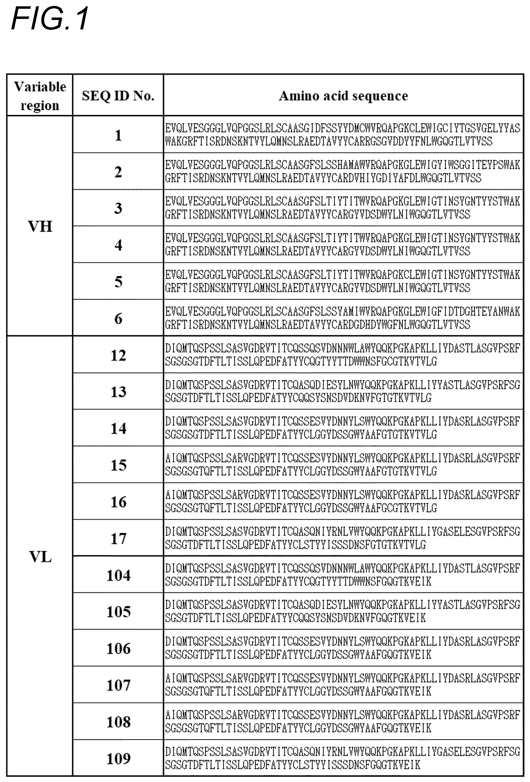

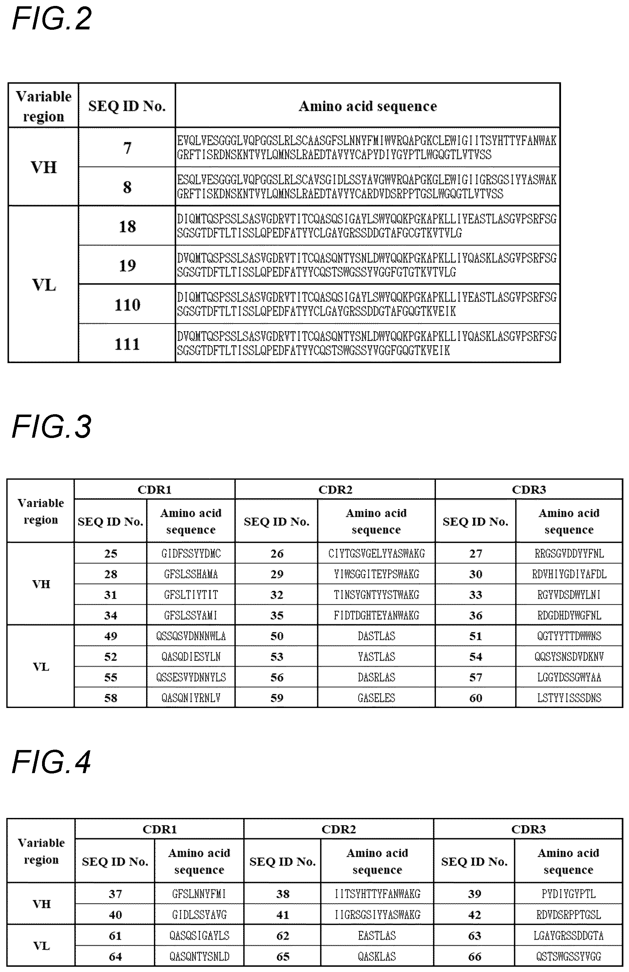

Herein, figure 1 and 2 show the correspondence relations between the respective amino acid sequences of the VH and VL constituting the S1-A binding site and the S1-B binding site, respectively, and SEQ ID Nos thereof, respectively. Furthermore, figure 3 and 4 show the correspondence relations between the amino acid sequences of multiple CDRs contained in those VH and VL, respectively, and SEQ ID Nos thereof, respectively.

### [S2-C binding site and S2-D binding site]

Examples of the respective embodiments of the "S2-C binding site" and "S2-D binding site" of the present invention include, as to the S2-C binding site,
(C1) an antigen-binding site comprising the VH having
   (a) the VH-CDR1 comprising the amino acid sequence set forth in SEQ ID No. 43,
   (b) the VH-CDR2 comprising the amino acid sequence set forth in SEQ ID No. 44, and
   (c) the VH-CDR3 comprising the amino acid sequence set forth in SEQ ID No. 45, and
   the VL having:
   (d) the VL-CDR1 comprising the amino acid sequence set forth in SEQ ID No. 67,
   (e) the VL-CDR2 comprising the amino acid sequence set forth in SEQ ID No. 68, and
   (f) the VL-CDR3 comprising the amino acid sequence set forth in SEQ ID No. 69, and
on the other hand, as to the S2-D binding site,
(D1) an antigen-binding site comprising the VH having
   (a) the VH-CDR1 comprising the amino acid sequence set forth in SEQ ID No. 46,
   (b) the VH-CDR2 comprising the amino acid sequence set forth in SEQ ID No. 47, and
   (c) the VH-CDR3 comprising the amino acid sequence set forth in SEQ ID No. 48, and
   the VL having:
   (d) the VL-CDR1 comprising the amino acid sequence set forth in SEQ ID No. 70,
   (e) the VL-CDR2 comprising the amino acid sequence set forth in SEQ ID No. 71, and
   (f) the VL-CDR3 comprising the amino acid sequence set forth in SEQ ID No. 72.

Furthermore, examples of the "S2-C binding site" and "S2-D binding site" of the present invention include those of which one to five arbitrary amino acid residues are substituted with other amino acids (preferably, conservative amino acids thereof) in each CDR constituting the respective VH and VL in the above-mentioned (C1) and (D1), respectively, and which have substantially the same binding activity as that of each original antigen-binding site without any substitutions with the same amino acids to each epitope on the S2 domain. Examples thereof include those of which one amino acid residue in the VH-CDR1 of the S2-C binding site is substituted with a conservative amino acid thereof, and those of which one or two amino acid residues in the VH-CDR2 or VH-CDR3 were substituted with conservative amino acids thereof. Herein, examples of substitutions with a "conservative amino acid" include those of amino acid substitutions in case of the "S1-A binding site" or "S1-B binding site". Furthermore, the sentence "which have substantially the same binding activity as that of each original antigen-binding site without any substitutions with the same amino acids to each epitope on the S2 domain" means, as well as mentioned above, that the binding activity to each epitope on the S2 domain of the S2-C binding site or S2-D binding site substituted with the same amino acids is 95% or more, preferably 98% or more, and more preferably 99% or more to that of the original S2-C binding site or S2-D binding site without any substitutions with the same amino acids.

Furthermore, examples of the "S2-C binding site" and "S2-D binding site" of the present invention include those which contain each CDR having the above-mentioned specific amino acid sequences in the VH and VL constituting them, respectively, and of which the FW is humanized so that it is encoded by a human specific germ-line gene or gene thereof with one or more somatic mutations and/or back mutations. For example, the FWs encoded by human germ-line VH gene IGHV3-66 and VL gene IGKV1-39 or genes thereof with one or more somatic mutations and/or back mutations, respectively, can be applied to each CDR in the VH and VL represented by the above-mentioned (C1) and (D1), respectively. Examples of the S2-C binding site having the VH and VL humanized like that, respectively, include the antigen-binding site constituted by the VH and VL comprising a pair of the amino acid sequences set forth in SEQ ID No. 9 and SEQ ID No. 20 or 112, and examples of the S2-D binding site include the antigen-binding site constituted by the VH and VL comprising a pair of the amino acid sequences set forth in
(a) SEQ ID No. 10 and SEQ ID No. 21 or 113, or
(b) SEQ ID No. 11 and SEQ ID No. 22 or 114.

Furthermore, examples of the "S2-C binding site" of the present invention also include those which have a VH and VL comprising the respective amino acid sequences having at least 80%, preferably at least 90%, more preferably at least 95%, furthermore preferably at least 98%, or further more preferably at least 99% identity, each independently, to a pair of the amino acid sequences (excluding three CDRs, respectively) set forth in SEQ ID No. 9 and SEQ ID No. 20 or 112, and in which the differences from the respective amino acid sequences of the original VH and VL have no substantial effect on the binding activity to the same epitope on the S2 domain (hereinafter, may be abbreviated as "homologous S2-C binding site"). Examples of the homologous S2-C binding sites include the S2-C binding site having the VH in which glycine at position 44 in the VH comprising the amino acid sequence set forth in SEQ ID No. 9 (at position 44 according to the Kabat numbering system) was substituted by cysteine.

Examples of the "S2-D binding site" of the present invention also include those which have a VH and VL comprising the respective amino acid sequences having at least 80%, preferably at least 90%, more preferably at least 95%, furthermore preferably at least 98%, or further more preferably at least 99% identity, each independently, to a pair of the amino acid sequences (excluding three CDRs, respectively) set forth in
(a) SEQ ID No. 10 and SEQ ID No. 21 or 113, or
(b) SEQ ID No. 11 and SEQ ID No. 22 or 114, and in which the differences from the respective amino acid sequences of the original VH and VL have no substantial effect on the binding activity to the same epitope on the S2 domain (hereinafter, may be abbreviated as "homologous S2-D binding site"). Examples of the homologous S2-D binding sites include the S2-D binding site having the VH in which cysteine at position 44 in the VH comprising the amino acid sequence set forth in SEQ ID No. 11 (corresponding to position 44 according to the Kabat numbering system) was substituted by glycine and the S2-D binding site having the VH in which glycine at position 44 in the VH comprising the amino acid sequence set forth in SEQ ID No. 10 (corresponding to position 44 according to the Kabat numbering system) was substituted by cysteine.

In the present invention, the VL comprising the amino acid sequence selected from SEQ ID No. 20 preferably constitute a scFv, and the VH comprising the amino acid sequence set forth in SEQ ID No. 9 and the VL comprising the amino acid sequence set forth in SEQ ID No. 112 preferably constitute parts of the heavy chain/light chain complex, respectively. And, in the present invention, the VH comprising the amino acid sequence set forth in SEQ ID No. 11 and the VL comprising the amino acid sequence set forth in SEQ ID No. 21 or 22 preferably constitute scFvs, respectively, and the VH comprising the amino acid sequence set forth in SEQ ID No. 10 and the VL comprising the amino acid sequence set forth in SEQ ID No. 113 or 114 preferably constitute parts of the heavy chain/light chain complex, respectively.

In a yet another embodiment, examples of the "S2-C binding site" of the present invention also include an antigen-binding site cross-competing for binding to the epitope on the S2 domain with an antibody or an antigen-binding site thereof having the VH and VL comprising the respective amino acid sequences set forth in SEQ ID No. 9 and SEQ ID No. 112, and an antigen-binding site of which the binding to the same epitope on the S2 domain is cross-competed by an antibody or an antigen-binding site thereof having the VH and VL comprising the respective amino acid sequences set forth in SEQ ID No. 9 and SEQ ID No. 112.

Examples of the "S2-D binding site" of the present invention also include an antigen-binding site cross-competing for binding to the epitope on the S2 domain with an antibody or an antigen-binding site thereof having the VH and VL comprising a pair of the amino acid sequences set forth in
(a) SEQ ID No. 10 and SEQ ID No. 113, or
(b) SEQ ID No. 11 and SEQ ID No. 114, and an antigen-binding site of which the binding to the same epitope on the S2 domain is cross-competed by an antibody or an antigen-binding site thereof having the VH and VL comprising a pair of the amino acid sequences set forth in
   (a) SEQ ID No. 10 and SEQ ID No. 113, or
   (b) SEQ ID No. 11 and SEQ ID No. 114.

Examples thereof cross-competing for binding to the same epitope on the S2 domain with the S2-D binding site having the VH and VL comprising the respective amino acid sequences set forth in SEQ ID No. 10 and SEQ ID No. 21 or 113 include an antigen-binding site having the VH and VL comprising the respective amino acid sequences set forth in SEQ ID No. 11 and SEQ ID No. 22 or 114.

Herein, the "S2-C binding site" of the present invention is preferably an antigen-binding site constituted by the VH and VL comprising a pair of the amino acid sequences set forth in (a) SEQ ID No. 9 and SEQ ID No. 20 or 112, and
the "S2-D binding site" of the present invention is preferably an antigen-binding site constituted by the VH and VL comprising a pair of the amino acid sequences set forth in
(a) SEQ ID No. 10 and SEQ ID No. 21 or 113, or
(b) SEQ ID No. 11 and 22.

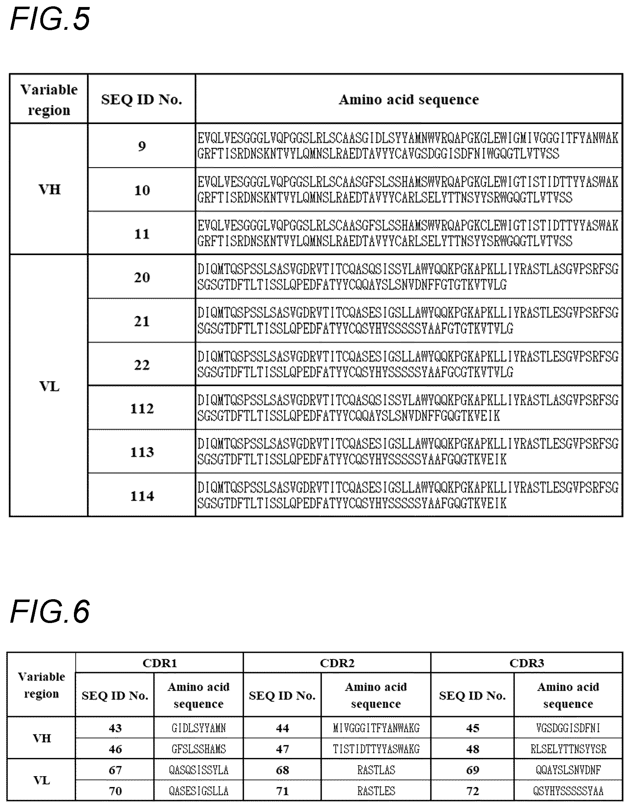

Herein, figure 5 shows the correspondence relations between the respective amino acid sequences of the VH and VL constituting the S2-C binding site and the S2-D binding site, respectively, and SEQ ID Nos thereof. Furthermore, figure 6 shows the correspondence relations between the amino acid sequences of multiple CDRs contained in those VH and VL, respectively, and SEQ ID Nos thereof.

### [Forms of anti-Allergin-1 bispecific antibody]

Examples of forms of the anti-Allergin-1 bispecific antibody of the present invention include a diabody (see Proc. Natl. Acad. Sci. USA (1993), Vol. 90, No.14: p.6444-6448), bispecific sc(Fv)₂ (see J. Biological Chemistry (1994), Vol. 269: p.199-206), bispecific F(ab')₂ (see Invest. Ophthalmol. Visual Sci. (2015), Vol. 56: p.5390-5400), bispecific minibody (see Biochemistry (1992), Vo.31, No.6, p.1579-1584), bispecific hybrid antibody, covalent diabody (bispecific DART) (see Blood (2011), Vol. 117, No.17, p.4542-4551), bispecific (FvCys)₂ (see J. Immunol. (1992), Vol. 149: p.120-126), bispecific F(ab'-zipper)₂ (see J. Immunol. (1992), Vol. 148, No. 5: p.1547-1553), bispecific (Fv-zipper)₂ (see Biochemistry (1992), Vol. 31, No. 6: p.1579-1584), bispecific three-chain antibody (see Proc. Natl. Acad. Sci. USA (1993), Vol. 90, No. 14: p.6444-6448) and bispecific mAb² (www.f-star.com/technology_mab.html), Morrison form antibody and the like.

The bispecific hybrid antibody is an intact antibody in which complexes consisting of a heavy chain and light chain of antibody recognizing two different antigens are covalently bound each other through disulfide bonds or the like. The bispecific hybrid antibody can be produced from, for example, a hybridoma produced according to a hybrid hybridoma method (see US4474893). Alternatively, it can be produced by having mammal animal cells co-express four kinds of cDNAs encoding a heavy chain and light chain of antibody recognizing different antigens, respectively, and secrete them.

The Morrison form antibody is a bispecific antibody in a form of which to the C-terminus of heavy chains or the C-terminus of light chains, scFvs binding an epitope different from the same antibody were linked (see Nat. Biotechnol. (1997), Vol. 15, No. 2: p.159-63 and Protein Eng. Des. Sel. (2010), Vol. 23, No. 4: p.221-228).

The anti-Allergin-1 bispecific antibody of the present invention is preferably the Morrison form antibody.

If the anti-Allergin-1 bispecific antibody of the present invention is the Morrison form antibody, one antigen-binding site constituting the bispecific antibody forms a part of complex of a heavy chain and light chain of the same bispecific antibody and another antigen-binding site forms scFv comprising a VH and VL constituting it. For example, the complex of the heavy chain and light chain can be formed by any one of combination selected from
(a) combination of a heavy chain having the VH constituting the S1-A binding site and a light chain having the VL constituting the same binding site,
(b) combination of a heavy chain having the VH constituting the S1-B binding site and a light chain having the VL constituting the same binding site,
(c) combination of a heavy chain having the VH constituting the S2-C binding site and a light chain having the VL constituting the same binding site, and
(d) combination of a heavy chain having the VH constituting the S2-D binding site and a light chain having the VL constituting the same binding site. Herein, for the heavy chain and light chain, those of IgG antibody can be used. In that case, the heavy chain has a heavy chain constant region 1 (hereinafter, may be abbreviated as "CH1"), heavy chain constant region 2 (hereinafter, may be abbreviated as "CH2") and heavy chain constant region 3 (hereinafter, may be abbreviated as "CH3") of IgG antibody, and the light chain has a light chain constant region (hereinafter, may be abbreviated as "CL") of IgG antibody.

On the other hand, the scFv is different from the antigen-binding site constituting a part of the complex of the heavy chain and light chain (hereinafter, may be abbreviated as "antigen-binding site in the complex of the heavy chain and light chain"), and is selected from antigen-binding sites which do not mutually compete, for example,
(a) if the antigen-binding site in the complex of the heavy chain and light chain is the S1-A binding site, it can be selected from any of the S1-B binding site, the S2-C binding site and the S2-D binding site,
(b) if the antigen-binding site in the complex of the heavy chain and light chain is the S1-B binding site, it can be selected from any of the S1-A binding site, the S2-C binding site and the S2-D binding site,
(c) if the antigen-binding site in the complex of the heavy chain and light chain is the S2-C binding site, it can be selected from any of the S1-A binding site, the S1-B binding site and the S2-D binding site, or
(d) if the antigen-binding site in the complex of the heavy chain and light chain is the S2-D binding site, it can be selected from any of the S1-A binding site, the S1-B binding site and the S2-C binding site.

Herein, a combination of the antigen-binding site in the complex of the heavy chain and light chain and that in the scFv is preferably a combination of the antigen-binding sites binding the S1 domain and the S2 domain of Allergin-1, respectively, for example, if the antigen-binding site in the complex of the heavy chain and light chain is the S1-A binding site or the S1-B binding site, that in the scFv is the S2-C binding site or the S2-D binding site, and if the antigen-binding site in the complex of the heavy chain and light chain is the S2-C binding site or the S2-D binding site, that in the scFv is the S1-A binding site or the S1-B binding site. Furthermore, if the antigen-binding site in the complex of the heavy chain and light chain is the S1-A binding site, a combination in which that in the scFv is the S2-C binding site or the S2-D binding site is more preferable, and if the antigen-binding site in the complex of the heavy chain and light chain is the S2-C binding site or the S2-D binding site, a combination in which that in the scFv is the S1-A binding site is more preferable.

Furthermore, the scFv links to the C-terminus of any one of the heavy chain or light chain. For example, the N-terminus of the scFv may link to the C-terminus of any one of the heavy chain or light chain through a direct amide bond, or may link to it through a peptide linker. The scFv preferably links to the C-terminus of the heavy chain, more preferably links to its C-terminus through the peptide linker. Herein the sentence "link to it through a peptide linker" means that the N-terminus of the peptide linker link to the C-terminus of any one of the heavy chain or light chain through an amide bond, and its C-terminus links to the N-terminus of the scFv through an amide bond. Note here that in one embodiment, the heavy chain or light chain which is linked with the scFv, constituting the anti-Allergin-1 bispecific antibody of the present invention is produced as a protein in a continuum thereof from host cells expressing them.

The VH and VL constituting the scFv may link to each other in order from the VH to VL from its N-terminus through a direct amide bond or a peptide linker, and conversely, may link to each other in order from the VL to VH from its N-terminus through a direct amide bond or a peptide linker, but preferably link to each other in order from the VL to VH from its N-terminus through a peptide linker.

The peptide linker linking the scFv to the C-terminus of any one of the heavy chain or light chain is not limited as long as the bispecific antibody of the present invention develops its expected activity, but such a peptide linker preferably comprises the amino acid sequence set forth in Gly-Gly-Gly-Gly-Ser-Gly-Gly-Gly-Gly-Ser (SEQ ID No. 73). On the other hand, the peptide linker mutually linking the VH and VL constituting the scFv is not limited as long as it does not cripple the binding activity to antigens of the scFv, but such a peptide linker preferably comprises the amino acid sequence set forth in Gly-Gly-Gly-Gly-Ser-Gly-Gly-Gly-Gly-Ser-Gly-Gly-Gly-Gly-Ser-Gly-Gly-Gly-Gly-Ser (SEQ ID No. 74). Herein, "Gly" represents glycine and "Ser" represents serine.

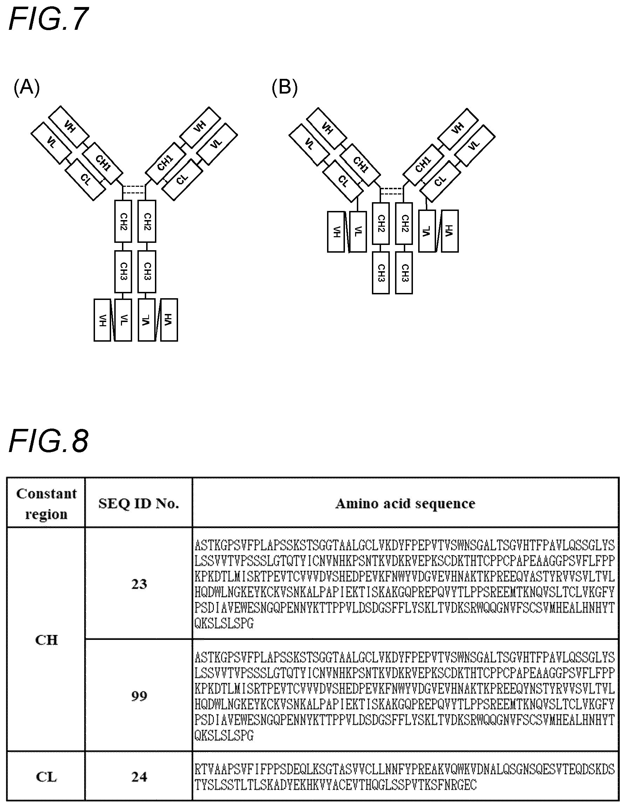

If the anti-Allergin-1 bispecific antibody of the present invention is the Morrison form antibody, it is preferably in a form in which two same complexes of heavy chain/light chain constituted by the heavy chain and light chain of which the scFv linked to any one of the C-terminuses in the aforementioned manner associate through disulfide bonds, and (A) and (B) of figure 7 illustrate such forms.

The anti-Allergin-1 bispecific antibody of the present invention may be derived from any of a chimeric antibody, humanized antibody and human antibody.

The chimeric antibody can be prepared by linking a gene encoding an antibody variable region isolated from antibody-producing hybridomas isolated according to hybridoma method (see Nature (1975), Vol. 256: p.495-97, Hongo. et. al, Hybridoma (1995), Vol. 14, No. 3, p.253-260, Antibodies: A Laboratory Manual, (Cold Spring Harbor Laboratory Press (1988), Vol. 2) or Monoclonal Antibodies and T-Cell Hybridomas, p.563-681(Elsevier, N.Y, 1981)), by well-known techniques, to a gene encoding the constant region of a human-derived antibody according to well-known methods (see US4816567).

The humanized antibody can also be prepared by linking genes encoding CDRs of an antibody isolated from the antibody-producing hybridomas isolated according to the above-mentioned method, by well-known techniques, respectively, to a gene encoding a framework region of the human-derived antibody according to well-known methods (see, e.g., US5225539, US5530101, US5585089 and US6180370).

On the other hand, the human antibody means an antibody in which all components are derived from human germline immunoglobulin sequences, and can be prepared by methods using mice transformed to produce a human antibody, for example, Humab mice (see US5545806, US5569825, US5625126, US5633425, US5789650, US5877397, US5661016, US5814318, US5874299 and US5770429), KM mice (see WO2002/43478), Xeno mice (see US5939598, US6075181, US6114598, US6150584 and US6162963), or Tc mice (see Proc. Natl. Acad. Sci. USA (2000), pp.722-727). It can also be prepared using SCID mice into which human immune cells have been reconstructed so that the human antibody response is made upon immunization (see US5476996 and US5698767). Furthermore, it can also be prepared by phage display method (see US5223409, US5403484, US5571698, US5427908, US5580717, US5969108, US6172197, US5885793, US6521404, US6544731, US6555313, US6582915 or US6593081).

The isotype for the anti-Allergin-1 bispecific antibody of the present invention is not limited, but is preferably IgG, more preferably IgG₁ or IgG₄.

Herein, the IgG₁ is preferably of which the binding to Fc receptor was eliminated or decreased, and which can be obtained by substituting, deleting or inserting arbitrary amino acids of its heavy chain constant region. Examples thereof include the antibody in which leucine at position 235 according to the EU numbering system was substituted with glycine, and/or glycine at position 236 was substituted with arginine on two heavy chain constant regions or hinge regions of the bispecific antibody. Furthermore, in order to reduce the heterogeneity of antibody, the IgG₁ in which an amino acid at its C-terminus, for example, lysine at position 447 according to the EU numbering system has been deleted is preferable.

If the isotype for the anti-Allergin-1 bispecific antibody of the present invention is IgG₄, in order to suppress the intramolecular swapping, a variant in which arbitrary amino acids in its heavy chain constant region were substituted, deleted or inserted is more preferable. For example, the antibody of which serine located in hinge regions and at position 228 according to the EU numbering system were substituted with proline is preferable. Note herein that in the present specification, amino acid positions assigned to CDRs and framework regions in variable regions of an antibody may be specified according to Kabat's numbering (see Sequences of Proteins of Immunological Interest, National Institute of Health, Bethesda, Md., (1987) and 1991)), and amino acids in constant regions are indicated according to the EU numbering system according to Kabat's amino acid positions (see Sequences of proteins of immunological interest, NIH Publication No. 91-3242).

The isotype for the anti-Allergin-1 bispecific antibody of the present invention is preferably IgG₁.

If the isotype for the anti-Allergin-1 bispecific antibody of the present invention is IgG₁, examples of embodiments thereof include an antibody having the heavy chain constant region comprising the amino acid sequence set forth in SEQ ID No. 23 or 99 (arginine at position 97 in the amino acid sequence set forth in SEQ ID No. 23 or 99 (corresponding to position 214 according to the EU numbering system in the heavy chain constant region) may be substituted by lysine) and the light chain constant region comprising the amino acid sequence set forth in SEQ ID No. 24, which are shown in figure 8, respectively.

The "antibody fragment" of the anti-Allergin-1 bispecific antibody of the present invention is a part of the bispecific antibody and an antibody having at least any one of antigen-binding sites specifically binding to Allergin-1. An embodiment of the antibody fragment is an antibody fragment comprising the VH and VL constituting any one of the S1-A binding site, the S1-B binding site, the S2-C binding site and the S2-D binding site, and examples thereof include an antibody fragment comprising the VH and VL exemplified in the preceding items "S1-A binding site and S1-B binding site" and "S2-C binding site and S2-D binding site", respectively. Examples of antibody fragments comprising the VH and VL include Fab, Fab', Fv, F(ab')₂ and the like, as well as a complex of heavy chain/light chain having the VH and VL and a complex in which the scFv comprising the VH and VL constituting another antigen-binding site linked to the C-terminus of any one of the heavy chain and light chain in the complex of heavy chain/light chain. Furthermore, examples thereof also include the scFv itself.

If the anti-Allergin-1 bispecific antibody of the present invention is a Morrison form antibody, examples of embodiments thereof include a bispecific antibody specifically which binds to two different epitopes on extracellular domains of Allergin-1, respectively, and has two different antigen-binding sites which do not mutually compete for Allergin-1 and which is selected from the S1-A binding site, the S1-B binding site, the S2-C binding site and the S2-D binding site, wherein
(I) the S1-A binding site is any one selected from
   (i) an antigen-binding site comprising the VH having
      (a) the VH-CDR1 comprising the amino acid sequence set forth in SEQ ID No. 25,
      (b) the VH-CDR2 comprising the amino acid sequence set forth in SEQ ID No. 26, and
      (c) the VH-CDR3 comprising the amino acid sequence set forth in SEQ ID No. 27, and
      the VL having
      (d) the VL-CDR1 comprising the amino acid sequence set forth in SEQ ID No. 49,
      (e) the VL-CDR2 comprising the amino acid sequence set forth in SEQ ID No. 50, and
      (f) the VL-CDR3 comprising the amino acid sequence set forth in SEQ ID No. 51,
   (ii) an antigen-binding site comprising the VH having
      (a) the VH-CDR1 comprising the amino acid sequence set forth in SEQ ID No. 28,
      (b) the VH-CDR2 comprising the amino acid sequence set forth in SEQ ID No. 29, and
      (c) the VH-CDR3 comprising the amino acid sequence set forth in SEQ ID No. 30, and
      the VL having
      (d) the VL-CDR1 comprising the amino acid sequence set forth in SEQ ID No. 52,
      (e) the VL-CDR2 comprising the amino acid sequence set forth in SEQ ID No. 53, and
      (f) the VL-CDR3 comprising the amino acid sequence set forth in SEQ ID No. 54,
   (iii) an antigen-binding site comprising the VH having
      (a) the VH-CDR1 comprising the amino acid sequence set forth in SEQ ID No. 31,
      (b) the VH-CDR2 comprising the amino acid sequence set forth in SEQ ID No. 32, and
      (c) the VH-CDR3 comprising the amino acid sequence set forth in SEQ ID No. 33, and
      the VL having:
      (d) the VL-CDR1 comprising the amino acid sequence set forth in SEQ ID No. 55,
      (e) the VL-CDR2 comprising the amino acid sequence set forth in SEQ ID No. 56, and
      (f) the VL-CDR3 comprising the amino acid sequence set forth in SEQ ID No. 57, and
   (iv) an antigen-binding site comprising the VH having
      (a) the VH-CDR1 comprising the amino acid sequence set forth in SEQ ID No. 34,
      (b) the VH-CDR2 comprising the amino acid sequence set forth in SEQ ID No. 35, and
      (c) the VH-CDR3 comprising the amino acid sequence set forth in SEQ ID No. 36, and
      the VL having:
      (d) the VL-CDR1 comprising the amino acid sequence set forth in SEQ ID No. 58,
      (e) the VL-CDR2 comprising the amino acid sequence set forth in SEQ ID No. 59, and
      (f) the VL-CDR3 comprising the amino acid sequence set forth in SEQ ID No. 60, the S1-B binding site is
   (v) an antigen-binding site comprising the VH having
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39, and
      the VL having
      (d) the VL-CDR1 comprising the amino acid sequence set forth in SEQ ID No. 61,
      (e) the VL-CDR2 comprising the amino acid sequence set forth in SEQ ID No. 62, and
      (f) the VL-CDR3 comprising the amino acid sequence set forth in SEQ ID No. 63, or
   (vi) an antigen-binding site comprising the VH having
      (a) the VH-CDR1 comprising the amino acid sequence set forth in SEQ ID No. 40,
      (b) the VH-CDR2 comprising the amino acid sequence set forth in SEQ ID No. 41, and
      (c) the VH-CDR3 comprising the amino acid sequence set forth in SEQ ID No. 42, and
      the VL having
      (d) the VL-CDR1 comprising the amino acid sequence set forth in SEQ ID No. 64,
      (e) the VL-CDR2 comprising the amino acid sequence set forth in SEQ ID No. 65, and
      (f) the VL-CDR3 comprising the amino acid sequence set forth in SEQ ID No. 66,

   the S2-C binding site is an antigen-binding site comprising the VH having
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
      the VL having
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69, and
   the S2-D binding site is an antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
      the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72,
(II) wherein one of the two different antigen-binding sites is one in the complex of heavy chain/light chain constituting the bispecific antibody, and another is an antigen-binding site in the form of scFv, and wherein
   (i) the antigen-binding site in the complex of heavy chain/light chain constituting the bispecific antibody is the S1-A binding site, and the antigen-binding site in the form of the scFv is any one kind of the antigen-binding site selected from the S1-B binding site, the S2-C binding site and the S2-D binding site,
   (ii) the antigen-binding site in the complex of heavy chain/light chain constituting the bispecific antibody is the S1-B binding site, and the antigen-binding site in the form of the scFv is any one kind of the antigen-binding site selected from the S1-A binding site, the S2-C binding site and the S2-D binding site,
   (iii) the antigen-binding site in the complex of heavy chain/light chain constituting the bispecific antibody is the S2-C binding site, and the antigen-binding site in the form of the scFv is any one kind of the antigen-binding site selected from the S1-A binding site, the S 1-B binding site and the S2-D binding site, or
   (iv) the antigen-binding site in the complex of heavy chain/light chain constituting the bispecific antibody is the S2-D binding site, and the antigen-binding site in the form of the scFv is any one kind of the antigen-binding site selected from the S1-A binding site, the S1-B binding site and the S2-C binding site,
(III) wherein the N-terminus of the VH or VL constituting the scFv links to the C-terminus of any one of the heavy chain or light chain through a peptide linker, and
(IV) (i) having the heavy chain and light chain in the form described in the preceding items (I) to (III), or (ii) consisting of two same complexes of heavy chain/light chain constituted by the same heavy chain and light chain in the form described in the preceding items (I) to (III).

Herein, preferable examples of combinations of two different antigen-binding site constituting the anti-Allergin-1 bispecific antibody of the present invention include a combination of any two selected from:
(i) the S1-A binding site constituted by the VH and VL comprising any one pair of the amino acid sequences selected from
   (a) SEQ ID No. 1 and 12,
   (b) SEQ ID No. 2 and 13,
   (c) SEQ ID No. 3 and SEQ ID No. 14 or 106,
   (d) SEQ ID No. 4 and SEQ ID No. 15 or 107,
   (e) SEQ ID No. 5 and 16, and
   (f) SEQ ID No. 6 and SEQ ID No. 17 or 109,
(ii) the S1-B binding site constituted by the VH and VL comprising a pair of the amino acid sequences set forth in (a) SEQ ID No. 7 and 18 or (b) SEQ ID No. 8 and 19,
(iii) the S2-C binding site constituted by the VH and VL comprising a pair of the amino acid sequences set forth in (a) SEQ ID No. 9 and SEQ ID No. 20 or 112, and
(iv) the S2-D binding site constituted by the VH and VL comprising a pair of the amino acid sequences set forth in (a) SEQ ID No. 10 and SEQ ID No. 21 or 113, or (b) SEQ ID No. 11 and 22, and
more preferable examples thereof include a combination of any two selected from:
(i) the S1-A binding site constituted by the VH and VL comprising any one pair of the amino acid sequences selected from
   (a) SEQ ID No. 3 and SEQ ID No. 14 or 106,
   (b) SEQ ID No. 4 and SEQ ID No. 15 or 107,
   (c) SEQ ID No. 5 and 16, and
   (d) SEQ ID No. 6 and SEQ ID No. 17 or 109,
(ii) the S2-C binding site constituted by the VH and VL comprising a pair of the amino acid sequences set forth in (a) SEQ ID No. 9 and SEQ ID No. 20 or 112, and
(iii) the S2-D binding site constituted by the VH and VL comprising a pair of the amino acid sequences set forth in (a) SEQ ID No. 10 and SEQ ID No. 21 or 113, or (b) SEQ ID No. 11 and 22.

Furthermore, preferable examples of combinations of two different antigen-binding site constituting the anti-Allergin-1 bispecific antibody of the present invention include
(i) a combination of
   (a) the S1-A binding site constituted by the VH and VL comprising a pair of the amino acid sequences set forth in SEQ ID No. 3 and SEQ ID No. 14 or 106, and
   (b) the S2-C binding site constituted by the VH and VL comprising a pair of the amino acid sequences set forth in SEQ ID No. 9 and SEQ ID No. 20 or 112,
(ii) a combination of
   (a) the S1-A binding site constituted by the VH and VL comprising a pair of the amino acid sequences set forth in SEQ ID No. 6 and SEQ ID No. 17 or 109, and
   (b) the S2-D binding site constituted by the VH and VL comprising a pair of the amino acid sequences set forth in SEQ ID No. 10 and SEQ ID No. 21 or 113, and
(iii) a combination of
   (a) the S1-A binding site constituted by the VH and VL comprising any one pair of the amino acid sequences selected from
   (a1) SEQ ID No. 3 and SEQ ID No. 14 or 106,
   (a2) SEQ ID No. 4 and SEQ ID No. 15 or 107, and
   (a3) SEQ ID No. 5 and 16, and
   (b) the S2-D binding site constituted by the VH and VL comprising a pair of the amino acid sequences set forth in
   (b1) SEQ ID No. 10 and SEQ ID No. 21 or 113, or
   (b2) SEQ ID No. 11 and 22, and
   more preferable examples thereof include
      (i) a combination of
         (a) the S1-A binding site constituted by the VH and VL comprising a pair of the amino acid sequences set forth in SEQ ID No. 3 and 14, and
         (b) the S2-C binding site constituted by the VH and VL comprising a pair of the amino acid sequences set forth in SEQ ID No. 9 and 112,
      (ii) a combination of
         (a) the S1-A binding site constituted by the VH and VL comprising a pair of the amino acid sequences set forth in SEQ ID No. 3 and 106, and
         (b) the S2-C binding site constituted by the VH and VL comprising a pair of the amino acid sequences set forth in SEQ ID No. 9 and 20,
      (iii) a combination of
         (a) the S1-A binding site constituted by the VH and VL comprising a pair of the amino acid sequences set forth in SEQ ID No. 6 and 17, and
         (b) the S2-D binding site constituted by the VH and VL comprising a pair of the amino acid sequences set forth in SEQ ID No. 10 and 113,
      (iv) a combination of
         (a) the S1-A binding site constituted by the VH and VL comprising a pair of the amino acid sequences set forth in SEQ ID No. 6 and 109, and
         (b) the S2-D binding site constituted by the VH and VL comprising a pair of the amino acid sequences set forth in SEQ ID No. 10 and 21,
      (v) a combination of
         (a) the S1-A binding site constituted by the VH and VL comprising a pair of the amino acid sequences set forth in SEQ ID No. 3 and 14, and
         (b) the S2-D binding site constituted by the VH and VL comprising a pair of the amino acid sequences set forth in SEQ ID No. 10 and 113,
      (vi) a combination of
         (a) the S1-A binding site constituted by the VH and VL comprising a pair of the amino acid sequences set forth in SEQ ID No. 4 and 15, and
         (b) the S2-D binding site constituted by the VH and VL comprising a pair of the amino acid sequences set forth in SEQ ID No. 10 and 113,
      (vii) a combination of
         (a) the S1-A binding site constituted by the VH and VL comprising any one pair of the amino acid sequences selected from SEQ ID No. 5 and 16, and
         (b) the S2-D binding site constituted by the VH and VL comprising a pair of the amino acid sequences set forth in SEQ ID No. 10 and 113,
      (viii) a combination of
         (a) the S1-A binding site constituted by the VH and VL comprising a pair of the amino acid sequences set forth in SEQ ID No. 3 and 106, and
         (b) the S2-D binding site constituted by the VH and VL comprising a pair of the amino acid sequences set forth in SEQ ID No. 10 and 21, and
      (ix) a combination of
         (a) the S1-A binding site constituted by the VH and VL comprising a pair of the amino acid sequences set forth in SEQ ID No. 4 and 107, and
         (b) the S2-D binding site constituted by the VH and VL comprising a pair of the amino acid sequences set forth in SEQ ID No. 11 and 22.

Furthermore, herein, the VH and VL constituting the scFv preferably links to each other in order from the VL to VH from its N-terminus through the peptide linker comprising the amino acid set forth in SEQ ID No. 74, and the peptide linker linking the scFv to the C-terminus of the heavy chain or light chain is preferably one comprising the amino acid set forth in SEQ ID No. 73.

Preferable examples of the Allergin-1 bispecific antibodies of the present invention in the form of which the scFv linked to the C-terminus of heavy chain include an antibody
(i) having the heavy chain and light chain comprising a pair of the amino acid sequences selected from
   (a) SEQ ID No. 75 and 76,
   (b) SEQ ID No. 77 and 78,
   (c) SEQ ID No. 79 and 78,
   (d) SEQ ID No. 80 and 78,
   (e) SEQ ID No. 81 and 82,
   (f) SEQ ID No. 83 and 82,
   (g) SEQ ID No. 84 and 85,
   (h) SEQ ID No. 86 and 87,
   (i) SEQ ID No. 98 and 78,
   (j) SEQ ID No. 100 and 78,
   (k) SEQ ID No. 101 and 78,
   (l) SEQ ID No. 102 and 78, and
   (m) SEQ ID No. 103 and 85, or
(ii) consisting of two same complexes of heavy chain/light chain constituted by the heavy chain and light chain of the preceding item (i), on the other hand, preferable examples of the Allergin-1 bispecific antibodies of the present invention in the form of which the scFv linked to the C-terminus of light chain include an antibody

(i) having the heavy chain and light chain comprising a pair of the amino acid sequences selected from
   (a) SEQ ID No. 88 and 89,
   (b) SEQ ID No. 90 and 91,
   (c) SEQ ID No. 90 and 92,
   (d) SEQ ID No. 93 and 94,
   (e) SEQ ID No. 93 and 95,
   (f) SEQ ID No. 96 and 97, or
(ii) consisting of two same complexes of heavy chain/light chain constituted by the heavy chain and light chain of the preceding item (i). Herein, arginine at position 214 according to the EU numbering system in the heavy chain constant regions of these Allergin-1 bispecific antibodies may be substituted by lysine, and/or alanine at position 299 in SEQ ID No. 75, 81, 83, 86, 88, 93 and 96 and at position 301 in SEQ ID No. 80 and 90 (corresponding to position 297 according to the EU numbering system in the heavy chain constant regions) may be substituted by asparagine, each independently.

The anti-Allergin-1 bispecific antibody of the present invention is more preferably an antibody (i) having the heavy chain and light chain comprising a pair of the amino acid sequences selected from
(a) SEQ ID No. 77 and 78,
(b) SEQ ID No. 79 and 78,
(c) SEQ ID No. 83 and 82,
(d) SEQ ID No. 84 and 85,
(e) SEQ ID No. 98 and 78,
(f) SEQ ID No. 100 and 78,
(g) SEQ ID No. 101 and 78,
(h) SEQ ID No. 102 and 78, and
(j) SEQ ID No. 103 and 85, or
(ii) consisting of two same complexes of heavy chain/light chain constituted by the heavy chain and light chain of the preceding item (i).

Preferable features of the anti-Allergin-1 bispecific antibody of the present invention include one which cytokine production during administration thereof or in 24 hours after administration thereof is sufficiently reduced. Herein, the sentence "cytokine production is sufficiently reduced" means that, e.g., during administration or in 24 hours after that administration, by drip infusion and the like of the Allergin-1 bispecific antibody of the present invention, e.g., concentration of cytokine containing at least one or more selected from IL-2, IL-4, IL-6, IL-10, IFN-γ and TNF-α in blood or tissue do not increase, or increase to only such a degree that it can be suppressed by steroid administration.

Embodiments of features of the anti-Allergin-1 bispecific antibody of the present invention include a feature that it inhibits the binding of TLR9 ligand (e.g., ODN M362, ODN 1668 and ODN 1826, etc.) to Allergin-1.

### [Methods for manufacturing and purifying the anti-Allergin-1 bispecific antibody]

The anti-Allergin-1 bispecific antibody and antibody fragment thereof of the present invention can be manufactured by having it secret from host cells, according to well-known methods, for example, can be manufactured by gene-transferring expression vectors in which (1) a polynucleotide encoding the heavy chain of which the scFv linked to its C-terminus and (2) a polynucleotide encoding the light chain have been inserted, respectively, into mammalian animal cells to transform them, and then having the heavy chain and light chain express and secret together.

Herein, preferable examples of host cells for expressing the anti-Allergin-1 bispecific antibody of the present invention include insect cells such as SF-9 cells and SF-21 cells, more preferable examples thereof include (i) mouse cells such as CHO cells, BHK cells, SP2/0 cells and NS-0 myeloma cells, (ii) primate cells such as COS cells and Vero cells and (iii) mammalian cells such as MDCK cells, BRL 3A cells, hybridoma, tumor cells, immortalized primary cells, W138 cells, HepG2 cells, HeLa cells, HEK293 cells, HT1080 cells and embryonic retina cells such as PER.C6 cells and the like. Note here that in selection of the expression system, mammalian cells and expression vectors therefor may be used so that antibodies are appropriately glycosylated. Human cells, preferably, PER.C6 cells are advantageously used to obtain antibodies corresponding to glycosylated patterns for human.

Protein production in host cells transformed by gene-transferring the expression vectors can be carried out with reference to, for example, Current Protocols in Protein Science (1995), Coligan JE, Dunn BM, Ploegh HL, Speicher DW, Wingfield PT, ISBN 0-471-11184-8, Bendig, 1988. Furthermore, general guidelines, procedures and practical methods to maximize the productivity of host cell culture can be carried out with reference to Mammalian Cell Biotechnology: a Practical Approach (M. Butler, ed., IRL Press, 1991). Expression of antibodies in host cells is described in, for example, publication such as EP0120694, EP0314161, EP0481790, EP0523949, US4816567, WO2000/63403 and the like.

Herein, culture conditions of host cells can be optimized by well-known methods, and the amount of protein production thereof can be optimized. The culture can be carried out by batch culture, feeding culture, continuous culture, and hollow-fiber culture in a petri dish, roller bottle or reaction chamber. In order to produce recombinant proteins by cell culture in a large-scale and continuously, it is preferable to allow cells to proliferate in a suspension. Furthermore, it is preferable to culture cells in a condition without animal- or human-derived serum or serum components thereof.

Antibodies expressed in host cells and recovered from the cells or cell culture by well-known methods can be purified using well-known methods. Examples of purification methods include immunoprecipitation method, centrifugation method, filtration, size-exclusion chromatography, affinity chromatography, cation and/or anion exchange chromatography, hydrophobic interaction chromatography, and the like. Furthermore, protein A or protein G affinity chromatography may be preferably used (see, e.g., US4801687 and US5151504).

### [Anti-Allergin-1 antibody]

The present inventions also include an antibody specifically binding to the S1 domain of Allergin-1 (hereinafter, may be just abbreviated as "anti-S1 domain antibody") and an antibody specifically binding to the S2 domain of Allergin-1 (hereinafter, may be just abbreviated as "anti-S2 domain antibody")(these may be collectively abbreviated as "anti-Allertgin-1 antibody") and antibody fragments thereof, which are used to construct the anti-Allergin-1 bispecific antibody of the present invention.

The anti-S1 domain antibody of the present invention can specifically bind to the S1 domain of Allergin-1 and is not limited as long as it can be used to construct the anti-Allergin-1 bispecific antibody of the present invention, but is an antibody which can directly bind to the S1 domain of Allergin-1 with the binding activity of at least 1 x 10⁻⁵ M, preferably 1 x 10⁻⁷ M and more preferably more than 1 x 10⁻⁹ M affinity (Kd value), and does not substantially bind to at least the S2 domain of Allergin-1 and any other proteins. Examples of the anti-S1 domain antibodies include an antibody specifically binding to the S1-A epitope (the epitope to which the S1-A binding site specifically binds) or the S1-B epitope (the epitope to which the S1-B binding site specifically binds).

On the other hand, the anti-S2 domain antibody of the present invention can specifically bind to the S2 domain of Allergin-1 and is not limited as long as it can be used to construct the anti-Allergin-1 bispecific antibody of the present invention, but is an antibody which can directly bind to the S2 domain of Allergin-1 with the binding activity of at least 1 x 10⁻⁵ M, preferably 1 x 10⁻⁷ M and more preferably more than 1 x 10⁻⁹ M affinity (Kd value), and does not substantially bind to at least the S1 domain of Allergin-1 and any other proteins. Examples of the anti-S2 domain antibodies include an antibody specifically binding to the S2-C epitope (the epitope to which the S2-C binding site specifically binds) or the S2-D epitope (the epitope to which the S2-D binding site specifically binds). Herein, the term "antibody" in the "anti-Allergin-1 antibody", "anti-S1 domain antibody" and "anti-S2 domain antibody" means full-length antibody, that is, an full-length antibody consisting of two heavy chains and two light chains, and the term "antibody fragment thereof" means a part of the full-length antibody, which contains at least an antigen-binding site, and examples thereof include Fab, Fab', Fv and F(ab')₂ and the like.

The anti-Allergin-1 antibody of the present invention is preferably a chimeric antibody, humanized antibody or human antibody in constructing the anti-Allergin-1 bispecific antibody of the present invention, but further examples thereof also includes a non-human antibody (e.g., a mouse antibody, rat antibody and rabbit antibody) which can be used to construct the chimeric antibody or humanized antibody.

Examples of embodiments of the anti-S 1 antibodies of the present invention include
(E1) an antibody comprising the VH having
   (a) the VH-CDR1 comprising the amino acid sequence set forth in SEQ ID No. 25,
   (b) the VH-CDR2 comprising the amino acid sequence set forth in SEQ ID No. 26, and
   (c) the VH-CDR3 comprising the amino acid sequence set forth in SEQ ID No. 27, and
      the VL having
   (d) the VL-CDR1 comprising the amino acid sequence set forth in SEQ ID No. 49,
   (e) the VL-CDR2 comprising the amino acid sequence set forth in SEQ ID No. 50, and
   (f) the VL-CDR3 comprising the amino acid sequence set forth in SEQ ID No. 51,
(E2) an antibody comprising the VH having
   (a) the VH-CDR1 comprising the amino acid sequence set forth in SEQ ID No. 28,
   (b) the VH-CDR2 comprising the amino acid sequence set forth in SEQ ID No. 29, and
   (c) the VH-CDR3 comprising the amino acid sequence set forth in SEQ ID No. 30, and
      the VL having
   (d) the VL-CDR1 comprising the amino acid sequence set forth in SEQ ID No. 52,
   (e) the VL-CDR2 comprising the amino acid sequence set forth in SEQ ID No. 53, and
   (f) the VL-CDR3 comprising the amino acid sequence set forth in SEQ ID No. 54,
(E3) an antibody comprising the VH having
   (a) the VH-CDR1 comprising the amino acid sequence set forth in SEQ ID No. 31,
   (b) the VH-CDR2 comprising the amino acid sequence set forth in SEQ ID No. 32, and
   (c) the VH-CDR3 comprising the amino acid sequence set forth in SEQ ID No. 33, and
      the VL having
   (d) the VL-CDR1 comprising the amino acid sequence set forth in SEQ ID No. 55,
   (e) the VL-CDR2 comprising the amino acid sequence set forth in SEQ ID No. 56, and
   (f) the VL-CDR3 comprising the amino acid sequence set forth in SEQ ID No. 57, and
(E4) an antibody comprising the VH having
   (a) the VH-CDR1 comprising the amino acid sequence set forth in SEQ ID No. 34,
   (b) the VH-CDR2 comprising the amino acid sequence set forth in SEQ ID No. 35, and
   (c) the VH-CDR3 comprising the amino acid sequence set forth in SEQ ID No. 36, and
      the VL having
   (d) the VL-CDR1 comprising the amino acid sequence set forth in SEQ ID No. 58,
   (e) the VL-CDR2 comprising the amino acid sequence set forth in SEQ ID No. 59, and
   (f) the VL-CDR3 comprising the amino acid sequence set forth in SEQ ID No. 60,
(E5) an antibody comprising the VH having
   (a) the VH-CDR1 comprising the amino acid sequence set forth in SEQ ID No. 37,
   (b) the VH-CDR2 comprising the amino acid sequence set forth in SEQ ID No. 38, and
   (c) the VH-CDR3 comprising the amino acid sequence set forth in SEQ ID No. 39, and
   the VL having:
   (d) the VL-CDR1 comprising the amino acid sequence set forth in SEQ ID No. 61,
   (e) the VL-CDR2 comprising the amino acid sequence set forth in SEQ ID No. 62, and
   (f) the VL-CDR3 comprising the amino acid sequence set forth in SEQ ID No. 63, and
(E6) an antibody comprising the VH having
   (a) the VH-CDR1 comprising the amino acid sequence set forth in SEQ ID No. 40,
   (b) the VH-CDR2 comprising the amino acid sequence set forth in SEQ ID No. 41, and
   (c) the VH-CDR3 comprising the amino acid sequence set forth in SEQ ID No. 42, and
   the VL having:
   (d) the VL-CDR1 comprising the amino acid sequence set forth in SEQ ID No. 64,
   (e) the VL-CDR2 comprising the amino acid sequence set forth in SEQ ID No. 65, and
   (f) the VL-CDR3 comprising the amino acid sequence set forth in SEQ ID No. 66. On the other hand, examples of embodiments of the anti-S2 antibodies include
(F1) an antibody comprising the VH having
   (a) the VH-CDR1 comprising the amino acid sequence set forth in SEQ ID No. 43,
   (b) the VH-CDR2 comprising the amino acid sequence set forth in SEQ ID No. 44, and
   (c) the VH-CDR3 comprising the amino acid sequence set forth in SEQ ID No. 45, and
   the VL having
   (d) the VL-CDR1 comprising the amino acid sequence set forth in SEQ ID No. 67,
   (e) the VL-CDR2 comprising the amino acid sequence set forth in SEQ ID No. 68, and
   (f) the VL-CDR3 comprising the amino acid sequence set forth in SEQ ID No. 69, and
(F2) an antibody comprising the VH having
   (a) the VH-CDR1 comprising the amino acid sequence set forth in SEQ ID No. 46,
   (b) the VH-CDR2 comprising the amino acid sequence set forth in SEQ ID No. 47, and
   (c) the VH-CDR3 comprising the amino acid sequence set forth in SEQ ID No. 48, and
   the VL having
   (d) the VL-CDR1 comprising the amino acid sequence set forth in SEQ ID No. 70,
   (e) the VL-CDR2 comprising the amino acid sequence set forth in SEQ ID No. 71, and
   (f) the VL-CDR3 comprising the amino acid sequence set forth in SEQ ID No. 72.

Furthermore, examples of the anti-Allergin-1 antibodies of the present invention also include those of which one to five arbitrary amino acid residues in each CDR are substituted with other amino acids (preferably, conservative amino acids thereof), and which have substantially the same binding activity as that to the same epitope of the original antibody without any substitutions with the same amino acids. Examples thereof include those of which one amino acid residue in the VH-CDR1 is substituted with a conservative amino acid thereof, and those of which one or two amino acid residues in the VH-CDR2 or VH-CDR3 were substituted with conservative amino acids thereof.

Furthermore, examples of the anti-Allergin-1 antibodies of the present invention include those which have each CDR comprising the above-mentioned specific amino acid sequences in its VH and VL, and of which the amino acid sequences of framework regions thereof are encoded by specific germ-line genes or genes thereof with one or more somatic mutations and/or back mutations. Examples thereof include an VH and VL encoded by the above-mentioned germ-line VH gene IGHV3-66 and VL gene IGKV1-39 or genes thereof with one or more somatic mutations and/or back mutations, respectively, and the like.

Furthermore, in another embodiment, examples of the anti-S1 antibodies of the present invention include an antibody having the VH and VL comprising any one pair of the amino acid sequences selected from
(G1) SEQ ID No. 1 and 104,
(G2) SEQ ID No. 2 and 105,
(G3) SEQ ID No. 3 and 106,
(G4) SEQ ID No. 4 and 107,
(G5) SEQ ID No. 5 and 108,
(G6) SEQ ID No. 6 and 109,
(G7) SEQ ID No. 7 and 110, and
(G8) SEQ ID No. 8 and 111, on the other hand, in another embodiment, examples of the anti-S2 antibody include an antibody having the VH and VL comprising any one pair of the amino acid sequences selected from
(H1) SEQ ID No. 9 and 112,
(H2) SEQ ID No. 10 and 113, and
(H3) SEQ ID No. 11 and 114.

Furthermore, in such another embodiment, examples of the Allergin-1 antibodies also include those which have a VH and VL comprising the respective amino acid sequences having at least 80%, preferably at least 90%, more preferably at least 95%, furthermore preferably at least 98%, or further more preferably at least 99% identity, each independently, to any one pair of the amino acid sequences (excluding three CDRs, respectively) selected from the above-mentioned (G1) to (G8) or any one pair of the amino acid sequences (excluding three CDRs, respectively) selected from the above-mentioned (H1) to (H3), and in which the differences from the respective amino acid sequences of the original VH and VL have no substantial effect on the binding activity to the same epitope.

Furthermore, in a yet another embodiment, examples of the anti-S 1 antibodies of the present invention also include an antibody cross-competing for binding to the epitope on the S1 domain with an antibody or an antigen-binding site thereof having the VH and VL comprising any one pair of the amino acid sequences selected from the above-mentioned (G1) to (G8), and an antibody of which the binding to the same epitope on the S1 domain is cross-competed by an antibody or an antigen-binding site thereof having the VH and VL comprising any one pair of the amino acid sequences selected from the above-mentioned (G1) to (G8).

Furthermore, in a yet another embodiment, examples of the anti-S2 antibodies also include an antibody cross-competing for binding to the epitope on the S2 domain with an antibody or an antigen-binding site thereof having the VH and VL comprising any one pair of the amino acid sequences selected from the above-mentioned (H1) to (H3), and an antibody of which the binding to the same epitope on the S2 domain is cross-competed by an antibody or an antigen-binding site thereof having the VH and VL comprising any one pair of the amino acid sequences selected from the above-mentioned (H1) to (H3).

The form of the Allergin-1 antibody of the present invention is preferably a monoclonal antibody. The monoclonal antibody can be obtained according to the above-mentioned hybridoma method and various well-known methods.

The isotype for the anti-Allergin-1 antibody of the present invention is preferably IgG₁.

If the isotype for the anti-Allergin-1 antibody of the present invention is IgG₁, examples of embodiments thereof include an antibody having heavy chain constant regions comprising the amino acid sequence set forth in SEQ ID No. 23 or 99 (arginine at position 97 in the amino acid sequence set forth in SEQ ID No. 23 or 99 (corresponding to position 214 according to the EU numbering system in the heavy chain constant region) may be substituted by lysine) and light chain constant regions comprising the amino acid sequence set forth in SEQ ID No. 24.

### [Polynucleotide coding the anti-Allergin-1 bispecific antibody]

The present inventions also include polynucleotides coding a VH and VL constituting any two among each part of the anti-Allergin-1 bispecific antibody of the present invention, used to produce the bispecific antibody, which are the S1-A binding site, the S1-B binding site, the S2-C binding site and the S2-D binding site, respectively, and a heavy chain constant region and light chain constant region, respectively.

Examples of polynucleotides coding the VH and VL constituting the S1-A binding site, respectively, include those coding the VH and VL comprising any one pair of the amino acid sequences selected from
(a) SEQ ID No. 1 and SEQ ID No. 12 or 104,
(b) SEQ ID No. 2 and SEQ ID No. 13 or 105,
(c) SEQ ID No. 3 and SEQ ID No. 14 or 106,
(d) SEQ ID No. 4 and SEQ ID No. 15 or 107,
(e) SEQ ID No. 5 and SEQ ID No. 16 or 108, and
(f) SEQ ID No. 6 and SEQ ID No. 17 or 109, respectively, and
examples of polynucleotides coding the VH and VL constituting the S1-B binding site, respectively, include those coding the VH and VL comprising a pair of the amino acid sequences set forth in (a) SEQ ID No. 7 and SEQ ID No. 18 or 110, or (b) SEQ ID No. 8 and SEQ ID No. 19 or 111, respectively.

On the other hand, examples of polynucleotides coding the VH and VL constituting the S2-C binding site, respectively, include those coding the VH and VL comprising a pair of the amino acid sequences set forth in SEQ ID No. 9 and SEQ ID No. 20 or 112, respectively, and
examples of polynucleotides coding the VH and VL constituting the S2-D binding site, respectively, include those coding the VH and VL comprising a pair of the amino acid sequences set forth in (a) SEQ ID No. 10 and SEQ ID No. 21 or 113, or (b) SEQ ID No. 11 and SEQ ID No. 22 or 114, respectively.

Examples of polynucleotides coding the heavy chain constant region and light chain constant region, respectively, include those coding the amino acid sequence set forth in SEQ ID No. 23 or 99, and SEQ ID No. 24, respectively.

When the C-terminus of the heavy chain or light chain and the scFv or the VH and VL constituting the scFv are linked through a peptide linker, respectively, polynucleotides coding those peptide linkers, respectively, are inserted between the respective polynucleotides coding the heavy chain or light chain and the scFv, or the VH and VL constituting the scFv, respectively, and examples of polynucleotides coding the former peptide linker include one coding the amino acid sequence set forth in SEQ ID No. 73, and examples of polynucleotides coding the latter peptide linker include one coding the amino acid sequence set forth in SEQ ID No. 74.

Preferable examples of polynucleotides coding the anti-Allergin-1 bispecific antibody of the present invention in the form of which the scFv linked to the C-terminus of its heavy chain include those coding the heavy chain and light chain comprising any one pair of the amino acid sequences selected from
(a) SEQ ID No. 75 and 76,
(b) SEQ ID No. 77 and 78,
(c) SEQ ID No. 79 and 78,
(d) SEQ ID No. 80 and 78,
(e) SEQ ID No. 81 and 82,
(f) SEQ ID No. 83 and 82,
(g) SEQ ID No. 84 and 85,
(h) SEQ ID No. 86 and 87,
(i) SEQ ID No. 98 and 78,
(j) SEQ ID No. 100 and 78,
(k) SEQ ID No. 101 and 78,
(l) SEQ ID No. 102 and 78, and
(m) SEQ ID No. 103 and 85, respectively, on the other hand, preferable examples of polynucleotides coding the anti-Allergin-1 bispecific antibody of the present invention in the form of which the scFv linked to the C-terminus of its light chain include those coding the heavy chain and light chain comprising any one pair of the amino acid sequences selected from
   (a) SEQ ID No. 88 and 89,
   (b) SEQ ID No. 90 and 91,
   (c) SEQ ID No. 90 and 92,
   (d) SEQ ID No. 93 and 94,
   (e) SEQ ID No. 93 and 95, and
   (f) SEQ ID No. 96 and 97, respectively. As described above, arginine at position 214 according to the EU numbering system in the heavy chain constant regions of the Allergin-1 bispecific antibodies of the present invention may be substituted by lysine, and/or alanine at position 299 in SEQ ID No. 75, 81, 83, 86, 88, 93 and 96 and at position 301 in SEQ ID No. 80 and 90 (corresponding to position 297 according to the EU numbering system in the heavy chain constant regions) may be substituted by asparagine, each independently.

Herein, as codons encoding one amino acid, one to six types of codons are known, for example, Phe corresponds to TTT or TTC, Leu corresponds to TTA, TTG, CTT, CTC, CTA or CTG, Ile corresponds to ATT, ATC or ATA, Met correspond to ATG, Val corresponds to GTT, GTC, GTA or GTG, Ser corresponds to TCT, TCC, TCA or TCG, Pro corresponds to CCT, CCC, CCA or CCG, Thr corresponds to ACT, ACC, ACA or ACG, Ala corresponds to GCT, GCC, GCA or GCG, Tyr corresponds to TAT or TAC, His corresponds to CAT or CAC, Gln corresponds to CAA or CAG, Asn corresponds to AAT or AAC, Lys corresponds to AAA or AAG, Asp corresponds to GAT or GAC, Glu corresponds to GAA or GAG, Cys corresponds to TGT or TGC, Trp corresponds to TGG, Arg corresponds to CGT, CGC, CGA or CGG, Ser corresponds to AGT or AGC, Arg corresponds to AGA or AGG, and Gly corresponds to GGT, GGC, GGA or GGG, respectively. Accordingly, examples of the polynucleotides encoding parts of the anti-Allergin-1 bispecific antibody include one constituted by which each codon corresponding to each amino acid was arbitrarily combined.

Examples of these polynucleotides also include those in any forms of DNA and RNA.

### [Pharmaceutical Use]

Cancers to which the anti-Allergin-1 bispecific antibody of the present invention can be applied are not limited, and which include all of solid cancers and hematological cancers. Herein, examples of solid cancers include malignant melanoma (e.g., malignant melanoma in skin, oral mucosal epithelium or orbit, etc.), non-small cell lung cancer (e.g., squamous non-small cell lung cancer and non-squamous non-small cell lung cancer), small cell lung cancer, head and neck cancer (e.g., oral cancer, nasopharynx cancer, oropharyngeal cancer, hypopharyngeal cancer, laryngeal cancer, salivary gland cancer and tongue cancer), renal cell cancer (e.g., clear cell renal cell cancer), breast cancer, ovarian cancer (e.g., serous ovarian cancer and ovarian clear cell adenocarcinoma), uterine cancer (e.g., cervical cancer and endometrial cancer), anal cancer (e.g., anal canal cancer), colorectal cancer (e.g., highfrequency microsatellite instability (hereinafter, abbreviated as "MSI-H") and/or mismatch repair defect (hereinafter, abbreviated as "dMMR") positive colorectal cancer), rectal cancer, colon cancer, hepatocellular carcinoma, esophageal cancer, gastric cancer, esophagogastric junction cancer, pancreatic cancer (e.g., pancreatic duct cancer, insulinoma and intraductal papillary mucinous neoplasm), urine urothelial cancer (e.g., bladder cancer, upper urinary tract cancer, ureteral cancer, renal pelvis and urethral cancer), prostate cancer, fallopian tube cancer, primary peritoneal cancer, malignant pleural mesothelium tumor, gallbladder cancer, bile duct cancer, biliary tract cancer, skin cancer (e.g., uveal melanoma and Merkel cell carcinoma), testicular cancer (germ cell tumor), vagina cancer, vulvar cancer, penile cancer, small intestine cancer, endocrine system cancer, thyroid cancer, parathyroid cancer, adrenal cancer, spinal tumor, neuroblastoma, medulloblastoma, retinoblastoma, neuroendocrine tumor, brain tumor (e.g., gliomas (e.g., glioblastoma and gliosarcoma) and meningioma), squamous cell carcinomas and the like.

Furthermore, among solid cancers, examples of sarcomas include bone and soft tissue sarcomas (e.g., Ewing sarcoma, childhood rhabdomyosarcoma, uterine leiomyosarcoma, chondrosarcoma, lung sarcoma, osteosarcoma and congenital fibrosarcoma), Kaposi's sarcoma and the like.

Furthermore, examples of hematological cancers include multiple myeloma, malignant lymphoma (e.g., non-Hodgkin's Lymphoma (e.g., B-cell non-Hodgkin's lymphoma (e.g., precursor B-cell lymphoblastic lymphoma, precursor B-cell acute lymphoblastic leukemia, chronic B-lymphoid leukemia, B-cell precursor leukemia, B-cell prolymphocytic leukemia, lymphoplasmacytic lymphoma, nodal marginal zone B-cell lymphoma, extranodal marginal zone B-cell lymphoma (MALT lymphoma), primary splenic marginal zone B-cell lymphoma, hairy cell leukemia, hairly cell leukemia-variant, follicular lymphoma, pediatric type follicular lymphoma, diffuse large B cell lymphoma, diffuse large B-cell lymphoma, not otherwise specified, splenic diffuse red pulp small B-cell lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM monoclonal gammopathy of undetermined significance, µ heavy chain disease, λ heavy chain disease, α heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, HHV8 positive diffuse large B-cell lymphoma, not otherwise specified, Burkitt-like lymphoma with 11q aberration, high-grade B-cell lymphoma, with MYC and BCL2 and/or BCL6 rearrangement, high-grade B-cell lymphoma, not otherwise specified and B-cell lymphoma, unclassifiable, with intermediate features between diffuse large B-cell lymphoma and classical Hodgkin lymphoma), T/NK-cell non-Hodgkin's lymphoma (e.g., precursor T-cell lymphoblastic lymphoma, chronic T-cell lymphocytic leukemia, T-cell acute lymphocytic leukemia, T-cell large granular lymphoblastic lymphoma, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell leukemia, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the GI tract, hepatosplenic T-cell lymphoma, primary cutaneous CD30 positive T-cell lymphoproliferative disorders, lymphomatoid papulosis, primary cutaneous anaplastic large cell lymphoma, cutaneous T-cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, nodal peripheral T-cell lymphoma with T follicular helper phenotype, anaplastic large cell lymphoma, ALK positive, anaplastic large cell lymphoma, ALK negative and breast implant-associated anaplastic large-cell lymphoma)), and Hodgkin's Lymphoma (e.g., classical Hodgkin's lymphoma (e.g., nodular sclerosis, mixed cellularity, lymphocyte-rich and lymphopenic) and nodular lymphocyte predominant Hodgkin's lymphoma)), leukemia (e.g., acute myelogenous leukemia, acute promyelocytic leukemia, acute lymphoblastic leukemia (lymphoblastic lymphoma), chronic lymphocytic leukemia (small lymphocytic lymphoma), myelodysplastic syndrome, and chronic myelogenous leukemia), primary central nervous system lymphoma, myeloproliferative syndrome and the like.

Furthermore, examples of cancers to which the anti-Allergin-1 bispecific antibody of the present invention can be applied also include pediatric cancer and primary unknown cancer.

Furthermore, the anti-Allergin-1 bispecific antibody of the present invention may be applied to (a) a patient with cancer on which the therapeutic effects of other antineoplastic agents are insufficient or not sufficient or patient with cancer worsened after treatment with other antineoplastic agents, (b) a patient with incurable or unresectable, metastatic, recurrent, refractory and/or distant metastatic cancer, (c) a patient with cancer in which the percentage of PD-L1 expressing tumor cells among tumor cells in tumor tissue (hereinafter, abbreviated as "TPS") or the numerical value obtained by dividing the total number of PD-L1 positive cells (tumor cells, lymphocytes and macrophages) by the total number of tumor cells and multiplying by 100 (hereinafter, abbreviated as "CPS") is 50% or more, 25% or more, 10% or more, 5% or 1% or more, (d) a patient with MSI-H or dMMR cancer (e) a patient with BRAF V600E mutation-positive malignant melanoma or non-small cell lung cancer, (f) a patient with EGFR gene mutation-positive or ALK fusion gene-positive cancer or (g) a patient with cancer with tumor mutation burden (hereinafter, abbreviated as "TMB") high frequency (the number of mutations per 10⁶ bases is 10 or more).

Herein, examples of "other antineoplastic agents" include the antineoplastic agent pertaining to the present invention, that are, an alkylating agent, platinum preparation, antimetabolite antagonist (e.g., antifolate, pyridine metabolism inhibitor and purine metabolism inhibitor), ribonucleotide reductase inhibitor, nucleotide analog, topoisomerase inhibitor, microtubule polymerization inhibitor, microtubule depolymerization inhibitor, antitumor antibiotics, cytokine preparation, anti-hormonal drug, molecular targeting drug, tumor immunotherapeutic drug and other antibody drugs. Furthermore, "the therapeutic effects of antineoplastic agents are insufficient or not sufficient" means, for example, the case to be still determined as "stable (SD)" or "progression (PD)" according to Response Evaluation Criteria In Solid Tumours: RECIST by even treatment with antineoplastic agent.

Furthermore, the anti-Allergin-1 bispecific antibody of the present invention may also be applied to (h) a patient with cancer which has not been treated with any antineoplastic agents, (i) a patient with cancer in which TPS or CPS is less than 50%, less than 25%, less than 10%, less than 5% or less than 1%, (j) a patient with cancer without MSI-H and/or dMMR or with MSI-L, (k) a patient with BRAF V600 wild type malignant melanoma or non-small cell lung cancer, (l) a patient with EGFR gene mutation-negative and/or ALK fusion gene-negative non-small cell lung cancer, or (m) a patient with cancer with TMB low frequency (the number of mutations per 10⁶ bases is less than 10).

Furthermore, the anti-Allergin-1 bispecific antibody of the present invention can be applied as a postoperative adjuvant therapy for preventively suppressing recurrence or metastasis after surgical resection of cancer or preoperative adjuvant therapy performed before surgical resection.

In the present specification, examples of the "treating cancer" include each therapy (a) to decrease the proliferation of tumor cells, (b) to reduce symptoms caused by cancer, (c) to improve the quality of life of a patient with cancer (d) to reduce the dose of other already administered antineoplastic agents or cancer therapeutic adjuvants and/or (e) to prolong the survival of a patient with cancer. The term "suppressing the progress of cancer" means to delay the progress of cancer, to stabilize symptoms associated with cancer, and to reverse the progress of symptoms. The term " suppressing the recurrence of cancer" means to preventively suppress the recurrence of cancer in a patient in which cancer lesion had been completely or substantially eliminated or removed by cancer therapy or cancer resection surgery.

The anti-Allergin-1 bispecific antibody of the present invention is usually administered systemically or locally through parenteral administration. Specific examples of such administration methods include injection administration, intranasal administration, transpulmonary administration, percutaneous administration and the like. Examples of injection administration include intravenous injection, intramuscular injection, intraperitoneal injection and the like. For intravenous injection, drip intravenous infusion is preferable. The dosage thereof varies depending on the age, body weight, symptoms, therapeutic effect, administration method, treating period and the like, but is usually within a range of 0.1 µg/kg to 300 mg/kg per dose for an adult, and particularly preferably within a range of 0.1 mg/kg to 10 mg/kg, once to several times per day by parenteral administration, or within a range of 30 minutes to 24 hours per day by intravenous sustaining administration. Needless to say, as mentioned above, since the dose varies depending on various conditions, it may be lower than the above-mentioned dose, or may be needed to be more than the above.

### [Combination and Combination Preparation]

In order to (a) suppress the progression of, suppress the recurrence of and/or enhance the therapeutic effect on cancer, (b) decrease the dosage of a combined antineoplastic agent, (c) reduce the side effects of a combined antineoplastic agent and/or (d) enhance the immunopotentiation of a combined antineoplastic agent, that is, as an adjuvand, the anti-Allergin-1 bispecific antibody of the present invention may be prescribed in combination with one or more kinds of antineoplastic agents. In the present invention, when it is prescribed in combination with antineoplastic agents, it may be the form of combination drug which both components thereof are mixed in one preparation or that of separated preparations. Such combinations can complement the suppression of the progression of, the suppression of the recurrence of and/or the therapeutic effect on cancer of the antineoplastic agent or maintain or reduce the dosage or frequency of administration thereof. When administering the anti-Allergin-1 bispecific antibody of the present invention and the antineoplastic agent separately, both may be administered simultaneously for a certain period of time, and then only the anti-Allergin-1 bispecific antibody or only the antineoplastic agent may be administered. Alternatively, the anti-Allergin-1 bispecific antibody of the present invention may be administered initially, followed by administration of the antineoplastic agent, or the antineoplastic agent may be administered initially, followed by administration of the anti-Allergin-1 bispecific antibody of the present invention, or during that administration, there may be a certain period for which both may be administered simultaneously. Furthermore, the administration methods of both agents may be the same or different. Depending on the nature of agent, it can also be provided as a pharmaceutical kit containing the Allergin-1 bispecific antibody of the present invention and antineoplastic agent. Herein, the dosage of antineoplastic agent can be appropriately selected based on a dosage clinically used. Furthermore, the antineoplastic agent may be administered in combination of two or more kinds at an appropriate ratio. Furthermore, examples of antineoplastic agents include those which would be found in the future, as well as those which have been found to date.

Examples of other antineoplastic agents include an alkylating drug (e.g., Dacarbazine, Nimustine, Temozolomide, Fotemustine, Bendamustine, Cyclophosphamide, Ifosfamide, Carmustine, Chlorambucil and Procarbazine, etc.), platinum preparation (e.g., Cisplatin, Carboplatin, Nedaplatin and Oxaliplatin, etc.), antimetabolite (e.g., antifolate (e.g., Pemetrexed, Leucovorin and Methotrexate, etc.), pyridine metabolism inhibitor (e.g., TS-1 (registered trademark), 5-fluorouracil, UFT, Carmofur, Doxifluridine, FdUrd, Cytarabine and Capecitabine, etc.), purine metabolism inhibitor (e.g., Fludarabine, Cladribine and Nelarabine, etc.), ribonucleotide reductase inhibitor, nucleotide analog (e.g., Gemcitabine etc.)), topoisomerase inhibitior (e.g., Irinotecan, Nogitecan and Etoposide, etc.), microtubule polymerization inhibitor (e.g., Vinblastine, Vincristine, Vindesine, Vinorelbine and Eribulin, etc.), microtubule depolymerization inhibitor (e.g., Docetaxel and Paclitaxel, etc.), antitumor antibiotics (e.g., Bleomycin, Mitomycin C, Doxorubicin, Daunorubicin, Idarubicin, Etoposide, Mitoxantrone, Vinblastine, Vincristine, Peplomycin, Amrubicin, Aclarubicin and Epirubicin, etc.), cytokine preparation (e.g., IFN-α2a, IFN-α2b, Peg IFN-α2b, natural IFN-β and Interleukin-2, etc.), anti-hormonal drug (e.g., Tamoxifen, Fulvestrant, Goserelin, Leuprorelin, Anastrozole, Letrozole and Exemestane, etc.), molecular targeting drug, tumor immunotherapeutic drug and other antibody drugs and the like. Herein, the antineoplastic agent pertaining to the present invention is preferably one without any concerns of developing cytokine release syndrome when administered by itself alone.

Herein, examples of the molecular targeting drugs include an ALK inhibitor (e.g., Crizotinib, Ceritinib, Ensartinib, Alectinib and Lorlanib, etc.), BCR-ABL inhibitor (e.g., Imatinib and Dasatinib, etc.), EGFR inhibitor (e.g., Erlotinib, EGF816, Afatinib, Osimertinib mesilate, Gefitinib and Rociletinib, etc.), B-RAF inhibitor (e.g., Sorafenib, Vemurafenib, TAK-580, Dabrafenib, Encorafenib, LXH254, Emurafenib and Zanubrutinib, etc.), VEGFR inhibitor (e.g., Bevacizumab, Apatinib, Lenvatinib, Aflibercept and Axitinib, etc.), FGFR inhibitor (e.g., AZD4547, Vofatmab, Roblitinib and Pemigatinib, etc.), c-MET inhibitor (e.g., Savolitinib, Merestinib, Capmatinib, Capmatinib and Glesatinib, etc.), AXL inhibitor (e.g., ONO-7475 and Bemcentinib, etc.), MEK inhibitor (e.g., Cobimetinib, Binimetinib, Selumetinib, and Trametinib, etc.), CDK inhibitor (e.g., Dinaciclib, Abemaciclib, Palbociclib and Trilaciclib, etc.), BTK inhibitor (e.g., Ibrutinib and Acarabultinib, etc.), BCL-2 inhibitor (e.g., Navitoclax and Venetoclax, etc.), PI3K-δ/γ inhibitor (e.g., Umbralisib, Parsaclisib and IPI-549, etc.), JAK-1/2 inhibitior (e.g., Itacitinib and Ruxolitinib, etc.), ERK inhibitor (e.g., SCH 900353 etc.), TGFbR1 inhibitor (e.g., Galunisertib etc.), Cancer cell stemness kinase inhibitor (e.g., Amcasertib etc.), FAK inhibitor (e.g., Defactinib etc.), Syk/FLT3 dual inhibitor (e.g., Mivavotinib etc.), ATR inhibitor (e.g., Ceralasertib etc.), WEE1 kinase inhibitor (e.g., Adavosertib etc.), Multiple tyrosine kinase inhibitor (e.g., Sunitinib, Pazopanib, Cabozantinib, Regorafenib, Nintedanib, Sitravatinib and Midostaurin, etc.), mTOR inhibitor (e.g., Temsirolimus, Everolimus, Vistusertib and Irinotecan, etc.), HDAC inhibitor (e.g., Vorinostat, Romidepsin, Entinostat, Chidamide, Mocetinostat, Citarinostat, Panobinostat and Valproate, etc.), PARP inhibitor (e.g., Niraparib, Olaparib, Veliparib, Rucaparib and Beigene-290, etc.), aromatase inhibitor (e.g., Exemestane and Letrozole, etc.), EZH2 Inhibitor (e.g., Tazemetostat etc.), Galectin-3 inhibitor (e.g., Belapectin etc.), STAT3 inhibitor (e.g., Napabucasin etc.), DNMT Inhibitor (e.g., Azacitidine etc.), SMO Inhibitor (e.g., Vismodegib etc.), HSP90 inhibitor (e.g., XL888 etc.), γ-tubulin specific inhibitor (e.g., Glaziovianin A and Plinabulin, etc.), HIF2α inhibitor (e.g., PT2385 etc.), glutaminase inhibitor (e.g., Telaglenastat etc.), E3 ligase inhibitors inhibitor (e.g., Avadomide etc.), NRF2 activator (e.g., Omaveloxolone etc.), arginase inhibitor (e.g., CB-1158 etc.), cell cycle inhibitor (e.g., Trabectedin etc.), Ephrin B4 inhibitor (e.g., sEphB4-HAS etc.), IAP antagonist (e.g., Birinapant etc.), anti-HER2 antibody (e.g., Pertuzumab, Margetuximab, Disitamab, Disitamab vedotin, Gancotamab, Timigutuzumab, Zanidatamab, Zenocutuzumab, Trastuzumab, Trastuzumab beta, Trastuzumab deruxtecan, Trastuzumab duocarmazine, Trastuzumab emtansine, R48 and ZW33, etc.), anti-HER1 antibody (e.g. Cetuximab, Cetuximab sarotalocan, Panitumumab, Necitumumab, Nimotuzumab, Depatuxizumab, Depatuxizumab mafodotin, Futuximab, Laprituximab, Laprituximab emtansine, Matuzumab, Modotuximab, Petosemtamab, Tomuzotuximab, Losatuxizumab, Losatuxizumab vedotin, Serclutamab, Serclutamab talirine, Imgatuzumab and Zalutumumab, etc.), anti-HER3 antibody (e.g., Duligotuzumab, Elgemtumab, Istiratumab, Lumretuzumab, Zenocutuzumab, Patritumab, Patritumab deruxtecan and Seribantumab, etc.), anti-CD40 antibody (e.g., Bleselumab, Dacetuzumab, Iscalimab, Lucatumumab, Mitazalimab, Ravagalimab, Selicrelumab, Teneliximab, ABBV-428 and APX005M, etc.), anti-CD70 antibody (e.g., Cusatuzumab, Vorsetuzumab, Vorsetuzumab mafodotin and ARGX-110, etc.), anti-VEGF antibody (e.g. Bevacizumab, Bevacizumab beta, Ranibizumab, Abicipar pegol, Aflibercept, Brolucizumab, Conbercept, Dilpacimab, Faricimab, Navicixizumab Varisacumab and IMC-1C11, etc.), anti-VEGFR1 antibody (e.g., Icrucumab etc.), anti-VEGFR2 antibody (e.g., Ramucirumab, Alacizumab, Alacizumab pegol, Olinvacimab, Pegdinetanib and AMG 596, etc.), anti-CD20 antibody (e.g., Rituximab, Blontuvetmab, Epitumomab, Ibritumomab tiuxetan, Ocaratuzumab, Ocrelizumab, Technetium (⁹⁹mTc) nofetumomab merpentan, Tositumomab, Veltuzumab, Ofatumumab, Ublituximab, Obinutuzumab and Nofetumomab, etc.), anti-CD30 antibody (e.g., Brentuximab vedotin and Iratumumab, etc.), anti-CD38 antibody (e.g., Daratumumab, Isatuximab, Mezagitamab, AT13/5 and MOR202, etc.), anti-TNFRSF10B antibody (e.g., Benufutamab, Conatumumab, Drozitumab, Lexatumumab, Tigatuzumab, Eftozanermin alfa and DS-8273a, etc.), anti-TNFRSF 10A antibody (e.g., Mapatumumab etc.), anti-MUC1 antibody (e.g., Cantuzumab, Cantuzumab ravtansine, Clivatuzumab, Clivatuzumab tetraxetan, Yttrium (⁹⁰Y) clivatuzumab tetraxetan, Epitumomab Epitumomab cituxetan, Sontuzumab, Gatipotuzumab, Nacolomab, Nacolomab tafenatox, 7F11C7, BrE-3, CMB-401, CTM01 and HMFG1, etc.), anti-MUC5AC antibody (e.g. Ensituximab etc.), anti-MUC16 antibody (e.g., Oregovomab, Abagovomab, Igovomab, and Sofituzumab vedotin, etc.), anti-DLL4 antibody (e.g., Demcizumab, Dilpacimab, Navicixizumab and Enoticumab, etc.), anti-fucosyl GM1 antibody (e.g., BMS-986012 etc.), anti-gpNMB antibody (e.g., Glembatumumab vedotin etc.), anti-mesothelin antibody (e.g., Amatuximab, Anetumab ravtansine Anetumab corixetan, RG7784, and BMS-986148, etc.), anti-MMP9 antibody (e.g., Andecaliximab etc.), anti-GD2 antibody (e.g., Dinutuximab, Dinutuximab beta, Lorukafusp alfa, Naxitamab, 14G2a, MORAb-028, Surek, TRBs07 and ME361, etc.), anti-MET antibody (e.g., Emibetuzumab, Onartuzumab, Telisotuzumab and Telisotuzumab vedotin, etc.), anti-FOLR1 antibody (e.g., Farletuzumab, Mirvetuximab and Mirvetuximab soravtansine, etc.), anti-CD79b antibody (e.g., Iladatuzumab, Iladatuzumab vedotin and Polatuzumab vedotin, etc.), anti-DLL3 antibody (e.g., Rovalpituzumab and Rovalpituzumab Tesirine, etc.), anti-CD51 antibody (e.g., Abituzumab, Etaracizumab and Intetumumab, etc.), anti-EpCAM antibody (e.g., Adecatumumab Catumaxomab, Edrecolomab, Oportuzumab monatox, Citatuzumab bogatox and Tucotuzumab celmoleukin, etc.), anti-CEACAM5 antibody (e.g., Altumomab, Arcitumomab Cergutuzumab amunaleukin, Labetuzumab, Labetuzumab govitecan, ⁹⁰Y-cT84.66, AMG211, BW431/26, CE25/B7, COL-1 and T84.66 M5A, etc.), anti-CEACAM6 antibody (e.g. Tinurilimab etc.), anti-FGFR2 antibody (e.g., Aprutumab, Aprutumab ixadotin and Bemarituzumab, etc.), anti-CD44 antibody (e.g., Bivatuzumab mertansine etc.), anti-PSMA antibody (e.g., Indium (¹¹¹In) capromab pendetide, ¹⁷⁷Lu-J591 and ES414, etc.), anti-Endoglin antibody (e.g., Carotuximab etc.), anti-IGF1R antibody (e.g., Cixutumumab, Figitumumab, Ganitumab, Dalotuzumab, Teprotumumab, and Robatumumab, etc.), anti-TNFSF11 antibody (e.g., Denosumab etc.), anti-GUCY2C antibody (e.g., Indusatumumab vedotin etc.), anti-SLC39A6 antibody (e.g., Ladiratuzumab vedotin etc.), anti-SLC34A2 antibody (e.g., Lifastuzumab vedotin etc.), anti-NCAM1 antibody (e.g., Lorvotuzumab mertansine etc.), anti-ganglioside GD3 antibody (e.g., Ecromeximab and Mitumomab, etc.), anti-AMHR2 antibody (e.g., Murlentamab etc.), anti-CD37 antibody (e.g., Lilotomab, Lutetium (¹⁷⁷lu) lilotomab satetraxetan, Naratuximab, Naratuximab emtansine and Otlertuzumab, etc.), anti-IL1RAP antibody (e.g., Nidanilimab etc.), anti-PDGFR2 antibody (e.g., Olaratumab and Tovetumab, etc.), anti-CD200 antibody (e.g., Samalizumab etc.), anti-TAG-72 antibody (e.g., Anatumomab mafenatox, Minretumomab, Indium (¹¹¹In) satumomab pendetide, CC49, HCC49 and M4, etc.), anti-SLITRK6 antibody (e.g., Sirtratumab vedotin etc.), anti-DPEP3 antibody (e.g., Tamrintamab pamozirine etc.), anti-CD19 antibody (e.g., Axicabtagene ciloleucel, Coltuximab ravtansine, Denintuzumab mafodotin, Inebilizumab, Loncastuximab, Loncastuximab tesirine, Obexelimab, Tafasitamab, Taplitumomab paptox and huAnti-B4, etc.), anti-NOTCH2/3 antibody (e.g. Tarextumab, etc.), anti-tenascin C antibody (e.g., Tenatumomab etc.), anti-AXL antibody (e.g., Enapotamab, Enapotamab vedotin and Tilvestamab, etc.), anti-STEAP1 antibody (e.g., Vandortuzumab vedotin etc.), anti-CTAA16 antibody (e.g., technetium (⁹⁹mTc) votumumab etc.), anti-CLDN18 antibody (e.g., Zolbetuximab etc.), anti-GM3 antibody (e.g., Racotumomab, FCGR1 and H22, etc.), anti-PSCA antibody (e.g. MK-4721 etc.), anti-FN extra domain B antibody (e.g., AS1409 etc.), anti-HAVCR1 antibody (e.g., CDX-014 etc.), anti-TNFRSF4 antibody (e.g., MEDI6383 etc.), anti-HER1-MET bispecific antibody (e.g., Amivantamab etc.), anti-EpCAM-CD3 bispecific antibody (e.g., Solitomab and Catumaxomab, etc.), anti-Ang2-VEGF bispecific antibody (e.g., Vanucizumab etc.), anti-HER2-CD3 bispecific antibody (e.g., Ertumaxomab etc.), anti-HER3-IGF1R bispecific antibody (e.g., Istiratumab etc.), anti-PMSA-CD3 bispecific antibody (e.g., Pasotuxizumab etc.), anti-HER1-LGR5 bispecific antibody (e.g., Petosemtamab etc.), anti-SSTR2-CD3 bispecific antibody (e.g., Tidutamab etc.), anti-CD30-CD16A bispecific antibody (e.g., AFM13 etc.), anti-CEA-CD3 bispecific antibody (e.g., Cibisatamab and RO6958688, etc.), anti-CD3-CD19 bispecific antibody (e.g., Duvortuxizumab and Blinatumomab, etc.), anti-IL3RA-CD3 bispecific antibody (e.g., Flotetuzumab and Vibecotamab, etc.), anti-GPRC5D-CD3 bispecific antibody (e.g., Talquetamab etc.), anti-CD20-CD3 bispecific antibody (e.g., Plamotamab, Odronextamab, Mosunetuzumab, Glofitamab, Epcoritamab and REGN1979, etc.), anti-TNFRSF17-CD3 bispecific antibody (e.g., Teclistamab etc.), anti-CLEC12A-CD3 bispecific antibody (e.g., Tepoditamab etc.), anti-HER2-HER3 bispecific antibody (e.g., Zenocutuzumab etc.), anti-FAP antibody/IL-2 fusion protein (e.g., RO6874281 etc.), anti-CEA antibody/IL-2 fusion protein (e.g., Cergutuzumab amunaleukin etc.) and the like.

Furthermore, examples of the tumor immunotherapeutic drugs include an anti-PD-1 antibody (e.g., Nivolumab, Cemiplimab, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, Geptanolimab, AMP-514, STI-A1110, ENUM 388D4, ENUM 244C8, Zimberelimab, CS1003, BAT-1306, Penpulimab, AK103, Ezabenlimab, LZM009, CMAB819, Sym021, SSI-361, JY034, Pucotenlimab, ISU106 and CX-188, etc.), anti-PD-L1 antibody (e.g., Atezolizumab, Avelumab, Durvalumab, Manelimab, Pacmilimab, Envafolimab, Cosibelimab, Sugemalimab, BMS-936559, STI-1014, HLX20, Adebrelimab, MSB2311, BGB-A333, KL-A167, AK106, Socazolimab, FAZ053, CBT-502 and JS003, etc.), PD-1 antagonist (e.g., AUNP-12, the respective compounds of BMS-M1 to BMS-M10 (see WO2014/151634, WO2016/039749, WO2016/057624, WO2016/077518, WO2016/100285, WO2016/100608, WO2016/126646, WO2016/149351, WO2017/151830 and WO2017/176608), BMS-1, BMS-2, BMS-3, BMS-8, BMS-37, BMS-200, BMS-202, BMS-230, BMS-242, BMS-1001 and BMS-1166 (see WO2015/034820, WO2015/160641, WO2017/066227 and Oncotarget. 2017 Sep 22; 8(42): 72167-72181.), the respective compounds of Incyte-1 to Incyte-6 (see WO2017/070089, WO2017/087777, WO2017/106634, WO2017/112730, WO2017/192961 and WO2017/205464), the respective compounds of CAMC-1 to CAMC-4 (see WO2017/202273, WO2017/202274, WO2017/202275 and WO2017/202276), RG_1 (see WO2017/118762) and DPPA-1 (see Angew. Chem. Int. Ed. 2015, 54, 11760-11764), etc.), PD-L1/VISTA antagonist (e.g., CA-170 etc.), PD-L1/TIM3 antagonist (e.g., CA-327 etc.), anti-PD-L2 antibody, PD-L1 fusion protein, PD-L2 fusion protein (e.g., AMP-224 etc.), anti-CTLA-4 antibody (e.g., Ipilimumab, Zalifrelimab, Nurulimab and Tremelimumab, etc.), anti-LAG-3 antibody (e.g., Relatlimab, Ieramilimab, Fianlimab, Encelimab and Mavezelimab, etc.), anti-TIM3 antibody (e.g., MBG453 and Cobolimab, etc.), anti-KIR antibody (e.g., Lirilumab, IPH2101, LY3321367 and MK-4280, etc.), anti-BTLA antibody, anti-TIGIT antibody (e.g., Tiragolumab, Etigilimab, Vibostolimab and BMS-986207, etc.), anti-VISTA antibody (e.g., Onvatilimab etc.), anti-CD137 antibody (e.g., Urelumab and Utomilumab, etc.), anti-CSF-1R antibody or CSF-1R inhibitor (e.g., Cabiralizumab, Ecactuzumab, LY3022855, Axatilimab, MCS-110, IMC-CS4, AMG820, Pexidartinib, BLZ945 and ARRY-382, etc.), anti-OX40 antibody (e.g., MEDI6469, Ivuxolimab, MEDI0562, MEDI6383, Efizonerimod, GSK3174998, BMS-986178 and MOXR0916, etc.), anti-HVEM antibody, anti-CD27 antibody (e.g., Varlilumab etc.), anti-GITR antibody or GITR fused protein (e.g., Efaprinermin alfa, Efgivanermin alfa, MK-4166, INCAGN01876, GWN323 and TRX-518, etc.), anti-CD28 antibody, anti-CCR4 antibody (e.g., Mogamulizumab etc.), anti-B7-H3 antibody (e.g., Enoblituzumab, Mirzotamab, Mirzotamab clezutoclax and Omburtamab, etc.), anti-ICOS agonist antibody (e.g., Vopratelimab and GSK3359609, etc.), anti-CD4 antibody (e.g., Zanolimumab and IT1208, etc.), anti-DEC-205 antibody/NY-ESO-1 fusion protein (e.g., CDX-1401 etc.), anti-SLAMF7 antibody (e.g., Azintuxizumab, Azintuxizumab vedotin and Elotuzumab, etc.), anti-CD73 antibody (e.g., Oleclumab and BMS-986179, etc.), pegylated IL-2 (Bempegaldesleukin), IDO inhibitor (e.g., Epacadostat, Indoximod and Linrodostat, etc.), TLR agonist (e.g., Motolimod, CMP-001, G100, Tilsotolimod, SD-101 and MEDI9197, etc.), adenosine A2A receptor antagonist (e.g., Preladenant, AZD 635, Taminadenant and Ciforadenant, etc.), anti-NKG2A antibody (e.g., Monalizazumab etc.), anti-CSF-1 antibody (e.g., PD036032 etc.), immunopotentiating agent (e.g., PV-10 etc.), IL-15 superagonist (e.g., ALT-803 etc.), soluble LAG3 (e.g., Eftilagimod alpha etc.), anti-CD47 antibody or CD47 antagonist (e.g., ALX148 etc.), IL-12 antagonist (e.g., M9241 etc.) and the like. Herein, Nivolumab can be produced according to the method described in WO2006/121168, Pembrolizumab can be produced according to the method described in WO2008/156712, BMS-936559 can be produced according to the method described in WO2007/005874, and Ipilimumab can be produced according to the method described in WO2001/014424.

Furthermore, examples of other antibody drugs include an anti-IL-1β antibody (e.g., Canakinumab etc.), anti-CCR2 antibody (e.g., Plozalizumab etc.) and the like.

When used in combination with the anti-Allergin-1 bispecific antibody of the present invention, the tumor immunotherapeutic drug pertaining to the present invention can be administered, for example, in the following usage and dosage. That is, it can be intravenously administered (e.g., intravenous drip infusion) at about 1 to 21 mg/kg (body weight) per dose or about 80 to 1680 mg per dose, of an active ingredient of each tumor immunotherapeutic drug, every 1 to 8 weeks, over about 30 minutes to about 60 minutes or about 60 minutes or more. Herein, examples of the single dosages based on body weight include 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 12 mg/kg, 14 mg/kg, 15 mg/kg, 20 mg/kg and 21 mg/kg while examples of the single dosages include 200 mg, 240 mg, 250 mg, 280 mg, 300 mg, 320 mg, 350 mg, 360 mg, 400 mg, 420 mg, 450 mg, 480 mg, 500 mg, 540 mg, 560 mg, 600 mg, 640 mg, 700 mg, 720 mg, 750 mg, 800 mg, 840 mg, 900 mg, 1000 mg, 1080 mg, 1100 mg, 1120 mg, 1200 mg, 1600 mg and 1680 mg. Examples of the administration intervals include 1 week, 2 weeks, 3 weeks, 4 weeks, 5 week, 6 weeks, 7 weeks and 8 weeks and examples of the single dosing times include about 30 minutes, about 60 minutes or about 60 minutes or more.

Herein, if the active ingredient of tumor immunotherapeutic drug is the anti-PD-1 antibody Nivolumab, and which is used in combination with the anti-Allergin-1 bispecific antibody of the present invention, it can be administered to an adult at (a) 1 mg/kg (body weight) per dose every 3 weeks, (b) 3 mg/kg (body weight) per dose every 2 weeks, (c) 2 mg/kg (body weight) per dose every 3 weeks, (d) 80 mg per dose every 3 weeks, (e) 240 mg per dose every 2 weeks, (f) 360 mg per dose every 3 weeks or (g) 480 mg per dose every 4 weeks, of Nivolumab, (more preferably 240 mg per dose every 2 weeks, 360 mg per dose every 3 weeks or 480 mg per dose every 4 weeks) by intravenous drip infusion.

If the active ingredient of tumor immunotherapeutic drug is the anti-PD-1 antibody Pembrolizumab, and which is used in combination with the anti-Allergin-1 bispecific antibody of the present invention, it can be administered to an adult at (a) 200 mg per dose every 3 weeks, (b) 400 mg per dose every 6 weeks or (c) 2 mg/kg (body weight) per dose (up to 200 mg per dose) every 3 weeks, of Pembrolizumab, by intravenous drip infusion.

If the active ingredient of tumor immunotherapeutic drug is the anti-PD-1 antibody Cemiplimab-rwlc, and which is used in combination with the anti-Allergin-1 bispecific antibody of the present invention, it can be administered to an adult at 350 mg of Cemiplimab-rwlc per dose every 3 weeks by intravenous drip infusion. Specially, to a patient with spinous cell carcinoma, it can be administered at the same usage and dosage as mentioned above.

If the active ingredient of tumor immunotherapeutic drug is the anti-PD-1 antibody Dostarlimab, and which is used in combination with the anti-Allergin-1 bispecific antibody of the present invention, it can be administered to an adult at 500 mg of Dostarlimab per dose every 3 weeks, 4 times, and from the fifth dose onwards, which is 3 weeks later, at 1000 mg per dose every 6 weeks, by intravenous drip infusion. Specially, to a patient with dMMR-positive endometrial cancer or solid cancer, it can be administered at the same usage and dosage as mentioned above.

If the active ingredient of tumor immunotherapeutic drug is the anti-PD-L1 antibody Avelumab, and which is used in combination with the anti-Allergin-1 bispecific antibody of the present invention, it can be administered to an adult at 10 mg/kg (body weight) of Avelumab per dose every 2 weeks by intravenous drip infusion.

If the active ingredient of tumor immunotherapeutic drug is the anti-PD-L1 antibody Atezolizumab, and which is used in combination with the anti-Allergin-1 bispecific antibody of the present invention, it can be administered to an adult at (a) 840 mg per dose every 2 weeks, (b) 1200 mg per dose every 3 weeks or (c) 1680 mg per dose every 4 weeks, of Atezolizumab, by intravenous drip infusion.

If the active ingredient of tumor immunotherapeutic drug is the anti-PD-L1 antibody Durvalumab, and which is used in combination with the anti-Allergin-1 bispecific antibody of the present invention, it can be administered to an adult at 10 mg/kg (body weight) of Durvalumab per dose every 2 weeks by intravenous drip infusion.

If the active ingredient of tumor immunotherapeutic drug is the anti-CTLA-4 antibody Ipilimumab, and which is used in combination with the anti-Allergin-1 bispecific antibody of the present invention, it can be administered to an adult at (a) 3 mg/kg (body weight) per dose daily or (b) 1 mg/kg (body weight) per dose daily, of Ipilimumab, four times every 3 weeks by intravenous drip infusion.

### [Formulation]

When the anti-Allergin-1 bispecific antibody of the present invention is formulated to be used as an injection or infusion solution for drip infusion, the injection or infusion solution may be in any form of an aqueous solution, suspension or emulsion, or may be formulated as a solid agent along with pharmaceutically acceptable carriers so that it can be dissolved, suspended or emulsified by adding a solvent at the time of use. Examples of solvents which can be used in the injection or the infusion solution for drip infusion include distilled water for injection, physiological saline, glucose solutions and isotonic solutions and the like (e.g., solutions in which sodium chloride, potassium chloride, glycerin, mannitol, sorbitol, boric acid, borax, propylene glycol or the like is dissolved.).

Herein, examples of the pharmaceutically acceptable carriers include a stabilizer, solubilizer, suspending agent, emulsifier, soothing agent, buffering agent, preservative, antiseptic agent, pH adjuster, antioxidant and the like. As examples of the stabilizers, various amino acids, albumin, globulin, gelatin, mannitol, glucose, dextran, ethylene glycol, propylene glycol, polyethylene glycol, ascorbic acid, sodium bisulfite, sodium thiosulfate, sodium edetate, sodium citrate, dibutylhydroxytoluene or the like can be used. As examples of the solubilizers, alcohol (e.g., ethanol etc.), polyalcohol (e.g., propylene glycol and polyethylene glycol, etc.), nonionic surfactant (e.g., Polysorbate 20 (registered trademark), Polysorbate 80 (registered trademark) and HCO-50, etc.) or the like can be used. As examples of the suspending agents, glyceryl monostearate, aluminum monostearate, methyl cellulose, carboxymethyl cellulose, hydroxymethyl cellulose, sodium lauryl sulfate or the like can be used. As examples of the emulsifiers, gum arabic, sodium alginate, tragacanth or the like can be used. As examples of the soothing agents, benzyl alcohol, chlorobutanol, sorbitol or the like can be used. As examples of the buffering agents, phosphate buffer, acetate buffer, borate buffer, carbonate buffer, citrate buffer, Tris buffer, glutamic acid buffer, epsilon aminocaproic acid buffer or the like can be used. As examples of the preservatives, methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edeate, boric acid, borax or the like can be used. As examples of the antiseptic agents, benzalkonium chloride, parahydroxybenzoic acid, chlorobutanol or the like can be used. As examples of the pH adjusters, hydrochloric acid, sodium hydroxide, phosphoric acid, acetic acid or the like can be used. As examples of the antioxidants, (1) aqueous antioxidants such as ascorbic acid, cysteine hydrochloride, sodium bisulfate, sodium metabisulfite and sodium sulfite, (2) oil-soluble antioxidants such as ascorbyl palmitate, butylated hydroxy anisole, butylated hydroxyl toluene, lecithin, propyl gallate and α-tocopherol, and (3) metal chelating agents such as citric acid, ethylenediaminetetraacetic acid, sorbitol, tartaric acid and phosphoric acid can be used.

The injection or infusion solution for drip infusion can be produced by performing sterilization in the final process, or aseptic manipulation, for example, sterilization by filtration with a filter, and subsequently filling an aseptic container. The injection or infusion solution for drip infusion may be used by dissolving the vacuum dried and lyophilized aseptic powder (which may include pharmaceutically acceptable carrier powders) in an appropriate solvent at the time of use.

The present invention provides embodiments listed in the following [1] to [150], [1-1] and [1-2], [2-1] to [2-32], [3-1] to [3-3], [4-1], [5-1], [6-1], [7-1] to [7-20] and [8-1] to [8-3], respectively.
[1] A bispecific antibody or an antibody fragment thereof, having antigen-binding sites specifically binding to two different epitopes on the extracellular domains of Allergin-1, respectively,
   wherein the antigen-binding sites do not mutually compete for Allergin-1, and are any two different antigen-binding sites selected from
      (i) an antigen-binding site specifically binding to a specific epitope on the S1 domain of Allergin-1 (S1-A binding site),
      (ii) an antigen-binding site specifically binding to another epitope on the S1 domain different from the epitope described in the preceding item (i) (S1-B binding site),
      (iii) an antigen-binding site specifically binding to a specific epitope on the S2 domain of Allergin-1 (S2-C binding site), and
      (iv) an antigen-binding site specifically binding to another epitope on the S2 domain different from the epitope described in the preceding item (iii) (S2-D binding site), and
   wherein the S1-A binding site is any one selected from
      (A) an antigen-binding site comprising the VH having
         (a) the VH-CDR1 comprising the amino acid sequence set forth in SEQ ID No. 25,
         (b) the VH-CDR2 comprising the amino acid sequence set forth in SEQ ID No. 26, and
         (c) the VH-CDR3 comprising the amino acid sequence set forth in SEQ ID No. 27, and
            the VL having
         (d) the VL-CDR1 comprising the amino acid sequence set forth in SEQ ID No. 49,
         (e) the VL-CDR2 comprising the amino acid sequence set forth in SEQ ID No. 50, and
         (f) the VL-CDR3 comprising the amino acid sequence set forth in SEQ ID No. 51,
      (B) an antigen-binding site comprising the VH having
         (a) the VH-CDR1 comprising the amino acid sequence set forth in SEQ ID No. 28,
         (b) the VH-CDR2 comprising the amino acid sequence set forth in SEQ ID No. 29, and
         (c) the VH-CDR3 comprising the amino acid sequence set forth in SEQ ID No. 30, and
            the VL having
         (d) the VL-CDR1 comprising the amino acid sequence set forth in SEQ ID No. 52,
         (e) the VL-CDR2 comprising the amino acid sequence set forth in SEQ ID No. 53, and
         (f) the VL-CDR3 comprising the amino acid sequence set forth in SEQ ID No. 54,
      (C) an antigen-binding site comprising the VH having
         (a) the VH-CDR1 comprising the amino acid sequence set forth in SEQ ID No. 31,
         (b) the VH-CDR2 comprising the amino acid sequence set forth in SEQ ID No. 32, and
         (c) the VH-CDR3 comprising the amino acid sequence set forth in SEQ ID No. 33, and
         the VL having:
         (d) the VL-CDR1 comprising the amino acid sequence set forth in SEQ ID No. 55,
         (e) the VL-CDR2 comprising the amino acid sequence set forth in SEQ ID No. 56, and
         (f) the VL-CDR3 comprising the amino acid sequence set forth in SEQ ID No. 57, and
      (D) an antigen-binding site comprising the VH having
         (a) the VH-CDR1 comprising the amino acid sequence set forth in SEQ ID No. 34,
         (b) the VH-CDR2 comprising the amino acid sequence set forth in SEQ ID No. 35, and
         (c) the VH-CDR3 comprising the amino acid sequence set forth in SEQ ID No. 36, and
            the VL having
         (d) the VL-CDR1 comprising the amino acid sequence set forth in SEQ ID No. 58,
         (e) the VL-CDR2 comprising the amino acid sequence set forth in SEQ ID No. 59, and
         (f) the VL-CDR3 comprising the amino acid sequence set forth in SEQ ID No. 60,
         the S1-B binding site is
      (E) an antigen-binding site comprising the VH having
         (a) the VH-CDR1 comprising the amino acid sequence set forth in SEQ ID No. 37,
         (b) the VH-CDR2 comprising the amino acid sequence set forth in SEQ ID No. 38, and
         (c) the VH-CDR3 comprising the amino acid sequence set forth in SEQ ID No. 39, and
            the VL having
         (d) the VL-CDR1 comprising the amino acid sequence set forth in SEQ ID No. 61,
         (e) the VL-CDR2 comprising the amino acid sequence set forth in SEQ ID No. 62, and
         (f) the VL-CDR3 comprising the amino acid sequence set forth in SEQ ID No. 63, or
      (F) an antigen-binding site comprising the VH having
         (a) the VH-CDR1 comprising the amino acid sequence set forth in SEQ ID No. 40,
         (b) the VH-CDR2 comprising the amino acid sequence set forth in SEQ ID No. 41, and
         (c) the VH-CDR3 comprising the amino acid sequence set forth in SEQ ID No. 42, and
         the VL having:
         (d) the VL-CDR1 comprising the amino acid sequence set forth in SEQ ID No. 64,
         (e) the VL-CDR2 comprising the amino acid sequence set forth in SEQ ID No. 65, and
         (f) the VL-CDR3 comprising the amino acid sequence set forth in SEQ ID No. 66,
   the S2-C binding site is an antigen-binding site comprising the VH having
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
         the VL having
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69, and
   the S2-D binding site is an antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
         the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72;
[2] a bispecific antibody to Allergin-1 or an antibody fragment thereof, comprising any two different antigen-binding sites selected from the S 1-A binding site, the S1-B binding site, the S2-C binding site and the S2-D binding site,
   wherein the S1-A binding site is any one selected from
      (A) an antigen-binding site comprising the VH having
         (a) the VH-CDR1 comprising the amino acid sequence set forth in SEQ ID No. 25,
         (b) the VH-CDR2 comprising the amino acid sequence set forth in SEQ ID No. 26, and
         (c) the VH-CDR3 comprising the amino acid sequence set forth in SEQ ID No. 27, and
            the VL having
         (d) the VL-CDR1 comprising the amino acid sequence set forth in SEQ ID No. 49,
         (e) the VL-CDR2 comprising the amino acid sequence set forth in SEQ ID No. 50, and
         (f) the VL-CDR3 comprising the amino acid sequence set forth in SEQ ID No. 51,
      (B) an antigen-binding site comprising the VH having
         (a) the VH-CDR1 comprising the amino acid sequence set forth in SEQ ID No. 28,
         (b) the VH-CDR2 comprising the amino acid sequence set forth in SEQ ID No. 29, and
         (c) the VH-CDR3 comprising the amino acid sequence set forth in SEQ ID No. 30, and
            the VL having
         (d) the VL-CDR1 comprising the amino acid sequence set forth in SEQ ID No. 52,
         (e) the VL-CDR2 comprising the amino acid sequence set forth in SEQ ID No. 53, and
         (f) the VL-CDR3 comprising the amino acid sequence set forth in SEQ ID No. 54,
      (C) an antigen-binding site comprising the VH having
         (a) the VH-CDR1 comprising the amino acid sequence set forth in SEQ ID No. 31,
         (b) the VH-CDR2 comprising the amino acid sequence set forth in SEQ ID No. 32, and
         (c) the VH-CDR3 comprising the amino acid sequence set forth in SEQ ID No. 33, and
         the VL having:
         (d) the VL-CDR1 comprising the amino acid sequence set forth in SEQ ID No. 55,
         (e) the VL-CDR2 comprising the amino acid sequence set forth in SEQ ID No. 56, and
         (f) the VL-CDR3 comprising the amino acid sequence set forth in SEQ ID No. 57, and
      (D) an antigen-binding site comprising the VH having
         (a) the VH-CDR1 comprising the amino acid sequence set forth in SEQ ID No. 34,
         (b) the VH-CDR2 comprising the amino acid sequence set forth in SEQ ID No. 35, and
         (c) the VH-CDR3 comprising the amino acid sequence set forth in SEQ ID No. 36, and
            the VL having
         (d) the VL-CDR1 comprising the amino acid sequence set forth in SEQ ID No. 58,
         (e) the VL-CDR2 comprising the amino acid sequence set forth in SEQ ID No. 59, and
         (f) the VL-CDR3 comprising the amino acid sequence set forth in SEQ ID No. 60,
         the S1-B binding site is
      (E) an antigen-binding site comprising the VH having
         (a) the VH-CDR1 comprising the amino acid sequence set forth in SEQ ID No. 37,
         (b) the VH-CDR2 comprising the amino acid sequence set forth in SEQ ID No. 38, and
         (c) the VH-CDR3 comprising the amino acid sequence set forth in SEQ ID No. 39, and
            the VL having
         (d) the VL-CDR1 comprising the amino acid sequence set forth in SEQ ID No. 61,
         (e) the VL-CDR2 comprising the amino acid sequence set forth in SEQ ID No. 62, and
         (f) the VL-CDR3 comprising the amino acid sequence set forth in SEQ ID No. 63, or
      (F) an antigen-binding site comprising the VH having
         (a) the VH-CDR1 comprising the amino acid sequence set forth in SEQ ID No. 40,
         (b) the VH-CDR2 comprising the amino acid sequence set forth in SEQ ID No. 41, and
         (c) the VH-CDR3 comprising the amino acid sequence set forth in SEQ ID No. 42, and
         the VL having:
         (d) the VL-CDR1 comprising the amino acid sequence set forth in SEQ ID No. 64,
         (e) the VL-CDR2 comprising the amino acid sequence set forth in SEQ ID No. 65, and
         (f) the VL-CDR3 comprising the amino acid sequence set forth in SEQ ID No. 66,
   the S2-C binding site is an antigen-binding site comprising the VH having
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
         the VL having
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69, and
   the S2-D binding site is an antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
         the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72;
[3] the bispecific antibody or antibody fragment thereof according to the preceding item [1] or [2], wherein one to five arbitrary amino acid residues in any one or more CDRs of VH-CDR1, VH-CDR2, VH-CDR3, VL-CDR1, VL-CDR2 and VL-CDR3 constituting any two different antigen-binding sites selected from the S 1-A binding site, the S1-B binding site, the S2-C binding site and the S2-D binding site, respectively, are substituted with other amino acids, respectively;
[4] the bispecific antibody or antibody fragment thereof according to the preceding item [1] or [2], wherein the two different antigen-binding sites are the S 1-A binding site and the S 1-B binding site,
   wherein (i) the S 1-A binding site is the antigen-binding site comprising the VH having
      (a) the VH-CDR1 comprising the amino acid sequence set forth in SEQ ID No. 25,
      (b) the VH-CDR2 comprising the amino acid sequence set forth in SEQ ID No. 26, and
      (c) the VH-CDR3 comprising the amino acid sequence set forth in SEQ ID No. 27, and
         the VL having
      (d) the VL-CDR1 comprising the amino acid sequence set forth in SEQ ID No. 49,
      (e) the VL-CDR2 comprising the amino acid sequence set forth in SEQ ID No. 50, and
      (f) the VL-CDR3 comprising the amino acid sequence set forth in SEQ ID No. 51,
      and
   (ii) the S1-B binding site is the antigen-binding site comprising the VH having
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39, and
         the VL having
      (d) the VL-CDR1 comprising the amino acid sequence set forth in SEQ ID No. 61,
      (e) the VL-CDR2 comprising the amino acid sequence set forth in SEQ ID No. 62, and
      (f) the VL-CDR3 comprising the amino acid sequence set forth in SEQ ID No. 63;
[5] the bispecific antibody or antibody fragment thereof according to the preceding item [1] or [2], wherein the two different antigen-binding sites are the S 1-A binding site and the S 1-B binding site,
   wherein (i) the S 1-A binding site is the antigen-binding site comprising the VH having
      (a) the VH-CDR1 comprising the amino acid sequence set forth in SEQ ID No. 25,
      (b) the VH-CDR2 comprising the amino acid sequence set forth in SEQ ID No. 26, and
      (c) the VH-CDR3 comprising the amino acid sequence set forth in SEQ ID No. 27, and
         the VL having
      (d) the VL-CDR1 comprising the amino acid sequence set forth in SEQ ID No. 49,
      (e) the VL-CDR2 comprising the amino acid sequence set forth in SEQ ID No. 50, and
      (f) the VL-CDR3 comprising the amino acid sequence set forth in SEQ ID No. 51,
      and
   (ii) the S1-B binding site is the antigen-binding site comprising the VH having
      (a) the VH-CDR1 comprising the amino acid sequence set forth in SEQ ID No. 40,
      (b) the VH-CDR2 comprising the amino acid sequence set forth in SEQ ID No. 41, and
      (c) the VH-CDR3 comprising the amino acid sequence set forth in SEQ ID No. 42, and
         the VL having
      (d) the VL-CDR1 comprising the amino acid sequence set forth in SEQ ID No. 64,
      (e) the VL-CDR2 comprising the amino acid sequence set forth in SEQ ID No. 65, and
      (f) the VL-CDR3 comprising the amino acid sequence set forth in SEQ ID No. 66;
[6] the bispecific antibody or antibody fragment thereof according to the preceding item [1] or [2], wherein the two different antigen-binding sites are the S 1-A binding site and the S 1-B binding site,
   wherein (i) the S 1-A binding site is the antigen-binding site comprising the VH having
      (a) the VH-CDR1 comprising the amino acid sequence set forth in SEQ ID No. 28,
      (b) the VH-CDR2 comprising the amino acid sequence set forth in SEQ ID No. 29, and
      (c) the VH-CDR3 comprising the amino acid sequence set forth in SEQ ID No. 30, and
         the VL having
      (d) the VL-CDR1 comprising the amino acid sequence set forth in SEQ ID No. 52,
      (e) the VL-CDR2 comprising the amino acid sequence set forth in SEQ ID No. 53, and
      (f) the VL-CDR3 comprising the amino acid sequence set forth in SEQ ID No. 54,
      and
   (ii) the S1-B binding site is the antigen-binding site comprising the VH having
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39, and
      the VL having:
      (d) the VL-CDR1 comprising the amino acid sequence set forth in SEQ ID No. 61,
      (e) the VL-CDR2 comprising the amino acid sequence set forth in SEQ ID No. 62, and
      (f) the VL-CDR3 comprising the amino acid sequence set forth in SEQ ID No. 63;
[7] the bispecific antibody or antibody fragment thereof according to the preceding item [1] or [2], wherein the two different antigen-binding sites are the S 1-A binding site and the S 1-B binding site,
   wherein (i) the S 1-A binding site is the antigen-binding site comprising the VH having
      (a) the VH-CDR1 comprising the amino acid sequence set forth in SEQ ID No. 28,
      (b) the VH-CDR2 comprising the amino acid sequence set forth in SEQ ID No. 29, and
      (c) the VH-CDR3 comprising the amino acid sequence set forth in SEQ ID No. 30, and
         the VL having
      (d) the VL-CDR1 comprising the amino acid sequence set forth in SEQ ID No. 52,
      (e) the VL-CDR2 comprising the amino acid sequence set forth in SEQ ID No. 53, and
      (f) the VL-CDR3 comprising the amino acid sequence set forth in SEQ ID No. 54,
      and
   (ii) the S1-B binding site is the antigen-binding site comprising the VH having
      (a) the VH-CDR1 comprising the amino acid sequence set forth in SEQ ID No. 40,
      (b) the VH-CDR2 comprising the amino acid sequence set forth in SEQ ID No. 41, and
      (c) the VH-CDR3 comprising the amino acid sequence set forth in SEQ ID No. 42, and
         the VL having
      (d) the VL-CDR1 comprising the amino acid sequence set forth in SEQ ID No. 64,
      (e) the VL-CDR2 comprising the amino acid sequence set forth in SEQ ID No. 65, and
      (f) the VL-CDR3 comprising the amino acid sequence set forth in SEQ ID No. 66;
[8] the bispecific antibody or antibody fragment thereof according to the preceding item [1] or [2], wherein the two different antigen-binding sites are the S 1-A binding site and the S 1-B binding site,
   wherein (i) the S 1-A binding site is the antigen-binding site comprising the VH having
      (a) the VH-CDR1 comprising the amino acid sequence set forth in SEQ ID No. 31,
      (b) the VH-CDR2 comprising the amino acid sequence set forth in SEQ ID No. 32, and
      (c) the VH-CDR3 comprising the amino acid sequence set forth in SEQ ID No. 33, and
      the VL having:
      (d) the VL-CDR1 comprising the amino acid sequence set forth in SEQ ID No. 55,
      (e) the VL-CDR2 comprising the amino acid sequence set forth in SEQ ID No. 56, and
      (f) the VL-CDR3 comprising the amino acid sequence set forth in SEQ ID No. 57,
      and
   (ii) the S1-B binding site is the antigen-binding site comprising the VH having
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39, and
         the VL having
      (d) the VL-CDR1 comprising the amino acid sequence set forth in SEQ ID No. 61,
      (e) the VL-CDR2 comprising the amino acid sequence set forth in SEQ ID No. 62, and
      (f) the VL-CDR3 comprising the amino acid sequence set forth in SEQ ID No. 63;
[9] the bispecific antibody or antibody fragment thereof according to the preceding item [1] or [2], wherein the two different antigen-binding sites are the S 1-A binding site and the S 1-B binding site,
   wherein (i) the S 1-A binding site is the antigen-binding site comprising the VH having
      (a) the VH-CDR1 comprising the amino acid sequence set forth in SEQ ID No. 31,
      (b) the VH-CDR2 comprising the amino acid sequence set forth in SEQ ID No. 32, and
      (c) the VH-CDR3 comprising the amino acid sequence set forth in SEQ ID No. 33, and
         the VL having
      (d) the VL-CDR1 comprising the amino acid sequence set forth in SEQ ID No. 55,
      (e) the VL-CDR2 comprising the amino acid sequence set forth in SEQ ID No. 56, and
      (f) the VL-CDR3 comprising the amino acid sequence set forth in SEQ ID No. 57,
      and
   (ii) the S1-B binding site is the antigen-binding site comprising the VH having
      (a) the VH-CDR1 comprising the amino acid sequence set forth in SEQ ID No. 40,
      (b) the VH-CDR2 comprising the amino acid sequence set forth in SEQ ID No. 41, and
      (c) the VH-CDR3 comprising the amino acid sequence set forth in SEQ ID No. 42, and
         the VL having
      (d) the VL-CDR1 comprising the amino acid sequence set forth in SEQ ID No. 64,
      (e) the VL-CDR2 comprising the amino acid sequence set forth in SEQ ID No. 65, and
      (f) the VL-CDR3 comprising the amino acid sequence set forth in SEQ ID No. 66;
[10] the bispecific antibody or antibody fragment thereof according to the preceding item [1] or [2], wherein the two different antigen-binding sites are the S 1-A binding site and the S 1-B binding site,
   wherein (i) the S 1-A binding site is the antigen-binding site comprising the VH having
      (a) the VH-CDR1 comprising the amino acid sequence set forth in SEQ ID No. 34,
      (b) the VH-CDR2 comprising the amino acid sequence set forth in SEQ ID No. 35, and
      (c) the VH-CDR3 comprising the amino acid sequence set forth in SEQ ID No. 36, and
         the VL having
      (d) the VL-CDR1 comprising the amino acid sequence set forth in SEQ ID No. 58,
      (e) the VL-CDR2 comprising the amino acid sequence set forth in SEQ ID No. 59, and
      (f) the VL-CDR3 comprising the amino acid sequence set forth in SEQ ID No. 60,
      and
   (ii) the S1-B binding site is the antigen-binding site comprising the VH having
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39, and
         the VL having
      (d) the VL-CDR1 comprising the amino acid sequence set forth in SEQ ID No. 61,
      (e) the VL-CDR2 comprising the amino acid sequence set forth in SEQ ID No. 62, and
      (f) the VL-CDR3 comprising the amino acid sequence set forth in SEQ ID No. 63;
[11] the bispecific antibody or antibody fragment thereof according to the preceding item [1] or [2], wherein the two different antigen-binding sites are the S 1-A binding site and the S 1-B binding site,
   wherein (i) the S 1-A binding site is the antigen-binding site comprising the VH having
      (a) the VH-CDR1 comprising the amino acid sequence set forth in SEQ ID No. 34,
      (b) the VH-CDR2 comprising the amino acid sequence set forth in SEQ ID No. 35, and
      (c) the VH-CDR3 comprising the amino acid sequence set forth in SEQ ID No. 36, and
         the VL having
      (d) the VL-CDR1 comprising the amino acid sequence set forth in SEQ ID No. 58,
      (e) the VL-CDR2 comprising the amino acid sequence set forth in SEQ ID No. 59, and
      (f) the VL-CDR3 comprising the amino acid sequence set forth in SEQ ID No. 60,
      and
   (ii) the S1-B binding site is the antigen-binding site comprising the VH having
      (a) the VH-CDR1 comprising the amino acid sequence set forth in SEQ ID No. 40,
      (b) the VH-CDR2 comprising the amino acid sequence set forth in SEQ ID No. 41, and
      (c) the VH-CDR3 comprising the amino acid sequence set forth in SEQ ID No. 42, and
         the VL having
      (d) the VL-CDR1 comprising the amino acid sequence set forth in SEQ ID No. 64,
      (e) the VL-CDR2 comprising the amino acid sequence set forth in SEQ ID No. 65, and
      (f) the VL-CDR3 comprising the amino acid sequence set forth in SEQ ID No. 66;
[12] the bispecific antibody or antibody fragment thereof according to the preceding item [1] or [2], wherein the two different antigen-binding sites are the S 1-A binding site and the S2-C binding site,
   wherein (i) the S 1-A binding site is the antigen-binding site comprising the VH having
      (a) the VH-CDR1 comprising the amino acid sequence set forth in SEQ ID No. 25,
      (b) the VH-CDR2 comprising the amino acid sequence set forth in SEQ ID No. 26, and
      (c) the VH-CDR3 comprising the amino acid sequence set forth in SEQ ID No. 27, and
         the VL having
      (d) the VL-CDR1 comprising the amino acid sequence set forth in SEQ ID No. 49,
      (e) the VL-CDR2 comprising the amino acid sequence set forth in SEQ ID No. 50, and
      (f) the VL-CDR3 comprising the amino acid sequence set forth in SEQ ID No. 51,
      and
   (ii) the S2-C binding site is the antigen-binding site comprising the VH having:
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
      the VL having:
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69;
[13] the bispecific antibody or antibody fragment thereof according to the preceding item [1] or [2], wherein the two different antigen-binding sites are the S 1-A binding site and the S2-C binding site,
   wherein (i) the S 1-A binding site is the antigen-binding site comprising the VH having
      (a) the VH-CDR1 comprising the amino acid sequence set forth in SEQ ID No. 28,
      (b) the VH-CDR2 comprising the amino acid sequence set forth in SEQ ID No. 29, and
      (c) the VH-CDR3 comprising the amino acid sequence set forth in SEQ ID No. 30, and
         the VL having
      (d) the VL-CDR1 comprising the amino acid sequence set forth in SEQ ID No. 52,
      (e) the VL-CDR2 comprising the amino acid sequence set forth in SEQ ID No. 53, and
      (f) the VL-CDR3 comprising the amino acid sequence set forth in SEQ ID No. 54,
      and
   (ii) the S2-C binding site is the antigen-binding site comprising the VH having
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
         the VL having
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69;
[14] the bispecific antibody or antibody fragment thereof according to the preceding item [1] or [2], wherein the two different antigen-binding sites are the S 1-A binding site and the S2-C binding site,
   wherein (i) the S 1-A binding site is the antigen-binding site comprising the VH having
      (a) the VH-CDR1 comprising the amino acid sequence set forth in SEQ ID No. 31,
      (b) the VH-CDR2 comprising the amino acid sequence set forth in SEQ ID No. 32, and
      (c) the VH-CDR3 comprising the amino acid sequence set forth in SEQ ID No. 33, and
         the VL having
      (d) the VL-CDR1 comprising the amino acid sequence set forth in SEQ ID No. 55,
      (e) the VL-CDR2 comprising the amino acid sequence set forth in SEQ ID No. 56, and
      (f) the VL-CDR3 comprising the amino acid sequence set forth in SEQ ID No. 57,
      and
   (ii) the S2-C binding site is the antigen-binding site comprising the VH having
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
         the VL having
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69;
[15] the bispecific antibody or antibody fragment thereof according to the preceding item [1] or [2], wherein the two different antigen-binding sites are the S 1-A binding site and the S2-C binding site,
   wherein (i) the S 1-A binding site is the antigen-binding site comprising the VH having
      (a) the VH-CDR1 comprising the amino acid sequence set forth in SEQ ID No. 34,
      (b) the VH-CDR2 comprising the amino acid sequence set forth in SEQ ID No. 35, and
      (c) the VH-CDR3 comprising the amino acid sequence set forth in SEQ ID No. 36, and
         the VL having
      (d) the VL-CDR1 comprising the amino acid sequence set forth in SEQ ID No. 58,
      (e) the VL-CDR2 comprising the amino acid sequence set forth in SEQ ID No. 59, and (f) the VL-CDR3 comprising the amino acid sequence set forth in SEQ ID No. 60,
      and
   (ii) the S2-C binding site is the antigen-binding site comprising the VH having
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
         the VL having
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69;
[16] the bispecific antibody or antibody fragment thereof according to the preceding item [1] or [2], wherein the two different antigen-binding sites are the S 1-A binding site and the S2-D binding site,
   wherein (i) the S 1-A binding site is the antigen-binding site comprising the VH having
      (a) the VH-CDR1 comprising the amino acid sequence set forth in SEQ ID No. 25,
      (b) the VH-CDR2 comprising the amino acid sequence set forth in SEQ ID No. 26, and
      (c) the VH-CDR3 comprising the amino acid sequence set forth in SEQ ID No. 27, and
         the VL having
      (d) the VL-CDR1 comprising the amino acid sequence set forth in SEQ ID No. 49,
      (e) the VL-CDR2 comprising the amino acid sequence set forth in SEQ ID No. 50, and
      (f) the VL-CDR3 comprising the amino acid sequence set forth in SEQ ID No. 51,
      and
   (ii) the S2-D binding site is the antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
         the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72;
[17] the bispecific antibody or antibody fragment thereof according to the preceding item [1] or [2], wherein the two different antigen-binding sites are the S 1-A binding site and the S2-D binding site,
   wherein (i) the S 1-A binding site is the antigen-binding site comprising the VH having
      (a) the VH-CDR1 comprising the amino acid sequence set forth in SEQ ID No. 28,
      (b) the VH-CDR2 comprising the amino acid sequence set forth in SEQ ID No. 29, and
      (c) the VH-CDR3 comprising the amino acid sequence set forth in SEQ ID No. 30, and
         the VL having
      (d) the VL-CDR1 comprising the amino acid sequence set forth in SEQ ID No. 52,
      (e) the VL-CDR2 comprising the amino acid sequence set forth in SEQ ID No. 53, and
      (f) the VL-CDR3 comprising the amino acid sequence set forth in SEQ ID No. 54,
      and
   (ii) the S2-D binding site is the antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
         the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72;
[18] the bispecific antibody or antibody fragment thereof according to the preceding item [1] or [2], wherein the two different antigen-binding sites are the S 1-A binding site and the S2-D binding site,
   wherein (i) the S 1-A binding site is the antigen-binding site comprising the VH having
      (a) the VH-CDR1 comprising the amino acid sequence set forth in SEQ ID No. 31,
      (b) the VH-CDR2 comprising the amino acid sequence set forth in SEQ ID No. 32, and
      (c) the VH-CDR3 comprising the amino acid sequence set forth in SEQ ID No. 33, and
         the VL having
      (d) the VL-CDR1 comprising the amino acid sequence set forth in SEQ ID No. 55,
      (e) the VL-CDR2 comprising the amino acid sequence set forth in SEQ ID No. 56, and
      (f) the VL-CDR3 comprising the amino acid sequence set forth in SEQ ID No. 57,
      and
   (ii) the S2-D binding site is the antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
         the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72;
[19] the bispecific antibody or antibody fragment thereof according to the preceding item [1] or [2], wherein the two different antigen-binding sites are the S 1-A binding site and the S2-D binding site,
   wherein (i) the S 1-A binding site is the antigen-binding site comprising the VH having
      (a) the VH-CDR1 comprising the amino acid sequence set forth in SEQ ID No. 34,
      (b) the VH-CDR2 comprising the amino acid sequence set forth in SEQ ID No. 35, and
      (c) the VH-CDR3 comprising the amino acid sequence set forth in SEQ ID No. 36, and
         the VL having
      (d) the VL-CDR1 comprising the amino acid sequence set forth in SEQ ID No. 58,
      (e) the VL-CDR2 comprising the amino acid sequence set forth in SEQ ID No. 59, and
      (f) the VL-CDR3 comprising the amino acid sequence set forth in SEQ ID No. 60,
      and
   (ii) the S2-D binding site is the antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
         the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72;
[20] the bispecific antibody or antibody fragment thereof according to the preceding item [1] or [2], wherein the two different antigen-binding sites are the S1-B binding site and the S2-C binding site,
   wherein (i) the S1-B binding site is the antigen-binding site comprising the VH having
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39, and
      the VL having:
      (d) the VL-CDR1 comprising the amino acid sequence set forth in SEQ ID No. 61,
      (e) the VL-CDR2 comprising the amino acid sequence set forth in SEQ ID No. 62, and
      (f) the VL-CDR3 comprising the amino acid sequence set forth in SEQ ID No. 63,
      and
   (ii) the S2-C binding site is the antigen-binding site comprising the VH having
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
         the VL having
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69;
[21] the bispecific antibody or antibody fragment thereof according to the preceding item [1] or [2], wherein the two different antigen-binding sites are the S1-B binding site and the S2-C binding site,
   wherein (i) the S1-B binding site is the antigen-binding site comprising the VH having
      (a) the VH-CDR1 comprising the amino acid sequence set forth in SEQ ID No. 40,
      (b) the VH-CDR2 comprising the amino acid sequence set forth in SEQ ID No. 41, and
      (c) the VH-CDR3 comprising the amino acid sequence set forth in SEQ ID No. 42, and
         the VL having
      (d) the VL-CDR1 comprising the amino acid sequence set forth in SEQ ID No. 64,
      (e) the VL-CDR2 comprising the amino acid sequence set forth in SEQ ID No. 65, and
      (f) the VL-CDR3 comprising the amino acid sequence set forth in SEQ ID No. 66.
      and
   (ii) the S2-C binding site is the antigen-binding site comprising the VH having
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
         the VL having
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69;
[22] the bispecific antibody or antibody fragment thereof according to the preceding item [1] or [2], wherein the two different antigen-binding sites are the S1-B binding site and the S2-D binding site,
   wherein (i) the S1-B binding site is the antigen-binding site comprising the VH having
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39, and
         the VL having
      (d) the VL-CDR1 comprising the amino acid sequence set forth in SEQ ID No. 61,
      (e) the VL-CDR2 comprising the amino acid sequence set forth in SEQ ID No. 62, and
      (f) the VL-CDR3 comprising the amino acid sequence set forth in SEQ ID No. 63,
      and
   (ii) the S2-D binding site is the antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
         the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72;
[23] the bispecific antibody or antibody fragment thereof according to the preceding item [1] or [2], wherein the two different antigen-binding sites are the S1-B binding site and the S2-D binding site,
   wherein (i) the S1-B binding site is the antigen-binding site comprising the VH having
      (a) the VH-CDR1 comprising the amino acid sequence set forth in SEQ ID No. 40,
      (b) the VH-CDR2 comprising the amino acid sequence set forth in SEQ ID No. 41, and
      (c) the VH-CDR3 comprising the amino acid sequence set forth in SEQ ID No. 42, and
         the VL having
      (d) the VL-CDR1 comprising the amino acid sequence set forth in SEQ ID No. 64,
      (e) the VL-CDR2 comprising the amino acid sequence set forth in SEQ ID No. 65, and
      (f) the VL-CDR3 comprising the amino acid sequence set forth in SEQ ID No. 66.
      and
   (ii) the S2-D binding site is the antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
         the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72;
[24] the bispecific antibody or antibody fragment thereof according to the preceding item [1] or [2], wherein the two different antigen-binding sites are the S2-C binding site and the S2-D binding site,
   wherein (i) the S2-C binding site is the antigen-binding site comprising the VH having
      (a) the VH-CDR1 comprising the amino acid sequence set forth in SEQ ID No. 43,
      (b) the VH-CDR2 comprising the amino acid sequence set forth in SEQ ID No. 44, and
      (c) the VH-CDR3 comprising the amino acid sequence set forth in SEQ ID No. 45, and
         the VL having
      (d) the VL-CDR1 comprising the amino acid sequence set forth in SEQ ID No. 67,
      (e) the VL-CDR2 comprising the amino acid sequence set forth in SEQ ID No. 68, and
      (f) the VL-CDR3 comprising the amino acid sequence set forth in SEQ ID No. 69,
      and
   (ii) the S2-D binding site is the antigen-binding site comprising the VH having
      (a) the VH-CDR1 comprising the amino acid sequence set forth in SEQ ID No. 46,
      (b) the VH-CDR2 comprising the amino acid sequence set forth in SEQ ID No. 47, and
      (c) the VH-CDR3 comprising the amino acid sequence set forth in SEQ ID No. 48, and
         the VL having
      (d) the VL-CDR1 comprising the amino acid sequence set forth in SEQ ID No. 70,
      (e) the VL-CDR2 comprising the amino acid sequence set forth in SEQ ID No. 71, and
      (f) the VL-CDR3 comprising the amino acid sequence set forth in SEQ ID No. 72;
[25] the bispecific antibody or antibody fragment thereof according to any one of the preceding items [1] to [24], wherein the FWs in the VHs constituting any two different antigen-binding sites selected from the S 1-A binding site, the S1-B binding site, the S2-C binding site and the S2-D binding site, respectively, comprise the amino acid sequence encoded by human germ-line VH gene IGHV3-66 or gene thereof with one or more somatic mutations and/or back mutations, and/or the FWs in the VLs constituting the same antigen-binding sites, respectively, comprise the amino acid sequence encoded by human germ-line VL gene IGKV1-39 or gene thereof with one or more somatic mutations and/or back mutations;
[26] the bispecific antibody or antibody fragment thereof according to any one of the preceding items [1] to [25], wherein the S1-A binding site is constituted by the VH and VL comprising any one pair of the amino acid sequences selected from
   (a) SEQ ID No. 1 and SEQ ID No. 12 or 104,
   (b) SEQ ID No. 2 and SEQ ID No. 13 or 105,
   (c) SEQ ID No. 3 and SEQ ID No. 14 or 106,
   (d) SEQ ID No. 4 and SEQ ID No. 15 or 107,
   (e) SEQ ID No. 5 and SEQ ID No. 16 or 108, and
   (f) SEQ ID No. 6 and SEQ ID No. 17 or 109, or a pair of amino acid sequences having at least 80%, 90%, 95%, 98% or 99% identity to the respective same amino acid sequences (excluding three CDRs, respectively), each independently;
[27] the bispecific antibody or antibody fragment thereof according to any one of the preceding items [1] to [26], wherein the S1-B binding site is constituted by the VH and VL comprising a pair of the amino acid sequences set forth in
   (a) SEQ ID No. 7 and SEQ ID No. 18 or 110, or
   (b) SEQ ID No. 8 and SEQ ID No. 19 or 111, or a pair of amino acid sequences having at least 80%, 90%, 95%, 98% or 99% identity to the respective same amino acid sequences (excluding three CDRs, respectively), each independently;
[28] the bispecific antibody or antibody fragment thereof according to any one of the preceding items [1] to [27], wherein the S2-C binding site is constituted by the VH and VL comprising a pair of the amino acid sequences set forth in SEQ ID No. 9 and SEQ ID No. 20 or 112, or a pair of amino acid sequences having at least 80%, 90%, 95%, 98% or 99% identity to the respective same amino acid sequences (excluding three CDRs, respectively), each independently;
[29] the bispecific antibody or antibody fragment thereof according to any one of the preceding items [1] to [28], wherein the S2-D binding site is constituted by the VH and VL comprising a pair of the amino acid sequences set forth in
   (a) SEQ ID No. 10 and SEQ ID No. 21 or 113, or
   (b) SEQ ID No. 11 and SEQ ID No. 22 or 114, or a pair of amino acid sequences having at least 80%, 90%, 95%, 98% or 99% identity to the respective same amino acid sequences (excluding three CDRs, respectively), each independently;
[30] the bispecific antibody or antibody fragment thereof according to any one of the preceding items [1] to [26], wherein the S1-A binding site is constituted by the VH and VL comprising any one pair of the amino acid sequences selected from
   (a) SEQ ID No. 1 and SEQ ID No. 12 or 104,
   (b) SEQ ID No. 2 and SEQ ID No. 13 or 105,
   (c) SEQ ID No. 3 and SEQ ID No. 14 or 106,
   (d) SEQ ID No. 4 and SEQ ID No. 15 or 107,
   (e) SEQ ID No. 5 and SEQ ID No. 16 or 108, and
   (f) SEQ ID No. 6 and SEQ ID No. 17 or 109;
[31] the bispecific antibody or antibody fragment thereof according to any one of the preceding items [1] to [25], [27]. and [30], wherein the S1-B binding site is constituted by the VH and VL comprising a pair of the amino acid sequences set forth in
   (a) SEQ ID No. 7 and SEQ ID No. 18 or 110, or
   (b) SEQ ID No. 8 and SEQ ID No. 19 or 111;
[32] the bispecific antibody or antibody fragment thereof according to any one of the preceding items [1] to [25], [28], [30], and [31], wherein the S2-C binding site is constituted by the VH and VL comprising a pair of the amino acid sequences set forth in SEQ ID No. 9 and SEQ ID No. 20 or 112;
[33] the bispecific antibody or antibody fragment thereof according to any one of the preceding items [1] to [25] and [29] to [32], wherein the S2-D binding site is constituted by the VH and VL comprising a pair of the amino acid sequences set forth in
   (a) SEQ ID No. 10 and SEQ ID No. 21 or 113, or
   (b) SEQ ID No. 11 and SEQ ID No. 22 or 114;
[34] a bispecific antibody or an antibody fragment thereof, having antigen-binding sites specifically binding to two different epitopes on the extracellular domains of Allergin-1, respectively,
   wherein the antigen-binding sites do not mutually compete for Allergin-1, and are any two different antigen-binding sites selected from the S1-A binding site, the S1-B binding site, the S2-C binding site and the S2-D binding site, and
   wherein (i) the S1-A binding site is constituted by the VH and VL comprising any one pair of the amino acid sequences selected from
      (a) SEQ ID No. 1 and SEQ ID No. 12 or 104,
      (b) SEQ ID No. 2 and SEQ ID No. 13 or 105,
      (c) SEQ ID No. 3 and SEQ ID No. 14 or 106,
      (d) SEQ ID No. 4 and SEQ ID No. 15 or 107,
      (e) SEQ ID No. 5 and SEQ ID No. 16 or 108, and
      (f) SEQ ID No. 6 and SEQ ID No. 17 or 109, or a pair of amino acid sequences having at least 80%, 90%, 95%, 98% or 99% identity to the respective same amino acid sequences (excluding three CDRs, respectively), each independently,
   (ii) the S1-B binding site is constituted by the VH and VL comprising a pair of the amino acid sequences set forth in
      (a) SEQ ID No. 7 and SEQ ID No. 18 or 110, or
      (b) SEQ ID No. 8 and SEQ ID No. 19 or 111, or a pair of amino acid sequences having at least 80%, 90%, 95%, 98% or 99% identity to the respective same amino acid sequences (excluding three CDRs, respectively), each independently,
   (iii) the S2-C binding site is constituted by the VH and VL comprising a pair of the amino acid sequences set forth in SEQ ID No. 9 and SEQ ID No. 20 or 112, or amino acid sequences having at least 80%, 90%, 95%, 98% or 99% identity to the respective same amino acid sequences (excluding three CDRs, respectively), each independently, and
   (iv) the S2-D binding site is constituted by the VH and VL comprising a pair of the amino acid sequences set forth in
      (a) SEQ ID No. 10 and SEQ ID No. 21 or 113, or
      (b) SEQ ID No. 11 and SEQ ID No. 22 or 114, or a pair of amino acid sequences having at least 80%, 90%, 95%, 98% or 99% identity to the respective same amino acid sequences (excluding three CDRs, respectively), each independently;
[35] a bispecific antibody to Allergin-1 or an antibody fragment thereof, comprising any two different antigen-binding sites selected from the S1-A binding site, the S1-B binding site, the S2-C binding site and the S2-D binding site,
   wherein (i) the S1-A binding site is constituted by the VH and VL comprising any one pair of the amino acid sequences selected from
      (a) SEQ ID No. 1 and SEQ ID No. 12 or 104,
      (b) SEQ ID No. 2 and SEQ ID No. 13 or 105,
      (c) SEQ ID No. 3 and SEQ ID No. 14 or 106,
      (d) SEQ ID No. 4 and SEQ ID No. 15 or 107,
      (e) SEQ ID No. 5 and SEQ ID No. 16 or 108, and
      (f) SEQ ID No. 6 and SEQ ID No. 17 or 109, or
   a pair of amino acid sequences having at least 80%, 90%, 95%, 98% or 99% identity to the respective same amino acid sequences (excluding three CDRs, respectively), each independently,
   (ii) the S1-B binding site is constituted by the VH and VL comprising a pair of the amino acid sequences set forth in
      (a) SEQ ID No. 7 and SEQ ID No. 18 or 110, or
      (b) SEQ ID No. 8 and SEQ ID No. 19 or 111, or
   a pair of amino acid sequences having at least 80%, 90%, 95%, 98% or 99% identity to the respective same amino acid sequences (excluding three CDRs, respectively), each independently,
   (iii) the S2-C binding site is constituted by the VH and VL comprising a pair of the amino acid sequences set forth in SEQ ID No. 9 and SEQ ID No. 20 or 112, or
   amino acid sequences having at least 80%, 90%, 95%, 98% or 99% identity to the respective same amino acid sequences (excluding three CDRs, respectively), each independently, and
   (iv) the S2-D binding site is constituted by the VH and VL comprising a pair of the amino acid sequences set forth in
      (a) SEQ ID No. 10 and SEQ ID No. 21 or 113, or
      (b) SEQ ID No. 11 and SEQ ID No. 22 or 114, or
   a pair of amino acid sequences having at least 80%, 90%, 95%, 98% or 99% identity to the respective same amino acid sequences (excluding three CDRs, respectively), each independently;
[36] the bispecific antibody or antibody fragment thereof according to the preceding item [34] or [35],
   wherein (i) the S1-A binding site is constituted by the VH and VL comprising any one pair of the amino acid sequences selected from:
      (a) SEQ ID No. 1 and SEQ ID No. 12 or 104,
      (b) SEQ ID No. 2 and SEQ ID No. 13 or 105,
      (c) SEQ ID No. 3 and SEQ ID No. 14 or 106,
      (d) SEQ ID No. 4 and SEQ ID No. 15 or 107,
      (e) SEQ ID No. 5 and SEQ ID No. 16 or 108, and
      (f) SEQ ID No. 6 and SEQ ID No. 17 or 109,
   (ii) the S1-B binding site is constituted by the VH and VL comprising a pair of the amino acid sequences set forth in
      (a) SEQ ID No. 7 and SEQ ID No. 18 or 110, or
      (b) SEQ ID No. 8 and SEQ ID No. 19 or 111,
   (iii) the VH and VL constituting the S2-C binding site are constituted by the VH and VL comprising a pair of the amino acid sequences set forth in SEQ ID No. 9 and SEQ ID No. 20 or 112, and
   (iv) the S2-D binding site is constituted by the VH and VL comprising a pair of the amino acid sequences set forth in
      (a) SEQ ID No. 10 and SEQ ID No. 21 or 113, or
      (b) SEQ ID No. 11 and SEQ ID No. 22 or 114;
[37] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 1 and SEQ ID No. 12 or 104, and the S1-B binding site is constituted by the VH and VL comprising a pair of the amino acid sequences set forth in SEQ ID No. 7 and SEQ ID No. 18 or 110;
[38] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 2 and SEQ ID No. 13 or 105, and the S1-B binding site is constituted by the VH and VL comprising a pair of the amino acid sequences set forth in SEQ ID No. 7 and SEQ ID No. 18 or 110;
[39] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 3 and SEQ ID No. 14 or 106, and the S1-B binding site is constituted by the VH and VL comprising a pair of the amino acid sequences set forth in SEQ ID No. 7 and SEQ ID No. 18 or 110;
[40] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 4 and SEQ ID No. 15 or 107, and the S1-B binding site is constituted by the VH and VL comprising a pair of the amino acid sequences set forth in SEQ ID No. 7 and SEQ ID No. 18 or 110;
[41] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 5 and SEQ ID No. 16 or 108, and the S1-B binding site is constituted by the VH and VL comprising a pair of the amino acid sequences set forth in SEQ ID No. 7 and SEQ ID No. 18 or 110;
[42] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 6 and SEQ ID No. 17 or 109, and the S1-B binding site is constituted by the VH and VL comprising a pair of the amino acid sequences set forth in SEQ ID No. 7 and SEQ ID No. 18 or 110;
[43] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 1 and SEQ ID No. 12 or 104, and the S1-B binding site is constituted by the VH and VL comprising a pair of the amino acid sequences set forth in SEQ ID No. 8 and SEQ ID No. 19 or 111;
[44] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 2 and SEQ ID No. 13 or 105, and the S1-B binding site is constituted by the VH and VL comprising a pair of the amino acid sequences set forth in SEQ ID No. 8 and SEQ ID No. 19 or 111;
[45] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 3 and SEQ ID No. 14 or 106, and the S1-B binding site is constituted by the VH and VL comprising a pair of the amino acid sequences set forth in SEQ ID No. 8 and SEQ ID No. 19 or 111;
[46] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 4 and SEQ ID No. 15 or 107, and the S1-B binding site is constituted by the VH and VL comprising a pair of the amino acid sequences set forth in SEQ ID No. 8 and SEQ ID No. 19 or 111;
[47] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 5 and SEQ ID No. 16 or 108, and the S1-B binding site is constituted by the VH and VL comprising a pair of the amino acid sequences set forth in SEQ ID No. 8 and SEQ ID No. 19 or 111;
[48] the bispecific antibody or antibody fragment thereof according to any one of the preceding items [1], [2], [25] and [34] to [36], wherein the two different antigen-binding sites are the S1-A binding site and the S1-B binding site, and wherein the S1-A binding site is constituted by the VH and VL comprising a pair of the amino acid sequences set forth in SEQ ID No. 6 and SEQ ID No. 17 or 109, and the S1-B binding site is constituted by the VH and VL comprising a pair of the amino acid sequences set forth in SEQ ID No. 8 and SEQ ID No. 19 or 111;
[49] the bispecific antibody or antibody fragment thereof according to any one of the preceding items [1], [2], [25] and [34] to [36], wherein the two different antigen-binding sites are the S1-A binding site and the S2-C binding site, and wherein the S1-A binding site is constituted by the VH and VL comprising a pair of the amino acid sequences set forth in SEQ ID No. 1 and SEQ ID No. 12 or 104, and the S2-C binding site is constituted by the VH and VL comprising a pair of the amino acid sequences set forth in SEQ ID No. 9 and SEQ ID No. 20 or 112;
[50] the bispecific antibody or antibody fragment thereof according to any one of the preceding items [1], [2], [25] and [34] to [36], wherein the two different antigen-binding sites are the S1-A binding site and the S2-C binding site, and wherein the S1-A binding site is constituted by the VH and VL comprising a pair of the amino acid sequences set forth in SEQ ID No. 2 and SEQ ID No. 13 or 105, and the S2-C binding site is constituted by the VH and VL comprising a pair of the amino acid sequences set forth in SEQ ID No. 9 and SEQ ID No. 20 or 112;
[51] the bispecific antibody or antibody fragment thereof according to any one of the preceding items [1], [2], [25] and [34] to [36], wherein the two different antigen-binding sites are the S1-A binding site and the S2-C binding site, and wherein the S1-A binding site is constituted by the VH and VL comprising a pair of the amino acid sequences set forth in SEQ ID No. 3 and SEQ ID No. 14 or 106, and the S2-C binding site is constituted by the VH and VL comprising a pair of the amino acid sequences set forth in SEQ ID No. 9 and SEQ ID No. 20 or 112;
[52] the bispecific antibody or antibody fragment thereof according to any one of the preceding items [1], [2], [25] and [34] to [36], wherein the two different antigen-binding sites are the S1-A binding site and the S2-C binding site, and wherein the S1-A binding site is constituted by the VH and VL comprising a pair of the amino acid sequences set forth in SEQ ID No. 4 and SEQ ID No. 15 or 107, and the S2-C binding site is constituted by the VH and VL comprising a pair of the amino acid sequences set forth in SEQ ID No. 9 and SEQ ID No. 20 or 112;
[53] the bispecific antibody or antibody fragment thereof according to any one of the preceding items [1], [2], [25] and [34] to [36], wherein the two different antigen-binding sites are the S1-A binding site and the S2-C binding site, and wherein the S1-A binding site is constituted by the VH and VL comprising a pair of the amino acid sequences set forth in SEQ ID No. 5 and SEQ ID No. 16 or 108, and the S2-C binding site is constituted by the VH and VL comprising a pair of the amino acid sequences set forth in SEQ ID No. 9 and SEQ ID No. 20 or 112;
[54] the bispecific antibody or antibody fragment thereof according to any one of the preceding items [1], [2], [25] and [34] to [36], wherein the two different antigen-binding sites are the S1-A binding site and the S2-C binding site, and wherein the S1-A binding site is constituted by the VH and VL comprising a pair of the amino acid sequences set forth in SEQ ID No. 6 and SEQ ID No. 17 or 109, and the S2-C binding site is constituted by the VH and VL comprising a pair of the amino acid sequences set forth in SEQ ID No. 9 and SEQ ID No. 20 or 112;
[55] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 1 and SEQ ID No. 12 or 114, and the S2-D binding site is constituted by the VH and VL comprising a pair of the amino acid sequences set forth in SEQ ID No. 10 and SEQ ID No. 21 or 113;
[56] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 2 and SEQ ID No. 13 or 105, and the S2-D binding site is constituted by the VH and VL comprising a pair of the amino acid sequences set forth in SEQ ID No. 10 and SEQ ID No. 21 or 113;
[57] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 3 and SEQ ID No. 14 or 106, and the S2-D binding site is constituted by the VH and VL comprising a pair of the amino acid sequences set forth in SEQ ID No. 10 and SEQ ID No. 21 or 113;
[58] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 4 and SEQ ID No. 15 or 107, and the S2-D binding site is constituted by the VH and VL comprising a pair of the amino acid sequences set forth in SEQ ID No. 10 and SEQ ID No. 21 or 113;
[59] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 5 and SEQ ID No. 16 or 108, and the S2-D binding site is constituted by the VH and VL comprising a pair of the amino acid sequences set forth in SEQ ID No. 10 and SEQ ID No. 21 or 113;
[60] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 6 and SEQ ID No. 17 or 109, and the S2-D binding site is constituted by the VH and VL comprising a pair of the amino acid sequences set forth in SEQ ID No. 10 and SEQ ID No. 21 or 113;
[61] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 1 and SEQ ID No. 12 or 104, and the S2-D binding site is constituted by the VH and VL comprising a pair of the amino acid sequences set forth in SEQ ID No. 11 and SEQ ID No. 22 or 114;
[62] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 2 and SEQ ID No. 13 or 105, and the S2-D binding site is constituted by the VH and VL comprising a pair of the amino acid sequences set forth in SEQ ID No. 11 and SEQ ID No. 22 or 114;
[63] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 3 and SEQ ID No. 14 or 106, and the S2-D binding site is constituted by the VH and VL comprising a pair of the amino acid sequences set forth in SEQ ID No. 11 and SEQ ID No. 22 or 114;
[64] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 4 and SEQ ID No. 15 or 107, and the S2-D binding site is constituted by the VH and VL comprising a pair of the amino acid sequences set forth in SEQ ID No. 11 and SEQ ID No. 22 or 114;
[65] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 5 and SEQ ID No. 16 or 108, and the S2-D binding site is constituted by the VH and VL comprising a pair of the amino acid sequences set forth in SEQ ID No. 11 and SEQ ID No. 22 or 114;
[66] the bispecific antibody or antibody fragment thereof according to any one of the preceding items [1], [2], [25] and [34] to [36], wherein the two different antigen-binding sites are the S1-A binding site and the S2-D binding site, and wherein the S1-A binding site is constituted by the VH and VL comprising a pair of the amino acid sequences set forth in SEQ ID No. 6 and SEQ ID No. 17 or 109, and the S2-D binding site is constituted by the VH and VL comprising a pair of the amino acid sequences set forth in SEQ ID No. 11 and SEQ ID No. 22 or 114;
[67] the bispecific antibody or antibody fragment thereof according to any one of the preceding items [1], [2], [25] and [34] to [36], wherein the two different antigen-binding sites are the S1-B binding site and the S2-C binding site, and wherein the S1-B binding site is constituted by the VH and VL comprising a pair of the amino acid sequences set forth in SEQ ID No. 7 and SEQ ID No. 18 or 110, and the S2-C binding site is constituted by the VH and VL comprising a pair of the amino acid sequences set forth in SEQ ID No. 9 and SEQ ID No. 20 or 112;
[68] the bispecific antibody or antibody fragment thereof according to any one of the preceding items [1], [2], [25] and [34] to [36], wherein the two different antigen-binding sites are the S1-B binding site and the S2-C binding site, and wherein the S1-B binding site is constituted by the VH and VL comprising a pair of the amino acid sequences set forth in SEQ ID No. 8 and SEQ ID No. 19 or 111, and the S2-C binding site is constituted by the VH and VL comprising a pair of the amino acid sequences set forth in SEQ ID No. 9 and SEQ ID No. 20 or 112;
[69] the bispecific antibody or antibody fragment thereof according to any one of the preceding items [1], [2], [25] and [34] to [36], wherein the two different antigen-binding sites are the S1-B binding site and the S2-D binding site, and wherein the S1-B binding site is constituted by the VH and VL comprising a pair of the amino acid sequences set forth in SEQ ID No. 7 and SEQ ID No. 18 or 110, and the S2-D binding site is constituted by the VH and VL comprising a pair of the amino acid sequences set forth in SEQ ID No. 10 and 21 or 113;
[70] the bispecific antibody or antibody fragment thereof according to any one of the preceding items [1], [2], [25] and [34] to [36], wherein the two different antigen-binding sites are the S1-B binding site and the S2-D binding site, and wherein the S1-B binding site is constituted by the VH and VL comprising a pair of the amino acid sequences set forth in SEQ ID No. 7 and SEQ ID No. 18 or 110, and the S2-D binding site is constituted by the VH and VL comprising a pair of the amino acid sequences set forth in SEQ ID No. 11 and SEQ ID No. 22 or 114;
[71] the bispecific antibody or antibody fragment thereof according to any one of the preceding items [1], [2], [25] and [34] to [36], wherein the two different antigen-binding sites are the S1-B binding site and the S2-D binding site, and wherein the S1-B binding site is constituted by the VH and VL comprising a pair of the amino acid sequences set forth in SEQ ID No. 8 and SEQ ID No. 19 or 111, and the S2-D binding site is constituted by the VH and VL comprising a pair of the amino acid sequences set forth in SEQ ID No. 10 and SEQ ID No. 21 or 113;
[72] the bispecific antibody or antibody fragment thereof according to any one of the preceding items [1], [2], [25] and [34] to [36], wherein the two different antigen-binding sites are the S1-B binding site and the S2-D binding site, and wherein the S1-B binding site is constituted by the VH and VL comprising a pair of the amino acid sequences set forth in SEQ ID No. 8 and SEQ ID No. 19 or 111, and the S2-D binding site is constituted by the VH and VL comprising a pair of the amino acid sequences set forth in SEQ ID No. 11 and SEQ ID No. 22 or 114;
[73] the bispecific antibody or antibody fragment thereof according to any one of the preceding items [1], [2], [25] and [34] to [36], wherein the two different antigen-binding sites are the S2-C binding site and the S2-D binding site, and wherein the S2-C binding site is constituted by the VH and VL comprising a pair of the amino acid sequences set forth in SEQ ID No. 9 and SEQ ID No. 20 or 112, and the S2-D binding site is constituted by the VH and VL comprising a pair of the amino acid sequences set forth in SEQ ID No. 10 and SEQ ID No. 21 or 113;
[74] the bispecific antibody or antibody fragment thereof according to any one of the preceding items [1], [2], [25] and [34] to [36], wherein the two different antigen-binding sites are the S2-C binding site and the S2-D binding site, and wherein the S2-C binding site is constituted by the VH and VL comprising a pair of the amino acid sequences set forth in SEQ ID No. 9 and SEQ ID No. 20 or 112, and the S2-D binding site is constituted by the VH and VL comprising a pair of the amino acid sequences set forth in SEQ ID No. 11 and SEQ ID No. 22 or 114;
[75] a bispecific antibody or an antibody fragment thereof, having antigen-binding sites specifically binding to two different epitopes on the extracellular domains of Allergin-1, respectively,
   wherein the antigen-binding sites do not mutually compete for Allergin-1, and are any two different antigen-binding sites selected from the S1-A binding site, the S1-B binding site, the S2-C binding site and the S2-D binding site, and
   wherein (i) the S1-A binding site can cross-compete for binding to the S1 domain on Allergin-1 with an antibody or an antigen-binding site thereof having the VH and VL comprising any one pair of the amino acid sequences selected from
      (a) SEQ ID No. 1 and SEQ ID No. 104,
      (b) SEQ ID No. 2 and SEQ ID No. 105,
      (c) SEQ ID No. 3 and SEQ ID No. 106,
      (d) SEQ ID No. 4 and SEQ ID No. 107,
      (e) SEQ ID No. 5 and SEQ ID No. 108, and
      (f) SEQ ID No. 6 and SEQ ID No. 109,
   (ii) the S1-B binding site can cross-compete for binding to the S1 domain on Allergin-1 with an antibody or an antigen-binding site thereof having the VH and VL comprising a pair of the amino acid sequences set forth in
      (a) SEQ ID No. 7 and SEQ ID No. 110, or
      (b) SEQ ID No. 8 and SEQ ID No. 111,
   (iii) the S2-C binding site can cross-compete for binding to the S2 domain on Allergin-1 with an antibody or an antigen-binding site thereof having the VH and VL comprising a pair of the amino acid sequences set forth in SEQ ID No. 9 and SEQ ID No. 112, and
   (iv) the S2-D binding site can cross-compete for binding to the S2 domain on Allergin-1 with an antibody or an antigen-binding site thereof having the VH and VL comprising a pair of the amino acid sequences set forth in
      (a) SEQ ID No. 10 and SEQ ID No. 113, or
      (b) SEQ ID No. 11 and SEQ ID No. 114;
[76] a bispecific antibody or an antibody fragment thereof, having antigen-binding sites specifically binding to two different epitopes on the extracellular domains of Allergin-1, respectively,
   wherein the antigen-binding sites do not mutually compete for Allergin-1, and are any two different antigen-binding sites selected from the S1-A binding site, the S1-B binding site, the S2-C binding site and the S2-D binding site, and
   wherein (i) the S1-A binding site is an antigen-binding site of which the binding to the S1 domain on Allergin-1 is cross-competed by an antibody or an antigen-binding site thereof having the VH and VL comprising any one pair of the amino acid sequences selected from
      (a) SEQ ID No. 1 and SEQ ID No. 104,
      (b) SEQ ID No. 2 and SEQ ID No. 105,
      (c) SEQ ID No. 3 and SEQ ID No. 106,
      (d) SEQ ID No. 4 and SEQ ID No. 107,
      (e) SEQ ID No. 5 and SEQ ID No. 108, and
      (f) SEQ ID No. 6 and SEQ ID No. 109,
   (ii) the S1-B binding site is an antigen-binding site of which the binding to the S1 domain on Allergin-1 is cross-competed by an antibody or an antigen-binding site thereof having the VH and VL comprising a pair of the amino acid sequences set forth in
      (a) SEQ ID No. 7 and SEQ ID No. 110, or
      (b) SEQ ID No. 8 and SEQ ID No. 111,
   (iii) the S2-C binding site is an antigen-binding site of which the binding to the S2 domain on Allergin-1 is cross-competed by an antibody or an antigen-binding site thereof having the VH and VL comprising a pair of the amino acid sequences set forth in SEQ ID No. 9 and SEQ ID No. 112, and
   (iv) the S2-D binding site is an antigen-binding site of which the binding to the S2 domain on Allergin-1 is cross-competed by an antibody or an antigen-binding site thereof having a VH and VL comprising a pair of the amino acid sequences set forth in
      (a) SEQ ID No. 10 and SEQ ID No. 113, or
      (b) SEQ ID No. 11 and SEQ ID No. 114;
[77] the bispecific antibody or antibody fragment thereof according to any one of the preceding items [1] to [76], of which one antigen-binding site forms a part of complex of heavy chain/light chain constituting the anti-Allergin-1 bispecific antibody;
[78] the bispecific antibody or antibody fragment thereof according to the preceding item [77], wherein the complex of heavy chain/light chain is formed by any one selected from:
   (a) a combination of a heavy chain having the VH constituting the S1-A binding site and a light chain having the VL constituting the S1-A binding site,
   (b) a combination of a heavy chain having the VH constituting the S1-B binding site and a light chain having the VL constituting the S1-B binding site,
   (c) a combination of a heavy chain having the VH constituting the S2-C binding site and a light chain having the VL constituting the S2-C binding site, and
   (d) a combination of a heavy chain having the VH constituting the S2-D binding site and a light chain having the VL constituting the S2-D binding site;
[79] the bispecific antibody or antibody fragment thereof according to any one of the preceding items [1] to [78], wherein another antigen-binding site is in a form of scFv comprising a VH and VL constituting it;
[80] the bispecific antibody or antibody fragment thereof according to the preceding item [79], wherein the scFv links to the C-terminus of the heavy chain or light chain;
[81] the bispecific antibody or antibody fragment thereof according to the preceding item [79], wherein the scFv links to the C-terminus of the heavy chain;
[82] the bispecific antibody or antibody fragment thereof according to the preceding item [79], wherein the scFv links to the C-terminus of the light chain;
[83] the bispecific antibody or antibody fragment thereof according to any one of the preceding items [79] to [81], (i) having (a) the heavy chain of which the C-terminus is linked with the scFv and (b) the light chain, or (ii) consisting of two same complexes of heavy chain/light chain constituted by the heavy chain and light chain of the preceding item (i);
[84] the bispecific antibody or antibody fragment thereof according to any one of the preceding items [79], [80], and [82], (i) having (a) the heavy chain and (b) the light chain of which the C-terminus is linked with the scFv, or (ii) consisting of two same complexes of heavy chain/light chain constituted by the heavy chain and light chain of the preceding item (i);
[85] the bispecific antibody or antibody fragment thereof according to any one of the preceding items [79] to [84], wherein the antigen-binding site in the complex of heavy chain/light chain is the S1-A binding site, and the antigen-binding site in the form of scFv is any one selected from the S1-B binding site, the S2-C binding site and the S2-D binding site;
[86] the bispecific antibody or antibody fragment thereof according to any one of the preceding items [79] to [84], wherein the antigen-binding site in the complex of heavy chain/light chain is the S1-B binding site, and the antigen-binding site in the form of scFv is any one selected from the S 1-A binding site, the S2-C binding site and the S2-D binding site;
[87] the bispecific antibody or antibody fragment thereof according to any one of the preceding items [79] to [84], wherein the antigen-binding site in the complex of heavy chain/light chain is the S2-C binding site, and the antigen-binding site in the form of scFv is any one selected from the S 1-A binding site, the S1-B binding site and the S2-D binding site;
[88] the bispecific antibody or antibody fragment thereof according to any one of the preceding items [79] to [84], wherein the antigen-binding site in the complex of heavy chain/light chain is the S2-D binding site, and the antigen-binding site in the form of scFv is any one selected from the S 1-A binding site, the S1-B binding site and the S2-C binding site;
[89] the bispecific antibody or antibody fragment thereof according to any one of the preceding items [79] to [84], wherein the antigen-binding site in the complex of heavy chain/light chain is the S1-A binding site, and the antigen-binding site in the form of scFv is the S1-B binding site;
[90] the bispecific antibody or antibody fragment thereof according to any one of the preceding items [79] to [84], wherein the antigen-binding site in the complex of heavy chain/light chain is the S1-A binding site, and the antigen-binding site in the form of scFv is the S2-C binding site;
[91] the bispecific antibody or antibody fragment thereof according to any one of the preceding items [79] to [84], wherein the antigen-binding site in the complex of heavy chain/light chain is the S1-A binding site, and the antigen-binding site in the form of scFv is the S2-D binding site;
[92] the bispecific antibody or antibody fragment thereof according to any one of the preceding items [79] to [84], wherein the antigen-binding site in the complex of heavy chain/light chain is the S1-B binding site, and the antigen-binding site in the form of scFv is the S1-A binding site;
[93] the bispecific antibody or antibody fragment thereof according to any one of the preceding items [79] to [84], wherein the antigen-binding site in the complex of heavy chain/light chain is the S1-B binding site, and the antigen-binding site in the form of scFv is the S2-C binding site;
[94] the bispecific antibody or antibody fragment thereof according to any one of the preceding items [79] to [84], wherein the antigen-binding site in the complex of heavy chain/light chain is the S1-B binding site, and the antigen-binding site in the form of scFv is the S2-D binding site;
[95] the bispecific antibody or antibody fragment thereof according to any one of the preceding items [79] to [84], wherein the antigen-binding site in the complex of heavy chain/light chain is the S2-C binding site, and the antigen-binding site in the form of scFv is the S1-A binding site;
[96] the bispecific antibody or antibody fragment thereof according to any one of the preceding items [79] to [84], wherein the antigen-binding site in the complex of heavy chain/light chain is the S2-C binding site, and the antigen-binding site in the form of scFv is the S1-B binding site;
[97] the bispecific antibody or antibody fragment thereof according to any one of the preceding items [79] to [84], wherein the antigen-binding site in the complex of heavy chain/light chain is the S2-C binding site, and the antigen-binding site in the form of scFv is the S2-D binding site;
[98] the bispecific antibody or antibody fragment thereof according to any one of the preceding items [79] to [84], wherein the antigen-binding site in the complex of heavy chain/light chain is the S2-D binding site, and the antigen-binding site in the form of scFv is the S1-A binding site;
[99] the bispecific antibody or antibody fragment thereof according to any one of the preceding items [79] to [84], wherein the antigen-binding site in the complex of heavy chain/light chain is the S2-D binding site, and the antigen-binding site in the form of scFv is the S1-B binding site;
[100] the bispecific antibody or antibody fragment thereof according to any one of the preceding items [79] to [84], wherein the antigen-binding site in the complex of heavy chain/light chain is the S2-D binding site, and the antigen-binding site in the form of scFv is the S2-C binding site;
[101] the bispecific antibody or antibody fragment thereof according to any one of the preceding items [79] to [100], wherein the scFv links to the C-terminus of the heavy chain or light chain through (a) a direct amide bond or (b) a peptide linker;
[102] the bispecific antibody or antibody fragment thereof according to any one of the preceding items [79] to [101], wherein the N-terminus of the VH constituting the scFv links to the C-terminus of the heavy chain or light chain through (a) a direct amide bond or (b) a peptide linker;
[103] the bispecific antibody or antibody fragment thereof according to any one of the preceding items [79] to [101], wherein the N-terminus of the VL constituting the scFv links to the C-terminus of the heavy chain or light chain through (a) a direct amide bond or (b) a peptide linker;
[104] the bispecific antibody or antibody fragment thereof according to any one of the preceding items [79] to [102], wherein the VH and VL constituting the scFv mutually links in order from the VH to the VL from the N-terminus thereof through (a) a direct amide bond or (b) a peptide linker;
[105] the bispecific antibody or antibody fragment thereof according to any one of the preceding items [79] to [101] and [103], wherein the VH and VL constituting the scFv mutually links in order from the VL to the VH from the N-terminus thereof through (a) a direct amide bond or (b) a peptide linker;
[106] the bispecific antibody or antibody fragment thereof according to any one of the preceding items [101] to [103], wherein the peptide linker for linking the scFv to the C-terminus of the heavy chain or light chain comprises the amino acid sequence set forth in SEQ ID No. 73;
[107] the bispecific antibody or antibody fragment thereof according to the preceding item [104] or [105], wherein the peptide linker for mutually linking the VH and VL constituting the scFv comprises the amino acid sequence set forth in SEQ ID No. 74;
[108] a bispecific antibody or an antibody fragment thereof, having antigen-binding sites specifically binding to two different epitopes on the extracellular domains of Allergin-1, respectively,
   wherein the antigen-binding sites do not mutually compete for Allergin-1, and are any two different ones selected from the S1-A binding site, the S1-B binding site, the S2-C binding site and the S2-D binding site, and
   (I) wherein the S1-A binding site is any one selected from
      (i) an antigen-binding site comprising the VH having
         (a) the VH-CDR1 comprising the amino acid sequence set forth in SEQ ID No. 25,
         (b) the VH-CDR2 comprising the amino acid sequence set forth in SEQ ID No. 26, and
         (c) the VH-CDR3 comprising the amino acid sequence set forth in SEQ ID No. 27, and
            the VL having
         (d) the VL-CDR1 comprising the amino acid sequence set forth in SEQ ID No. 49,
         (e) the VL-CDR2 comprising the amino acid sequence set forth in SEQ ID No. 50, and
         (f) the VL-CDR3 comprising the amino acid sequence set forth in SEQ ID No. 51,
      (ii) an antigen-binding site comprising the VH having
         (a) the VH-CDR1 comprising the amino acid sequence set forth in SEQ ID No. 28,
         (b) the VH-CDR2 comprising the amino acid sequence set forth in SEQ ID No. 29, and
         (c) the VH-CDR3 comprising the amino acid sequence set forth in SEQ ID No. 30, and
            the VL having
         (d) the VL-CDR1 comprising the amino acid sequence set forth in SEQ ID No. 52,
         (e) the VL-CDR2 comprising the amino acid sequence set forth in SEQ ID No. 53, and
         (f) the VL-CDR3 comprising the amino acid sequence set forth in SEQ ID No. 54,
      (iii) an antigen-binding site comprising the VH having
         (a) the VH-CDR1 comprising the amino acid sequence set forth in SEQ ID No. 31,
         (b) the VH-CDR2 comprising the amino acid sequence set forth in SEQ ID No. 32, and
         (c) the VH-CDR3 comprising the amino acid sequence set forth in SEQ ID No. 33, and
            the VL having
         (d) the VL-CDR1 comprising the amino acid sequence set forth in SEQ ID No. 55,
         (e) the VL-CDR2 comprising the amino acid sequence set forth in SEQ ID No. 56, and
         (f) the VL-CDR3 comprising the amino acid sequence set forth in SEQ ID No. 57, and
      (iv) an antigen-binding site comprising the VH having
         (a) the VH-CDR1 comprising the amino acid sequence set forth in SEQ ID No. 34,
         (b) the VH-CDR2 comprising the amino acid sequence set forth in SEQ ID No. 35, and
         (c) the VH-CDR3 comprising the amino acid sequence set forth in SEQ ID No. 36, and
            the VL having
         (d) the VL-CDR1 comprising the amino acid sequence set forth in SEQ ID No. 58,
         (e) the VL-CDR2 comprising the amino acid sequence set forth in SEQ ID No. 59, and
         (f) the VL-CDR3 comprising the amino acid sequence set forth in SEQ ID No. 60;
         the S1-B binding site is
      (v) an antigen-binding site comprising the VH having
         (a) the VH-CDR1 comprising the amino acid sequence set forth in SEQ ID No. 37,
         (b) the VH-CDR2 comprising the amino acid sequence set forth in SEQ ID No. 38, and
         (c) the VH-CDR3 comprising the amino acid sequence set forth in SEQ ID No. 39, and
            the VL having
         (d) the VL-CDR1 comprising the amino acid sequence set forth in SEQ ID No. 61,
         (e) the VL-CDR2 comprising the amino acid sequence set forth in SEQ ID No. 62, and
         (f) the VL-CDR3 comprising the amino acid sequence set forth in SEQ ID No. 63, or
      (vi) an antigen-binding site comprising the VH having
         (a) the VH-CDR1 comprising the amino acid sequence set forth in SEQ ID No. 40,
         (b) the VH-CDR2 comprising the amino acid sequence set forth in SEQ ID No. 41, and
         (c) the VH-CDR3 comprising the amino acid sequence set forth in SEQ ID No. 42, and
            the VL having
         (d) the VL-CDR1 comprising the amino acid sequence set forth in SEQ ID No. 64,
         (e) the VL-CDR2 comprising the amino acid sequence set forth in SEQ ID No. 65, and
         (f) the VL-CDR3 comprising the amino acid sequence set forth in SEQ ID No. 66;

      the S2-C binding site is an antigen-binding site comprising the VH having
         (a) the VH-CDR1 comprising the amino acid sequence set forth in SEQ ID No. 43,
         (b) the VH-CDR2 comprising the amino acid sequence set forth in SEQ ID No. 44, and
         (c) the VH-CDR3 comprising the amino acid sequence set forth in SEQ ID No. 45, and
            the VL having
         (d) the VL-CDR1 comprising the amino acid sequence set forth in SEQ ID No. 67,
         (e) the VL-CDR2 comprising the amino acid sequence set forth in SEQ ID No. 68, and
         (f) the VL-CDR3 comprising the amino acid sequence set forth in SEQ ID No. 69, and
      the S2-D binding site is an antigen-binding site comprising the VH having
         (a) the VH-CDR1 comprising the amino acid sequence set forth in SEQ ID No. 46,
         (b) the VH-CDR2 comprising the amino acid sequence set forth in SEQ ID No. 47, and
         (c) the VH-CDR3 comprising the amino acid sequence set forth in SEQ ID No. 48, and
            the VL having
         (d) the VL-CDR1 comprising the amino acid sequence set forth in SEQ ID No. 70,
         (e) the VL-CDR2 comprising the amino acid sequence set forth in SEQ ID No. 71, and
         (f) the VL-CDR3 comprising the amino acid sequence set forth in SEQ ID No. 72;
   (II) wherein one of the two different antigen-binding sites is an antigen-binding site in a complex of heavy chain/light chain constituting the bispecific antibody, and another is an antigen-binding site in the form of scFv, and wherein
      (i) the antigen-binding site in the complex of heavy chain/light chain is the S1-A binding site and the antigen-binding site in the form of scFv is any one selected from the S1-B binding site, the S2-C binding site and the S2-D binding site,
      (ii) the antigen-binding site in the complex of heavy chain/light chain is the S1-B binding site and the antigen-binding site in the form of scFv is any one selected from the S1-A binding site, the S2-C binding site and the S2-D binding site,
      (iii) the antigen-binding site in the complex of heavy chain/light chain is the S2-C binding site and the antigen-binding site in the form of scFv is any one selected from the S1-A binding site, the S1-B binding site and the S2-D binding site, or
      (iv) the antigen-binding site in the complex of heavy chain/light chain is the S2-D binding site and the antigen-binding site in the form of scFv is any one selected from the S1-A binding site, the S1-B binding site and the S2-C binding site,
   (III) wherein the N-terminus of any one of the VH and VL constituting the scFv links to the C-terminus of the heavy chain or light chain through a peptide linker, and
   (IV) (i) having the heavy chain and light chain in the form described in the preceding items (I) to (III), or (ii) consisting of two same complexes of heavy chain/light chain constituted by the heavy chain and light chain in the form described in the preceding items (I) to (III);
[109] a bispecific antibody to Allergin-1 or an antibody fragment thereof, comprising any two different antigen-binding sites selected from the S1-A binding site, the S1-B binding site, the S2-C binding site and the S2-D binding site,
   (I) wherein the S1-A binding site is any one selected from
      (i) an antigen-binding site comprising the VH having
         (a) the VH-CDR1 comprising the amino acid sequence set forth in SEQ ID No. 25,
         (b) the VH-CDR2 comprising the amino acid sequence set forth in SEQ ID No. 26, and
         (c) the VH-CDR3 comprising the amino acid sequence set forth in SEQ ID No. 27, and
            the VL having
         (d) the VL-CDR1 comprising the amino acid sequence set forth in SEQ ID No. 49,
         (e) the VL-CDR2 comprising the amino acid sequence set forth in SEQ ID No. 50, and
         (f) the VL-CDR3 comprising the amino acid sequence set forth in SEQ ID No. 51,
      (ii) an antigen-binding site comprising the VH having
         (a) the VH-CDR1 comprising the amino acid sequence set forth in SEQ ID No. 28,
         (b) the VH-CDR2 comprising the amino acid sequence set forth in SEQ ID No. 29, and
         (c) the VH-CDR3 comprising the amino acid sequence set forth in SEQ ID No. 30, and
            the VL having
         (d) the VL-CDR1 comprising the amino acid sequence set forth in SEQ ID No. 52,
         (e) the VL-CDR2 comprising the amino acid sequence set forth in SEQ ID No. 53, and
         (f) the VL-CDR3 comprising the amino acid sequence set forth in SEQ ID No. 54,
      (iii) an antigen-binding site comprising the VH having
         (a) the VH-CDR1 comprising the amino acid sequence set forth in SEQ ID No. 31,
         (b) the VH-CDR2 comprising the amino acid sequence set forth in SEQ ID No. 32, and
         (c) the VH-CDR3 comprising the amino acid sequence set forth in SEQ ID No. 33, and
            the VL having
         (d) the VL-CDR1 comprising the amino acid sequence set forth in SEQ ID No. 55,
         (e) the VL-CDR2 comprising the amino acid sequence set forth in SEQ ID No. 56, and
         (f) the VL-CDR3 comprising the amino acid sequence set forth in SEQ ID No. 57, and
      (iv) an antigen-binding site comprising the VH having
         (a) the VH-CDR1 comprising the amino acid sequence set forth in SEQ ID No. 34,
         (b) the VH-CDR2 comprising the amino acid sequence set forth in SEQ ID No. 35, and
         (c) the VH-CDR3 comprising the amino acid sequence set forth in SEQ ID No. 36, and
            the VL having
         (d) the VL-CDR1 comprising the amino acid sequence set forth in SEQ ID No. 58,
         (e) the VL-CDR2 comprising the amino acid sequence set forth in SEQ ID No. 59, and
         (f) the VL-CDR3 comprising the amino acid sequence set forth in SEQ ID No. 60;
         the S1-B binding site is
      (v) an antigen-binding site comprising the VH having
         (a) the VH-CDR1 comprising the amino acid sequence set forth in SEQ ID No. 37,
         (b) the VH-CDR2 comprising the amino acid sequence set forth in SEQ ID No. 38, and
         (c) the VH-CDR3 comprising the amino acid sequence set forth in SEQ ID No. 39, and
            the VL having
         (d) the VL-CDR1 comprising the amino acid sequence set forth in SEQ ID No. 61,
         (e) the VL-CDR2 comprising the amino acid sequence set forth in SEQ ID No. 62, and
         (f) the VL-CDR3 comprising the amino acid sequence set forth in SEQ ID No. 63, or
      (vi) an antigen-binding site comprising the VH having
         (a) the VH-CDR1 comprising the amino acid sequence set forth in SEQ ID No. 40,
         (b) the VH-CDR2 comprising the amino acid sequence set forth in SEQ ID No. 41, and
         (c) the VH-CDR3 comprising the amino acid sequence set forth in SEQ ID No. 42, and
            the VL having
         (d) the VL-CDR1 comprising the amino acid sequence set forth in SEQ ID No. 64,
         (e) the VL-CDR2 comprising the amino acid sequence set forth in SEQ ID No. 65, and
         (f) the VL-CDR3 comprising the amino acid sequence set forth in SEQ ID No. 66;

      the S2-C binding site is an antigen-binding site comprising the VH having
         (a) the VH-CDR1 comprising the amino acid sequence set forth in SEQ ID No. 43,
         (b) the VH-CDR2 comprising the amino acid sequence set forth in SEQ ID No. 44, and
         (c) the VH-CDR3 comprising the amino acid sequence set forth in SEQ ID No. 45, and
            the VL having
         (d) the VL-CDR1 comprising the amino acid sequence set forth in SEQ ID No. 67,
         (e) the VL-CDR2 comprising the amino acid sequence set forth in SEQ ID No. 68, and
         (f) the VL-CDR3 comprising the amino acid sequence set forth in SEQ ID No. 69, and
      the S2-D binding site is an antigen-binding site comprising the VH having
         (a) the VH-CDR1 comprising the amino acid sequence set forth in SEQ ID No. 46,
         (b) the VH-CDR2 comprising the amino acid sequence set forth in SEQ ID No. 47, and
         (c) the VH-CDR3 comprising the amino acid sequence set forth in SEQ ID No. 48, and
            the VL having
         (d) the VL-CDR1 comprising the amino acid sequence set forth in SEQ ID No. 70,
         (e) the VL-CDR2 comprising the amino acid sequence set forth in SEQ ID No. 71, and
         (f) the VL-CDR3 comprising the amino acid sequence set forth in SEQ ID No. 72;
   (II) wherein one of the two different antigen-binding sites is an antigen-binding site in a complex of heavy chain/light chain constituting the bispecific antibody, and another is an antigen-binding site in the form of scFv, and wherein
      (i) the antigen-binding site in the complex of heavy chain/light chain is the S1-A binding site and the antigen-binding site in the form of scFv is any one selected from the S1-B binding site, the S2-C binding site and the S2-D binding site,
      (ii) the antigen-binding site in the complex of heavy chain/light chain is the S1-B binding site and the antigen-binding site in the form of scFv is any one selected from the S1-A binding site, the S2-C binding site and the S2-D binding site,
      (iii) the antigen-binding site in the complex of heavy chain/light chain is the S2-C binding site and the antigen-binding site in the form of scFv is any one selected from the S1-A binding site, the S1-B binding site and the S2-D binding site, or
      (iv) the antigen-binding site in the complex of heavy chain/light chain is the S2-D binding site and the antigen-binding site in the form of scFv is any one selected from the S1-A binding site, the S1-B binding site and the S2-C binding site,
   (III) wherein the N-terminus of any one of the VH and VL constituting the scFv links to the C-terminus of the heavy chain or light chain through a peptide linker, and
   (IV) (i) having the heavy chain and light chain in the form described in the preceding items (I) to (III), or (ii) consisting of two same complexes of heavy chain/light chain constituted by the heavy chain and light chain in the form described in the preceding items (I) to (III);
[110] the bispecific antibody or antibody fragment thereof according to the preceding item [108] or [109], wherein the two different antigen-binding sites are any two selected from
   (i) the S1-A binding site constituted by the VH and VL comprising any one pair of the amino acid sequences selected from
      (a) SEQ ID No. 1 and 12,
      (b) SEQ ID No. 2 and 13,
      (c) SEQ ID No. 3 and SEQ ID No. 14 or 106,
      (d) SEQ ID No. 4 and SEQ ID No. 15 or 107,
      (e) SEQ ID No. 5 and 16, and
      (f) SEQ ID No. 6 and SEQ ID No. 17 or 109,
   (ii) the S1-B binding site constituted by the VH and VL comprising a pair of the amino acid sequences set forth in
      (a) SEQ ID No. 7 and 18, or
      (b) SEQ ID No. 8 and 19,
   (iii) the S2-C binding site constituted by the VH and VL comprising a pair of the amino acid sequences set forth in SEQ ID No. 9 and SEQ ID No. 20 or 112, and
   (iv) the S2-D binding site constituted by the VH and VL comprising a pair of the amino acid sequences set forth in
      (a) SEQ ID No. 10 and SEQ ID No. 21 or 113, or
      (b) SEQ ID No. 11 and 22;
[111] the bispecific antibody or antibody fragment thereof according to the preceding item [108] or [109], wherein the two different antigen-binding sites are any two selected from
   (i) the S1-A binding site constituted by the VH and VL comprising any one pair of the amino acid sequences selected from
      (a) SEQ ID No. 3 and SEQ ID No. 14 or 106,
      (b) SEQ ID No. 4 and SEQ ID No. 15 or 107,
      (c) SEQ ID No. 5 and 16, and
      (d) SEQ ID No. 6 and SEQ ID No. 17 or 109,
   (ii) the S2-C binding site constituted by the VH and VL comprising a pair of the amino acid sequences set forth in SEQ ID No. 9 and SEQ ID No. 20 or 112, and
   (iii) the S2-D binding site constituted by the VH and VL comprising a pair of the amino acid sequences set forth in
      (a) SEQ ID No. 10 and SEQ ID No. 21 or 113, or
      (b) SEQ ID No. 11 and 22;
[112] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 3 and SEQ ID No. 14 or 106, and
   (b) the S2-C binding site constituted by the VH and VL comprising a pair of the amino acid sequences set forth in SEQ ID No. 9 and SEQ ID No. 20 or 112;
[113] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 6 and SEQ ID No. 17 or 109, and
   (b) the S2-D binding site constituted by the VH and VL comprising a pair of the amino acid sequences set forth in SEQ ID No. 10 and SEQ ID No. 21 or 113;
[114] the bispecific antibody or antibody fragment thereof according to the preceding item [108] or [109], wherein the two different antigen-binding sites are any two selected from: the S1-A binding site constituted by the VH and VL comprising any one pair of the amino acid sequences selected from
   (a) SEQ ID No. 3 and SEQ ID No. 14 or 106,
   (b) SEQ ID No. 4 and SEQ ID No. 15 or 107, and
   (c) SEQ ID No. 5 and 16, and
      the S2-D binding site constituted by the VH and VL comprising a pair of the amino acid sequences set forth in
   (d) SEQ ID No. 10 and SEQ ID No. 21 or 113, or
   (e) SEQ ID No. 11 and 22;
[115] the bispecific antibody or antibody fragment thereof according to any one the preceding items [110] to [114], wherein (a) (i) if the S1-A binding site constitutes a scFv, the VL constituting the scFv comprises the amino acid sequence selected from SEQ ID No. 12 to 17, and
   (ii) if the S1-A binding site constitutes a part of a heavy chain/light chain complex, the VL constituting the light chain comprises the amino acid sequence selected from SEQ ID No. 106, 107 and 109,
   (b) (i) if the S1-B binding site constitutes a scFv, the VL constituting the scFv comprises the amino acid sequence set forth in SEQ ID No. 18 or 19, and
   (ii) if the S1-B binding site constitutes a part of a heavy chain/light chain complex, the VL constituting the light chain comprises the amino acid sequence set forth in SEQ ID No. 110 or 111,
   (c) (i) if the S2-C binding site constitutes a scFv, the VL constituting the scFv comprises the amino acid sequence set forth in SEQ ID No. 20, and
   (ii) if the S2-C binding site constitutes a part of a heavy chain/light chain complex, the VL constituting the light chain comprises the amino acid sequence set forth in SEQ ID No. 112, and
   (d) (i) if the S2-D binding site constitutes a scFv, the VL constituting the scFv comprises the amino acid sequence set forth in SEQ ID No. 21 or 22, and
   (ii) if the S2-D binding site constitutes a part of a heavy chain/light chain complex, the VL constituting the light chain comprises the amino acid sequence set forth in SEQ ID No. 113;
[116] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 3 and 14, and
   (b) the S2-C binding site constituted by the VH and VL comprising a pair of the amino acid sequences set forth in SEQ ID No. 9 and 112;
[117] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 3 and 106, and
   (b) the S2-C binding site constituted by the VH and VL comprising a pair of the amino acid sequences set forth in SEQ ID No. 9 and 20;
[118] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 6 and 17, and
   (b) the S2-D binding site constituted by the VH and VL comprising a pair of the amino acid sequences set forth in SEQ ID No. 10 and 113;
[119] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 6 and 109, and
   (b) the S2-D binding site constituted by the VH and VL comprising a pair of the amino acid sequences set forth in SEQ ID No. 10 and 21;
[120] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 3 and 14, and
   (b) the S2-D binding site constituted by the VH and VL comprising a pair of the amino acid sequences set forth in SEQ ID No. 10 and 113;
[121] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 4 and 15, and
   (b) the S2-D binding site constituted by the VH and VL comprising a pair of the amino acid sequences set forth in SEQ ID No. 10 and 113;
[122] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 5 and 16, and
   (b) the S2-D binding site constituted by the VH and VL comprising a pair of the amino acid sequences set forth in SEQ ID No. 10 and 113;
[123] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 3 and 106, and
   (b) the S2-D binding site constituted by the VH and VL comprising a pair of the amino acid sequences set forth in SEQ ID No. 10 and 21;
[124] the bispecific antibody or antibody fragment thereof according to the preceding item [108] or [109], wherein the two different antigen-binding sites are
   (a) the S1-A binding site constituted by the VH and VL comprising a pair of the amino acid sequences set forth in SEQ ID No. 4 and 107, and
   (b) the S2-D binding site constituted by the VH and VL comprising a pair of the amino acid sequences set forth in SEQ ID No. 11 and 22;
[125] the bispecific antibody or antibody fragment thereof according to any one of the preceding items [108] to [124], wherein the VH and VL constituting the scFv are mutually linked in order from the VL to the VH from the N-terminus thereof through a peptide linker comprising the amino acid sequence set forth in SEQ ID No. 74;
[126] the bispecific antibody or antibody fragment thereof according to any one of the preceding items [108] to [125], wherein the scFv is linked to the C-terminus of the heavy chain through a peptide linker comprising the amino acid sequence set forth in SEQ ID No. 73;
[127] the bispecific antibody or antibody fragment thereof according to any one of the preceding items [1] to [126], wherein the epitope to which the S1-A binding site specifically binds (the S1-A epitope) is constituted by at least any one or more, or all of phenylalanine at position 70, arginine at position 71, arginine at position 72, lysine at position 73, histidine at position 75, leucine at position 76, histidine at position 96, glutamic acid at position 97, lysine at position 102, lysine at position 104 and tyrosine at position 113 in Allergin-1 comprising the amino acid sequence set forth in SEQ ID No.115, wherein the epitope to which the S2-D binding (the S2-D epitope) site specifically binds is constituted by at least any one or more, or all of isoleucine at position 132, methionine at position 133, valine at position 134, isoleucine at position 135, glutamine at position 136, threonine at position 137, glutamic acid at position 138, histidine at position 146 and proline at position 215 in the same Allergin-1, wherein the epitope to which the S1-B binding site specifically binds is an epitope different from the S1-A epitope represented by at least the preceding amino acids, and wherein the epitope to which the S2-C binding site specifically binds is an epitope different from the S2-D epitope represented by at least the preceding amino acids;
[128] a bispecific antibody to Allergin-1 or an antibody fragment thereof, comprising any two different antigen-binding sites selected from the S1-A binding site, the S1-B binding site, the S2-C binding site and the S2-D binding site, wherein
   (a) the epitope to which the S1-A binding site specifically binds (the S1-A epitope) is constituted by at least any one or more, or all of phenylalanine at position 70, arginine at position 71, arginine at position 72, lysine at position 73, histidine at position 75, leucine at position 76, histidine at position 96, glutamic acid at position 97, lysine at position 102, lysine at position 104 and tyrosine at position 113 in Allergin-1 comprising the amino acid sequence set forth in SEQ ID No.115,
   (b) the epitope to which the S2-D binding site specifically binds (the S2-D epitope) is constituted by at least any one or more, or all of isoleucine at position 132, methionine at position 133, valine at position 134, isoleucine at position 135, glutamine at position 136, threonine at position 137, glutamic acid at position 138, histidine at position 146 and proline at position 215 in the same Allergin-1,
   (c) the epitope to which the S1-B binding site specifically bind is an epitope different from the S1-A epitope represented by at least the preceding amino acids, and
   (d) the epitope to which the S2-C binding site specifically binds is an epitope different from the S2-D epitope represented by at least the preceding amino acids;
[129] the bispecific antibody or antibody fragment thereof according to any one of the preceding items [77] to [128], wherein both of the heavy chain and light chain are derived from an IgG antibody;
[130] the bispecific antibody or antibody fragment thereof according to the preceding item [129], wherein the heavy chain has the CH1, CH2 and CH3 of IgG antibody and the light chain has the CL of the same antibody;
[131] the bispecific antibody or antibody fragment thereof according to the preceding item [129] or [130], wherein the IgG antibody is an IgG₁ antibody or IgG₄ antibody;
[132] the bispecific antibody or antibody fragment thereof according to the preceding item [129] or [130], wherein the IgG antibody is an IgG₁ antibody;
[133] the bispecific antibody or antibody fragment thereof according to the preceding item [132] , wherein in two heavy chain constant regions of the IgG₁ antibody, leucine at position 235 according to the EU numbering system was substituted with glycine, respectively, and/or glycine at position 236 was substituted with arginine, respectively;
[134] the bispecific antibody or antibody fragment thereof according to the preceding item [132] or [133], wherein lysines at position 447 according to the EU numbering system in two heavy chain constant regions of the IgG1 antibody are deleted, respectively;
[135] the bispecific antibody or antibody fragment thereof according to the preceding item [129] or [130], wherein the IgG antibody is an IgG₄ antibody;
[136] the bispecific antibody or antibody fragment thereof according to the preceding item [135] , wherein serine at position 228 according to the EU numbering system in two heavy chain constant regions of the IgG₄ antibody was substituted with proline, respectively;
[137] the bispecific antibody or antibody fragment thereof according to any one of the preceding items [77] to [132], wherein the heavy chain constant region comprises the amino acid sequence set forth in SEQ ID No. 23 or 99;
[138] the bispecific antibody or antibody fragment thereof according to any one of the preceding items [77] to [132] and [137], wherein the light chain constant region comprises the amino acid sequence set forth in SEQ ID No. 24;
[139] the bispecific antibody or antibody fragment thereof according to any one of the preceding items [77] to [132], wherein the heavy chain constant region and light chain constant region comprise the amino acid sequences set forth in SEQ ID No. 23 or 99, and SEQ ID No. 24, respectively;
[140] the bispecific antibody or antibody fragment thereof according to any one of the preceding items [137] to [139], wherein the heavy chain constant region comprises the amino acid sequences set forth in SEQ ID No. 23 or 99 in which arginine at position 97 was substituted by lysine;
[141] a bispecific antibody to Allergin-1, (i) having the heavy chain and light chain comprising any one pair of the amino acid sequences selected from
   (a) SEQ ID No. 75 and 76,
   (b) SEQ ID No. 77 and 78,
   (c) SEQ ID No. 79 and 78,
   (d) SEQ ID No. 80 and 78,
   (e) SEQ ID No. 81 and 82,
   (f) SEQ ID No. 83 and 82,
   (g) SEQ ID No. 84 and 85,
   (h) SEQ ID No. 86 and 87,
   (i) SEQ ID No. 88 and 89,
   (j) SEQ ID No. 90 and 91,
   (k) SEQ ID No. 90 and 92,
   (l) SEQ ID No. 93 and 94,
   (m) SEQ ID No. 93 and 95,
   (n) SEQ ID No. 96 and 97,
   (o) SEQ ID No. 98 and 78,
   (p) SEQ ID No. 100 and 78,
   (q) SEQ ID No. 101 and 78,
   (r) SEQ ID No. 102 and 78, and
   (s) SEQ ID No. 103 and 85, or
   (ii) consisting of two same complexes of heavy chain/light chain constituted by the heavy chain and light chain of the preceding item (i);
[142] a bispecific antibody to Allergin-1, (i) having the heavy chain and light chain comprising any one pair of the amino acid sequences selected from
   (a) SEQ ID No. 77 and 78,
   (b) SEQ ID No. 79 and 78,
   (c) SEQ ID No. 83 and 82,
   (d) SEQ ID No. 84 and 85,
   (e) SEQ ID No. 98 and 78,
   (f) SEQ ID No. 100 and 78,
   (g) SEQ ID No. 101 and 78,
   (h) SEQ ID No. 102 and 78, and
   (i) SEQ ID No. 103 and 85, or
   (ii) consisting of two same complexes of heavy chain/light chain constituted by the heavy chain and light chain of the preceding item (i);
[143] the bispecific antibody to Allergin-1 according to the preceding item [141] or [142], wherein arginine at position 214 according to the EU numbering system in the constant region of the heavy chain is substituted by lysine, and/or alanine at position 299 in SEQ ID No. 75, 81, 83, 86, 88, 93 and 96 and at position 301 in SEQ ID No. 80 and 90 (corresponding to position 297 according to the EU numbering system according to the EU numbering system in the constant region of the heavy chain) was substituted by asparagine, each independently;
[144] the bispecific antibody or antibody fragment thereof according to any one of the preceding items [1] to [143], wherein cytokine production is sufficiently reduced during administration thereof or within 24 hours after administration thereof;
[145] the bispecific antibody or antibody fragment thereof according to the preceding item [144], wherein the cytokine includes at least one or more selected from IL-2, IL-4, IL-6, IL-10, IFN-γ and TNF-α;
[146] the bispecific antibody or antibody fragment thereof according to any one of the preceding items [1] to [145], which inhibits the binding of TLR9 ligand (preferably, ODN M362, ODN 1668 and ODN 1826, etc.) to Allergin-1;
[147] the bispecific antibody or antibody fragment thereof according to any one of the preceding items [1] to [146], wherein Allergin-1 is human Allergin-1;
[148] the bispecific antibody or antibody fragment thereof according to the preceding item [147], wherein human Allergin-1 is/are one or more selected from human Allergin-1L, human Allergin-1S1 and human Allergin-1S2;
[149] the bispecific antibody or antibody fragment thereof according to any one of the preceding items [1] to [148], which is a monoclonal antibody;
[150] the bispecific antibody or antibody fragment thereof according to any one of the preceding items [1] to [149], which is an isolated antibody;

[1-1] a pharmaceutical composition containing the anti-Allergin-1 bispecific antibody or the antibody fragment thereof according to any one of the preceding items [1] to [150], as an active ingredient;
[1-2] the pharmaceutical composition according to the preceding item [1-1], further containing at least a pharmaceutically acceptable carrier;
[2-1] an agent for suppressing the progression of symptoms of, suppressing the recurrence of, and/or treating cancer, containing the anti-Allergin-1 bispecific antibody or the antibody fragment thereof of any one selected from the preceding items [1] to [150] as an active ingredient;
[2-2] the agent according to the preceding item [2-1], wherein the cancer is solid cancer or hematological cancer;
[2-3] the agent according to the preceding item [2-2], wherein the cancer is solid cancer, and wherein the solid cancer is one or more selected from malignant melanoma (e.g., malignant melanoma in skin, oral cavity, mucosal epithelium or orbit, etc.), non-small cell lung cancer (e.g., squamous non-small cell lung cancer and non-squamous non-small cell lung cancer), small cell lung cancer, head and neck cancer (e.g., oral cancer, nasopharynx cancer, oropharyngeal cancer, hypopharyngeal cancer, laryngeal cancer, salivary gland cancer and tongue cancer), renal cell cancer (e.g., clear cell renal cell cancer), breast cancer, ovarian cancer (e.g., serous ovarian cancer and ovarian clear cell adenocarcinoma), uterine cancer (e.g., cervical cancer and endometrial cancer), anal cancer (e.g., anal canal cancer), colorectal cancer (e.g., MSI-H and/or dMlVm positive colorectal cancer), rectal cancer, colon cancer, hepatocellular carcinoma, esophageal cancer, gastric cancer, esophagogastric junction cancer, pancreatic cancer (e.g., pancreatic duct cancer, insulinoma and intraductal papillary mucinous neoplasm), urine urothelial cancer (e.g., bladder cancer, upper urinary tract cancer, ureteral cancer, renal pelvis and urethral cancer), prostate cancer, fallopian tube cancer, primary peritoneal cancer, malignant pleural mesothelium tumor, gallbladder cancer, bile duct cancer, biliary tract cancer, skin cancer (e.g., uveal melanoma and Merkel cell carcinoma), testicular cancer (germ cell tumor), vagina cancer, vulvar cancer, penile cancer, small intestine cancer, endocrine system cancer, thyroid cancer, parathyroid cancer, adrenal cancer, spinal tumor, neuroblastoma, medulloblastoma, retinoblastoma, neuroendocrine tumor, brain tumor (e.g., gliomas (e.g., glioblastoma and gliosarcoma) and meningioma) and squamous cell carcinomas;
[2-4] the agent according to the preceding item [2-2], wherein the cancer is solid cancer and wherein the solid cancer is bone and soft tissue sarcoma (e.g., Ewing sarcoma, pediatric rhabdomyosarcoma, uterine leiomyosarcoma, chondrosarcoma, lung sarcoma, osteosarcoma and congenital fibrosarcoma) or Kaposi's sarcoma;
[2-5] the agent according to the preceding item [2-2], wherein the cancer is hematological cancer, and wherein the hematological cancer is one or more selected from multiple myeloma, malignant lymphoma (e.g., non-Hodgkin's Lymphoma (e.g., B-cell non-Hodgkin's lymphoma (e.g., precursor B-cell lymphoblastic lymphoma, precursor B-cell acute lymphoblastic leukemia, chronic B-lymphoid leukemia, B-cell precursor leukemia, B-cell prolymphocytic leukemia, lymphoplasmacytic lymphoma, nodal marginal zone B-cell lymphoma, extranodal marginal zone B-cell lymphoma (MALT lymphoma), primary splenic marginal zone B-cell lymphoma, hairy cell leukemia, hairly cell leukemia-variant, follicular lymphoma, pediatric type follicular lymphoma, diffuse large B cell lymphoma, diffuse large B-cell lymphoma, not otherwise specified, splenic diffuse red pulp small B-cell lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM monoclonal gammopathy of undetermined significance, µ heavy chain disease, λ heavy chain disease, α heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, HHV8 positive diffuse large B-cell lymphoma, not otherwise specified, Burkitt-like lymphoma with 11q aberration, high-grade B-cell lymphoma, with MYC and BCL2 and/or BCL6 rearrangement, high-grade B-cell lymphoma, not otherwise specified and B-cell lymphoma, unclassifiable, with intermediate features between diffuse large B-cell lymphoma and classical Hodgkin lymphoma), T/NK-cell non-Hodgkin's lymphoma (e.g., precursor T-cell lymphoblastic lymphoma, chronic T-cell lymphocytic leukemia, T-cell acute lymphocytic leukemia, T-cell large granular lymphoblastic lymphoma, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell leukemia, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the GI tract, hepatosplenic T-cell lymphoma, primary cutaneous CD30 positive T-cell lymphoproliferative disorders, lymphomatoid papulosis, primary cutaneous anaplastic large cell lymphoma, cutaneous T-cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, nodal peripheral T-cell lymphoma with T follicular helper phenotype, anaplastic large cell lymphoma, ALK positive, anaplastic large cell lymphoma, ALK negative and breast implant-associated anaplastic large-cell lymphoma)), and Hodgkin's Lymphoma (e.g., classical Hodgkin's lymphoma (e.g., nodular sclerosis, mixed cellularity, lymphocyte-rich and lymphopenic) and nodular lymphocyte predominant Hodgkin's lymphoma)), leukemia (e.g., acute myelogenous leukemia, acute promyelocytic leukemia, acute lymphoblastic leukemia (lymphoblastic lymphoma), chronic lymphocytic leukemia (small lymphocytic lymphoma), myelodysplastic syndrome, and chronic myelogenous leukemia), primary central nervous system lymphoma and myeloproliferative syndrome;
[2-6] the agent according to the preceding items [2-1] to [2-5], wherein the cancer is pediatric cancer or primary unknown cancer;
[2-7] the agent according to any one of the preceding items [2-1] to [2-6], wherein the cancer is one on which the therapeutic effect of other antineoplastic agent is insufficient or not sufficient;
[2-8] the agent according to any one of the preceding items [2-1] to [2-7], wherein the cancer is one which became worse after treatment with other antineoplastic agents;
[2-9] the agent according to any one of the preceding items [2-1] to [2-6], wherein a patient with cancer has no history of treatment with any other antineoplastic agents;
[2-10] the agent according to any one of the preceding items [2-1] to [2-9], which is prescribed in postoperative adjuvant therapy or preoperative adjuvant therapy;
[2-11] the agent according to any one of the preceding items [2-1] to [2-10], wherein the cancer is incurable or unresectable, metastatic, recurrent, refractory and/or distant metastatic;
[2-12] the agent according to any one of the preceding items [2-1] to [2-11], wherein TPS or CPS is 50% or more, 25% or more, 10% or more, 5% or more or 1% or more;
[2-13] the agent according to any one of the preceding items [2-1] to [2-11], wherein TPS or CPS is less than 50%, less than 25%, less than 10%, less than 5% or less than 1%;
[2-14] the agent according to any one of the preceding items [2-1] to [2-13], wherein TMB is high frequency;
[2-15] the agent according to any one of the preceding items [2-1] to [2-13], wherein TMB is low frequency;
[2-16] the agent according to any one of the preceding items [2-1] to [2-15], which is further prescribed in combination with other antineoplastic agents;
[2-17] the agent according to any one of the preceding items [2-7] to [2-9] and [2-16], wherein the antineoplastic agent is one or more selected from an alkylating agent, platinum preparation, antimetabolite (e.g., antifolate, pyridine metabolism inhibitor and purine metabolism inhibitor), ribonucleotide reductase inhibitor, nucleotide analog, topoisomerase inhibitor, microtubule polymerization inhibitor, microtubule depolymerization inhibitor, antitumor antibiotics, cytokine preparation, anti-hormonal drug, molecular targeting drug and tumor immunotherapeutic drug (preferably, agent without any concerns of developing cytokine release syndrome);
[2-18] the agent according to the preceding item [2-17], wherein other antineoplastic agent is a molecular targeting drug, which is one or more kinds selected from an ALK inhibitor, BCR-ABL inhibitor, EGFR inhibitor, B-RAF inhibitor, VEGFR inhibitor, FGFR inhibitor, c-MET inhibitor, AXL inhibitor, MEK inhibitor, CDK inhibitor, BTK inhibitor, BCL-2 inhibitor, PI3K-δ/γ inhibitor, JAK-1/2 inhibitor, ERK inhibitor, TGFbR1 inhibitor, Cancer cell stemness kinase inhibitor, FAK inhibitor, Syk/FLT3 dual inhibitor, ATR inhibitor, WEE1 kinase inhibitor, Multiple tyrosine kinase inhibitor, mTOR inhibitor, HDAC inhibitor, PARP inhibitor, aromatase inhibitor, EZH2 Inhibitor, Galectin-3 inhibitor, STAT3 inhibitor, DNMT Inhibitor, SMO Inhibitor, HSP90 inhibitor, γ-tubulin specific inhibitor, HIF2α inhibitor, glutaminase inhibitor, E3 ligase inhibitors inhibitor, NRF2 activator, arginase inhibitor, cell cycle inhibitor, Ephrin B4 inhibitor, IAP antagonist, anti-HER2 antibody, anti-HER1 antibody, anti-HER3 antibody, anti-CD40 antibody, anti-CD70 antibody, anti-VEGF antibody, anti-VEGFR1 antibody, anti-VEGFR2 antibody, anti-CD20 antibody, anti-CD30 antibody, anti-CD38 antibody, anti-TNFRSF10B antibody, anti-TNFRSF 10A antibody, anti-MUC1 antibody, anti-MUC5AC antibody, anti-MUC16 antibody, anti-DLL4 antibody, anti-fucosyl GM1 antibody, anti-gpNMB antibody, anti-mesothelin antibody, anti-MMP9 antibody, anti-GD2 antibody, anti-MET antibody, anti-FOLR1 antibody, anti-CD79b antibody, anti-DLL3 antibody, anti-CD51 antibody, anti-EpCAM antibody, anti-CEACAM5 antibody, anti-CEACAM6 antibody, anti-FGFR2 antibody, anti-CD44 antibody, anti-PSMA antibody, anti-Endoglin antibody, anti-IGF1R antibody, anti-TNFSF11 antibody, anti-GUCY2C antibody, anti-SLC39A6 antibody, anti-SLC34A2 antibody, anti-NCAM1 antibody, anti-ganglioside GD3 antibody, anti-AMHR2 antibody, anti-CD37 antibody, anti-IL1RAP antibody, anti-PDGFR2 antibody, anti-CD200 antibody, anti-TAG-72 antibody, anti-SLITRK6 antibody, anti-DPEP3 antibody, anti-CD19 antibody, anti-NOTCH2/3 antibody, anti-tenascin C antibody, anti-AXL antibody, anti-STEAP1 antibody, anti-CTAA16 antibody, anti-CLDN18 antibody, anti-GM3 antibody, anti-PSCA antibody, anti-FN extra domain B antibody, anti-HAVCR1 antibody, anti-TNFRSF4 antibody, anti-HER1-MET bispecific antibody, anti-EpCAM-CD3 bispecific antibody, anti-Ang2-VEGF bispecific antibody, anti-HER2-CD3 bispecific antibody, anti-BER3-IGF1R bispecific antibody, anti-PMSA-CD3 bispecific antibody, anti-HER1-LGR5 bispecific antibody, anti-SSTR2-CD3 bispecific antibody, anti-CD30-CD 16A bispecific antibody, anti-CEA-CD3 bispecific antibody, anti-CD3-CD19 bispecific antibody, anti-IL3RA-CD3 bispecific antibody, anti-GPRC5D-CD3 bispecific antibody, anti-CD20-CD3 bispecific antibody, anti-TNFRSF17-CD3 bispecific antibody, anti-CLEC12A-CD3 bispecific antibody, anti-HER2-HER3 bispecific antibody, anti-FAP antibody/IL-2 fusion protein and anti-CEA antibody/IL-2 fusion protein;
[2-19] the agent according to the preceding item [2-17], wherein other antineoplastic agent is a tumor immunotherapeutic drug, which is one or more selected from an anti-PD-1 antibody, anti-PD-L1 antibody, PD-1 antagonist, PD-L1/VISTA antagonist, PD-L1/TIM3 antagonist, anti-PD-L2 antibody, PD-L1 fusion protein, PD-L2 fusion protein, anti-CTLA-4 antibody, anti-LAG-3 antibody, anti-TIM3 antibody, anti-KIR antibody, anti-BTLA antibody, anti-TIGIT antibody, anti-VISTA antibody, anti-CD137 antibody, anti-CSF-1R antibody or CSF-1R inhibitor, anti-OX40 antibody, anti-HVEM antibody, anti-CD27 antibody, anti-GITR antibody or GITR fused protein, anti-CD28 antibody, anti-CCR4 antibody, anti-B7-H3 antibody, anti-ICOS agonist antibody, anti-CD4 antibody, anti-DEC-205 antibody/NY-ESO-1 fusion protein, anti-SLAMF7 antibody, anti-CD73 antibody, pegylated IL-2, IDO inhibitor, TLR agonist, adenosine A2A receptor antagonist, anti-NKG2A antibody, anti-CSF-1 antibody, immunopotentiating agent, IL-15 superagonist, soluble LAG3, anti-CD47 antibody or CD47 antagonist and IL-12 antagonist;
[2-20] the agent according to the preceding item [2-17], wherein other antineoplastic agent is a tumor immunotherapeutic drug, wherein the tumor immunotherapeutic drug is an anti-PD-1 antibody, an anti-PD-L1 antibody or an anti-CTLA-4 antibody;
[2-21] the agent according to the preceding item [2-20], wherein the tumor immunotherapeutic drug is an anti-PD-1 antibody, which is an antibody selected from Nivolumab, Cemiplimab-rwlc, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, Geptanolimab, AMP-514, STI-A1110, ENUM 388D4, ENUM 244C8, Zimberelimab, CS1003, BAT-1306, Penpulimab, AK103, Ezabenlimab, LZM009, CMAB819, Sym021, SSI-361, JY034, Pucotenlimab, ISU106 and CX-188;
[2-22] the agent according to the preceding item [2-20], wherein the tumor immunotherapeutic drug is an anti-PD-L1 antibody, which is an antibody selected from Atezolizumab, Avelumab, Durvalumab, Manelimab, Pacmilimab, Envafolimab, Cosibelimab, Sugemalimab, BMS-936559, STI-1014, HLX20, Adebrelimab, MSB2311, BGB-A333, KL-A167, AK106, Socazolimab, FAZ053, CBT-502 and JS003;
[2-23] the agent according to the preceding item [2-20], wherein the tumor immunotherapeutic drug is an anti-CTLA-4 antibody, which is an antibody selected from Ipilimumab, Zalifrelimab, Nurulimab and Tremelimumab;
[2-24] the agent according to the preceding item [2-21], wherein if the anti-PD-1 antibody is Nivolumab, it is administered to an adult at
   (a) 1 mg/kg (body weight) per dose every 3 weeks,
   (b) 3 mg/kg (body weight) per dose every 2 weeks,
   (c) 2 mg/kg (body weight) per dose every 3 weeks,
   (d) 80 mg per dose every 3 weeks,
   (e) 240 mg per dose every 2 weeks,
   (f) 360 mg per dose every 3 weeks or
   (g) 480 mg per dose every 4 weeks, of Nivolumab, by intravenous drip infusion;
[2-25] the agent according to the preceding item [2-21], wherein if the anti-PD-1 antibody is Pembrolizumab, it is administered to an adult at
   (a) 200 mg per dose every 3 weeks,
   (b) 400 mg per dose every 6 weeks, or
   (c) 2 mg/kg (body weight) per dose (up to 200 mg per dose) every 3 weeks, of Pembrolizumab, by intravenous drip infusion;
[2-26] the agent according to the preceding item [2-21], wherein if the anti-PD-1 antibody is Cemiplimab-rwlc, it is administered to an adult at 350 mg of Cemiplimab-rwlc per dose every 3 weeks by intravenous drip infusion;
[2-27] the agent according to the preceding item [2-21], wherein if the anti-PD-1 antibody is Dostarlimab, it is administered to an adult at 500 mg of Dostarlimab per dose every 3 weeks, 4 times, and from the fifth dose onwards, which is 3 weeks later, at 1000 mg per dose every 6 weeks, by intravenous drip infusion;
[2-28] the agent according to the preceding item [2-22], wherein if the anti-PD-L1 antibody is Avelumab, it is administered to an adult at 10 mg/kg (body weight) of Avelumab per dose every 2 weeks by intravenous drip infusion;
[2-29] the agent according to the preceding item [2-22], wherein if the anti-PD-L1 antibody is Atezolizumab, it is administered to an adult at
   (a) 840 mg per dose every 2 weeks,
   (b) 1200 mg per dose every 3 weeks, or
   (c) 1680 mg per dose every 4 weeks, of Atezolizumab, by intravenous drip infusion;
[2-30] the agent according to the preceding item [2-22], wherein if the anti-PD-L1 antibody is Durvalumab, it is administered to an adult at 10 mg/kg (body weight) per dose every 2 weeks, of Durvalumab, by intravenous drip infusion;
[2-31] the agent according to the preceding item [2-23], wherein if the anti-CTLA-4 antibody is Ipilimumab, it is administered to an adult at
   (a) 3 mg/kg (body weight) per dose daily, or
   (b) 1 mg/kg (body weight) per dose daily, of Ipilimumab, four times every 3 weeks by intravenous drip infusion;
[2-32] the agent according to any one of the preceding items [2-20] to [2-31], wherein the anti-PD-1 antibody, anti-PD-L1 antibody or anti-CTLA-4 antibody is administered by intravenous drip infusion, over 30 minutes, 60 minutes, 30 to 60 minutes or 60 minutes or more;
[3-1] an intravenous injection formulation containing the anti-Allergin-1 bispecific antibody or antibody fragment thereof according to any one of the preceding items [1] to [150] and at least a pharmaceutically acceptable carrier;
[3-2] the intravenous injection formulation according to the preceding item [3-1] for use in suppressing the progression of symptoms of, suppressing the recurrence of, and/or treating cancer;
[3-3] the intravenous injection formulation according to the preceding item [3-1] or [3-2] for use in drip infusion;
[4-1] a method for suppressing the progression of symptoms of, suppressing the recurrence of, and/or treating cancer, comprising administering to a patient an effective amount of the anti-Allergin-1 bispecific antibody or antibody fragment thereof of any one selected from the preceding items [1] to [150];
[5-1] an anti-Allergin-1 bispecific antibody or an antibody fragment thereof of any one selected from the preceding items [1] to [150] for use in suppressing the progression of symptoms of, suppressing the recurrence of and/or treating cancer;
[6-1] use of the anti-Allergin-1 bispecific antibody or antibody fragment thereof of any one selected from the preceding items [1] to [150] for manufacturing a drug for suppressing the progression of symptoms of, suppressing the recurrence of and/or treating cancer;
[7-1] an antibody specifically binding to the S1 domain on Allergin-1 (anti-S1 domain antibody) or an antibody fragment thereof, which cross-competes for binding to the S1 domain on Allergin-1 with an antibody or an antigen-binding site thereof having the VH and VL comprising any one pair of the amino acid sequences selected from
   (a) SEQ ID No. 1 and 104,
   (b) SEQ ID No. 2 and 105,
   (c) SEQ ID No. 3 and 106,
   (d) SEQ ID No. 4 and 107,
   (e) SEQ ID No. 5 and 108,
   (f) SEQ ID No. 6 and 109,
   (g) SEQ ID No. 7 and 110, and
   (h) SEQ ID No. 8 and 111;
[7-2] an anti-S1 domain antibody or an antibody fragment thereof, wherein the binding to the S 1 domain on Allergin-1 of the antibody or antibody fragment thereof is cross-competed by an antibody or an antigen-binding site thereof having the VH and VL comprising any one pair of the amino acid sequences selected from
   (a) SEQ ID No. 1 and 104,
   (b) SEQ ID No. 2 and 105,
   (c) SEQ ID No. 3 and 106,
   (d) SEQ ID No. 4 and 107,
   (e) SEQ ID No. 5 and 108,
   (f) SEQ ID No. 6 and 109,
   (g) SEQ ID No. 7 and 110, and
   (h) SEQ ID No. 8 and 111;
[7-3] an antibody specifically binding to the S2 domain on Allergin-1 (anti-S2 domain antibody) or an antibody fragment thereof, which cross-competes for binding to the S2 domain on Allergin-1 with an antibody or an antigen-binding site thereof having the VH and VL comprising any one pair of the amino acid sequences selected from
   (a) SEQ ID No. 9 and 112,
   (b) SEQ ID No. 10 and 113, and
   (c) SEQ ID No. 11 and 114;
[7-4] an anti-S2 domain antibody or an antibody fragment thereof, wherein the binding to the S2 domain on Allergin-1 of the antibody or antibody fragment thereof is cross-competed by an antibody or an antigen-binding site thereof having the VH and VL comprising any one pair of the amino acid sequences selected from:
   (a) SEQ ID No. 9 and 112,
   (b) SEQ ID No. 10 and 113, and
   (c) SEQ ID No. 11 and 114;
[7-5] the antibody or antibody fragment thereof according to the preceding item [7-1] or [7-2], having any one pair of the VH and VL selected from
   (A) the VH having
      (a) the VH-CDR1 comprising the amino acid sequence set forth in SEQ ID No. 25,
      (b) the VH-CDR2 comprising the amino acid sequence set forth in SEQ ID No. 26, and
      (c) the VH-CDR3 comprising the amino acid sequence set forth in SEQ ID No. 27, and
         the VL having
      (d) the VL-CDR1 comprising the amino acid sequence set forth in SEQ ID No. 49,
      (e) the VL-CDR2 comprising the amino acid sequence set forth in SEQ ID No. 50, and
      (f) the VL-CDR3 comprising the amino acid sequence set forth in SEQ ID No. 51,
   (B) the VH having
      (a) the VH-CDR1 comprising the amino acid sequence set forth in SEQ ID No. 28,
      (b) the VH-CDR2 comprising the amino acid sequence set forth in SEQ ID No. 29, and
      (c) the VH-CDR3 comprising the amino acid sequence set forth in SEQ ID No. 30, and
         the VL having
      (d) the VL-CDR1 comprising the amino acid sequence set forth in SEQ ID No. 52,
      (e) the VL-CDR2 comprising the amino acid sequence set forth in SEQ ID No. 53, and
      (f) the VL-CDR3 comprising the amino acid sequence set forth in SEQ ID No. 54,
   (C) the VH having
      (a) the VH-CDR1 comprising the amino acid sequence set forth in SEQ ID No. 31,
      (b) the VH-CDR2 comprising the amino acid sequence set forth in SEQ ID No. 32, and
      (c) the VH-CDR3 comprising the amino acid sequence set forth in SEQ ID No. 33, and
         the VL having
      (d) the VL-CDR1 comprising the amino acid sequence set forth in SEQ ID No. 55,
      (e) the VL-CDR2 comprising the amino acid sequence set forth in SEQ ID No. 56, and
      (f) the VL-CDR3 comprising the amino acid sequence set forth in SEQ ID No. 57,
   (D) the VH having
      (a) the VH-CDR1 comprising the amino acid sequence set forth in SEQ ID No. 34,
      (b) the VH-CDR2 comprising the amino acid sequence set forth in SEQ ID No. 35, and
      (c) the VH-CDR3 comprising the amino acid sequence set forth in SEQ ID No. 36, and
         the VL having
      (d) the VL-CDR1 comprising the amino acid sequence set forth in SEQ ID No. 58,
      (e) the VL-CDR2 comprising the amino acid sequence set forth in SEQ ID No. 59, and
      (f) the VL-CDR3 comprising the amino acid sequence set forth in SEQ ID No. 60,
   (E) the VH having
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39, and
         the VL having
      (d) the VL-CDR1 comprising the amino acid sequence set forth in SEQ ID No. 61,
      (e) the VL-CDR2 comprising the amino acid sequence set forth in SEQ ID No. 62, and
      (f) the VL-CDR3 comprising the amino acid sequence set forth in SEQ ID No. 63, and
   (F) the VH having
      (a) the VH-CDR1 comprising the amino acid sequence set forth in SEQ ID No. 40,
      (b) the VH-CDR2 comprising the amino acid sequence set forth in SEQ ID No. 41, and
      (c) the VH-CDR3 comprising the amino acid sequence set forth in SEQ ID No. 42, and
         the VL having
      (d) the VL-CDR1 comprising the amino acid sequence set forth in SEQ ID No. 64,
      (e) the VL-CDR2 comprising the amino acid sequence set forth in SEQ ID No. 65, and
      (f) the VL-CDR3 comprising the amino acid sequence set forth in SEQ ID No. 66;
[7-6] the antibody or antibody fragment thereof according to the preceding item [7-3] or [7-4], having a pair of (A) the VH having
   (a) the VH-CDR1 comprising the amino acid sequence set forth in SEQ ID No. 43,
   (b) the VH-CDR2 comprising the amino acid sequence set forth in SEQ ID No. 44, and
   (c) the VH-CDR3 comprising the amino acid sequence set forth in SEQ ID No. 45, and
      the VL having
   (d) the VL-CDR1 comprising the amino acid sequence set forth in SEQ ID No. 67,
   (e) the VL-CDR2 comprising the amino acid sequence set forth in SEQ ID No. 68, and
   (f) the VL-CDR3 comprising the amino acid sequence set forth in SEQ ID No. 69, or
   (B) the VH having
   (a) the VH-CDR1 comprising the amino acid sequence set forth in SEQ ID No. 46,
   (b) the VH-CDR2 comprising the amino acid sequence set forth in SEQ ID No. 47, and
   (c) the VH-CDR3 comprising the amino acid sequence set forth in SEQ ID No. 48, and
      the VL having
   (d) the VL-CDR1 comprising the amino acid sequence set forth in SEQ ID No. 70,
   (e) the VL-CDR2 comprising the amino acid sequence set forth in SEQ ID No. 71, and
   (f) the VL-CDR3 comprising the amino acid sequence set forth in SEQ ID No. 72;
[7-7] the antibody or antibody fragment thereof according to the preceding item [7-5] or [7-6], wherein one to five arbitrary amino acid residues in any one or more of CDRs selected from VH-CDR1, VH-CDR2, VH-CDR3, VL-CDR1, VL-CDR2 and VL-CDR3 are substituted with other amino acids, respectively;
[7-8] the antibody or antibody fragment thereof according to any one of the preceding items [7-1] to [7-7], wherein the FWs in the VH and VL comprise the amino acid sequences coding the germ-line VH gene IGHV3-66 and VL gene IGKV1-39 or genes thereof with one or more somatic mutations and/or back mutations, respectively;
[7-9] the antibody or antibody fragment thereof according to any one of the preceding items [7-1], [7-2], [7-5], [7-7], and [7-8], having the VH and VL comprising any one pair of the amino acid sequences selected from:
   (a) SEQ ID No. 1 and 104,
   (b) SEQ ID No. 2 and 105,
   (c) SEQ ID No. 3 and 106,
   (d) SEQ ID No. 4 and 107,
   (e) SEQ ID No. 5 and 108,
   (f) SEQ ID No. 6 and 109,
   (g) SEQ ID No. 7 and 110, and
   (h) SEQ ID No. 8 and 111, or a pair of amino acid sequences having at least 80%, 90%, 95%, 98% or 99% identity to the respective same amino acid sequences (excluding three CDRs, respectively), each independently;
[7-10] the antibody or antibody fragment thereof according to any one of the preceding items [7-3], [7-4] and [7-6] to [7-8], having the VH and VL comprising any one pair of the amino acid sequences selected from
   (a) SEQ ID No. 9 and 112,
   (b) SEQ ID No. 10 and 113, and
   (c) SEQ ID No. 11 and 114, or a pair of amino acid sequences having at least 80%, 90%, 95%, 98% or 99% identity to the respective same amino acid sequences (excluding three CDRs, respectively), each independently;
[7-11] an antibody to Allergin-1 or an antibody fragment thereof, having any one antigen-binding site selected from the S1-A binding site, the S1-B binding site, the S2-C binding site and the S2-D binding site, wherein
   (a) the epitope to which the S1-A binding site specifically binds (the S1-A epitope) is constituted by at least any one or more, or all of phenylalanine at position 70, arginine at position 71, arginine at position 72, lysine at position 73, histidine at position 75, leucine at position 76, histidine at position 96, glutamic acid at position 97, lysine at position 102, lysine at position 104 and tyrosine at position 113 in Allergin-1 comprising the amino acid sequence set forth in SEQ ID No.115,
   (b) the epitope to which the S2-D binding site specifically binds (the S2-D epitope) is constituted by at least any one or more, or all of isoleucine at position 132, methionine at position 133, valine at position 134, isoleucine at position 135, glutamine at position 136, threonine at position 137, glutamic acid at position 138, histidine at position 146 and proline at position 215 in the same Allergin-1,
   (c) the epitope to which the S1-B binding site specifically bind is an epitope different from the S1-A epitope represented by at least the preceding amino acids, and
   (d) the epitope to which the S2-C binding site specifically binds is an epitope different from the S2-D epitope represented by at least the preceding amino acids;
[7-12] the antibody or antibody fragment thereof according to any one of the preceding items [7-1] to [7-11], which is an IgG antibody;
[7-13] the antibody or antibody fragment thereof according to the preceding item [7-12], wherein the IgG antibody is an IgG₁ antibody or IgG₄ antibody;
[7-14] the antibody or antibody fragment thereof according to any one of the preceding items [7-1] to [7-13], wherein Allergin-1 is human Allergin-1;
[7-15] the antibody or antibody fragment thereof according to the preceding item [7-14], wherein human Allergin-1 is/are one or more selected from human Allergin-1L, human Allergin-1S1 and human Allergin-1S2;
[7-16] the antibody or antibody fragment thereof according to any one of the preceding items [7-1] to [7-7] and [7-11] to [7-15], which is a rabbit antibody;
[7-17] the antibody or antibody fragment thereof according to any one of the preceding items [7-1] to [7-7] and [7-11] to [7-16], which is a chimeric antibody;
[7-18] the antibody or antibody fragment thereof according to any one of the preceding items [7-1] to [7-7] and [7-11] to [7-16], which is a humanized antibody;
[7-19] the antibody or antibody fragment thereof according to any one of the preceding items [7-1] to [7-18], which is a monoclonal antibody;
[7-20] the antibody or antibody fragment thereof according to any one of the preceding items [7-1] to [7-19], which is an isolated antibody;
[8-1] a polynucleotide coding any one selected from the amino acid sequences set forth in SEQ ID No. 1 to 24 and SEQ ID No. 75 to 114;
[8-2] an expression vector containing any of the polynucleotides described in the preceding item [8-1]; and
[8-3] a mammal cell into which the expression vector described in the preceding item [8-2] is transferred, or which is transformed by transferring the same vector.

The contents of all patent documents and non-patent documents or references explicitly cited in the present specification may be incorporated herein as part of the present specification.

The present invention will be described in more detail by the following Examples, but the scope of the present invention is not limited thereto. Various changes or modifications can be made by those skilled in the art based on the description of the present invention, and these changes or modifications are also included in the present invention.

### EXAMPLES

### Example 1: Immunization in rabbits with recombinant human Allergin-1L-His fusion protein and expression plasmid vector for human Allergin-1L

In order to obtain antibodies that specifically bind to human Allergin-1 (including an anti-S1-domain antibody and anti-S2-domain antibody), the recombinant human Allergin-1L protein was immunized in rabbits. In addition, expression vectors containing cDNAs encoding human Allergin-1L, human Allergin-1S1, and human Allergin-1S2, respectively, were similarly immunized in rabbits.

In order to immunize the recombinant human Allergin-1L protein, the recombinant human Allergin-1L-His fusion protein emulsified using incomplete Freund's adjuvant and complete Freund's adjuvant (Sino Biological, model number 16083-H08H) was immunized in rabbits (nine rabbits) by subcutaneous administration four times over 56 days or five times over 104 days. Blood was collected from immunized rabbits, and the antibody titer in serum was evaluated by ELISA using the recombinant human Allergin-1L-His fusion protein or by Cell ELISA (CELISA) using CHO cell lines stably expressing human Allergin-1L.

In order to immunize rabbits (11 rabbits) with expression vectors containing cDNAs encoding human Allergin-1, human Allergin-1L, human Allergin-1S1, and human Allergin-1S2, respectively, were immunized by subcutaneous administration 8 times over 114 days. Blood was collected from immunized rabbits, and the antibody titer in serum was evaluated by CELISA using CHO cell lines stably expressing human Allergin-1L, human Allergin-1S1 and human Allergin-1S2, respectively.

Spleens were removed from individuals with elevated serum antibody titers, and among lymphocytes prepared from them, the percentage of cell population to which fluorescence-labeled recombinant human Allergin-1L-His fusion protein binds was analyzed by flow cytometry to calculate the percentage of B cells which produce antigen-specific antibodies. Splenic lymphocytes from individuals with high values in the serum antibody titer and the percentage of antigen-specific antibody-producing B cells in splenic lymphocytes were used to select B cells producing anti-Allergin-1 antibodies.

### Example 2: Screening of B cells producing anti-Allergin-1 antibody

From splenic lymphocytes collected individually from rabbits selected in Example 1, the cells to which fluorescence-labeled recombinant human Allergin-1L-His fusion protein binds, i.e., B cells producing antigen-specific antibodies, were isolated by flow cytometry. The isolated B cells were cultured, and the binding of antibody in culture supernatant therefrom to the recombinant human Allergin-1L protein was analyzed by ELISA, and the binding to human Allergin-1L expressed on membrane was analyzed by CELISA. Furthermore, the antibody clones showing binding in either of analysis were analyzed by CELISA for binding to CHO cells stably expressing human Allergin-1S1, human Allergin-1S2, and monkey Allergin-1, respectively. In said CELISA, the clone showing the signal (OD value) which was at least 10% of the signal (OD value) in positive control well (using acquired anti-human Allergin-1 antibody) was defined as a positive clone. Furthermore, the binding of antibody in the culture supernatant to recombinant human Allergin-1L protein was analyzed by surface plasmon resonance (SPR) using MASS-2 SPR device (Sierra Sensors) to calculate dissociation constant (KD value). This analysis was performed by binding rabbit antibodies in the culture supernatant to anti-rabbit IgG antibodies immobilized on a sensor chip (SPR-2 Affinity sensor, High Capacity Amine, Bruker LabScape) and then flowing the recombinant human Allergin-1L protein as an anolyte into a flow cell. As a result of this analysis, several antibody clones, including positive clones binding to both human Allergin-1L and monkey Allergin-1, were selected.

### Example 3: DNA sequencing of candidate clones for anti-Allergin-1 antibody

Each RNA was extracted from the positive clone of B cells isolated in Example 2, respectively, from which cDNA was prepared by reverse transcription reaction. PCR was performed using primers specific for the prepared cDNA and the respective variable regions of the immunoglobulin heavy and light chains, respectively. The amplified PCR products were subjected to DNA sequencing to determine DNA sequences encoding the respective variable region sequences of the heavy and light chains in the antibodies produced by the B cell clones.

### Example 4: Evaluation of binding to Allergin-1 expressing cells using rabbit IgG format and screening by competitive binding assay

Expression vectors encoding the heavy and light chains of the rabbit antibody of which DNA sequence was determined in Example 3, respectively, were produced, and both of the vectors were transfected into mammalian cells to produce antibodies in culture supernatant. Rabbit IgG was purified by collecting the culture supernatant and processing it by protein A affinity chromatography.

The bindings of the purified rabbit IgG to CHO cells stably expressing human Allergin-1L, human Allergin-1S1, human Allergin-1S2 and monkey Allergin-1, respectively, were analyzed by CELISA. In detail, CHO cells stably expressing each Allergin-1 were seeded in 96-well plates, to which diluted rabbit IgG was added, and which were incubated at 37°C. After washing the CHO cells, to which diluted HRP (Horseradish peroxidase)-conjugated goat anti-rabbit IgG antibody was added, and which were incubated at 37°C again. After washing the CHO cells again, to which HRP chromogenic substrate was added, and which were incubated at room temperature, and then of which absorbance was measured.

Competitive binding assay was performed to evaluate the cross-competitiveness of the 34 anti-S1 domain antibodies that were confirmed to bind to human Allergin-1L and human Allergin-1S1 by CELISA, and the 32 anti-S2 domain antibodies that were confirmed to bind to human Allergin-1L and human Allergin-1S2. In detail, two kinds of arbitrary antibodies from the 34 anti-S1 domain antibodies were selected, and one as a Competitor antibody and the other as a Detector antibody were defined, and the binding of the Detector antibody to human Allergin-1L in the presence of the Competitor antibody was evaluated. Initially, the Competitor antibody was added to CHO cells expressing human Allergin-1L, and which were incubated on ice. The same volume of buffer was added to samples to which the Competitor antibody was not added. Next, biotin-labeled rabbit IgG was added to the Competitor antibody as a Detector antibody, and which was incubated on ice. After washing the CHO cells, to which APC (allophycocyanin)-labeled streptavidin was added, and which were incubated. After washing the CHO cells again, the binding amount of the Detector antibody (MFI value) was measured by flow cytometry, and the binding amount of the Detector antibody in the presence of the Competitor antibody was calculated in setting the binding amount of the Detector antibody in the absence of the Competitor antibody at 100. A similar analysis was performed for 32 anti-S2 domain antibodies.

As a result of these competitive binding assays, the pattern of cross-competitiveness for each obtained antibody clone was analyzed to classify antibodies showing similar patterns with respect to binding to human Allergin-1L into four groups (S1-A epitope, S1-B epitope, S2-C epitope and S2-D epitope) sharing the same epitope.

### Example 5: Evaluation of binding of humanized scFv format to Allergin-1 expressing cells and of stability thereof

Humanized scFv was designed from rabbit antibodies to reduce its immunogenicity to human and stabilize it in scFv structure. In detail, initially, for the rabbit antibodies selected and evaluated in Examples 3 and 4, the complementary chain-determining regions (VH-CDR1, VH-CDR2, VH-CDR3, VL-CDR1, VL-CDR2 and VL-CDR3) of their heavy and light chains were determined, respectively. The determined DNA sequences encoding the 6 CDRs were designed to be transferred to DNA sequences encoding framework regions in the heavy and light chains of human antibodies, respectively, by a method known to those skilled in the art. Next, the DNA encoding the transplanted heavy and light chains, respectively, was recombined to be expressed in the form of scFv, by a method known to those skilled in the art, and the recombined DNA was inserted into an expression vector for mammalian cells. The expression vector was gene-transfected into mammalian cells to produce scFv in culture supernatant, and which was collected, and then humanized scFv was purified therefrom by Protein L affinity chromatography.

The purified humanized scFv was analyzed for binding to CHO cells stably expressing human Allergin-1L and monkey Allergin-1, by CELISA, respectively. In detail, the CHO cells were seeded in 96-well plates, to which diluted humanized scFv was added, and which were incubated at 37°C. After washing the CHO cells, to which diluted HRP-labeled Protein L was added, and which were incubated again at 37°C. After washing the CHO cells again, to which HRP chromogenic substrate was added, and which were incubated at room temperature, and then in which absorbance was measured. As a result, the bindings to human Allergin-1L and monkey Allergin-1 were confirmed in several clones.

Furthermore, for each humanized scFv, the binding to Fc-fused human Allergin-1L recombinant protein was analyzed by SPR method. In detail, Fc-fused human Allergin-1L recombinant protein was immobilized on a sensor chip, followed by flowing humanized scFv as an analyte into a flow cell. As a result, the bindings to human Allergin-1L were confirmed in several clones.

From results of the above series of binding evaluations, 17 humanized scFvs were selected for stability evaluation, taking into consideration the diversity of epitope groups. In detail, 50 mM phosphate-citrate buffer solution (containing humanized scFv: 10 mg/mL or more; 150 mM NaCl; pH 6.4) containing each selected humanized scFv was stored at -80°C or less, 4°C or less and 40°C or less for four weeks, respectively. After 1, 7, 14, 21, 28, and 35 days from storage, the changes in monomer ratio were evaluated by SE-HPLC, respectively, and the changes in protein concentration were measured by absorbance measurement (280 nm), respectively. In order to evaluate the effect of freezing and thawing on stability, the changes in monomer ratio after up to four freeze-thaw cycles were evaluated by SE-HPLC, respectively. Furthermore, thermal stabilities were evaluated using Differential Scanning Fluorimetry (DSF), respectively.

Taking into consideration the binding to human Allergin-1L and monkey Allergin-1 based on CELISA and SPR as well as the diversity among epitope groups, eight humanized scFvs shown in Table 1 were selected from the humanized scFvs, showing a small decrease in monomer ratio in 14-day stability evaluation, as humanized scFvs with VH and VL for subsequent design of anti-Allergin-1 bispecific antibodies.

**[Table 1]**

| Binding domain in Allergin-1 | Epitope group in Allergin-1 | Humanized scFv | VH/VL Construct | |
|---|---|---|---|---|
| | | | VH SEQ ID No. | VL SEQ ID No. |
| S1 | S1-A | Pro1751 | 1 | 12 |
| | | Pro1565 | 2 | 13 |
| | | Pro1588 | 3 | 14 |
| | | Pro1570 | 6 | 17 |
| | S1-B | Pro1742 | 7 | 18 |
| | | Pro1707 | 8 | 19 |
| S2 | S2-C | Pro1586 | 9 | 20 |
| | S2-D | Pro1571 | 10 | 21 |

### Example 6: Design and production of anti-Allergin-1 bispecific antibody

Two kinds of different humanized scFvs were arbitrarily selected from the eight humanized scFvs selected in Example 5, and each anti-Allergin-1 bispecific antibody was designed to have two binding sites derived from the two humanized scFvs, respectively. In detail, it was designed so that among two kinds selected from the eight humanized scFvs, which do not compete with each other (confirmed in the competitive binding assay shown in Example 4), the VH and VL in one humanized scFv comprise its Fab region and the other humanized scFv is linked to C-terminus of its heavy chain or light chain, which is in the form of Morrison form antibody. When linking the humanized scFv to C-terminus of the heavy chain or light chain, Gly-Gly-Gly-Gly-Gly-Gly-Gly-Gly-Ser (SEQ ID No. 73) was used as its linker sequence. DNAs encoding the heavy chain and light chain constituting the anti-Allergin-1 bispecific antibody, respectively, and expression vectors thereof were generated by a genetic recombination method known to those skilled in the art, according to the above design. pcDNA3.4 (Thermo Fisher Scientific) was used as an expression vector. Then, each produced expression vector was gene-transfected into HEK293 cells, simultaneously, and antibodies were secreted in culture supernatant. The culture supernatant was collected, and processed by protein A affinity chromatography to purify the desired anti-Allergin-1 bispecific antibody. The compositions of some of the anti-Allergin-1 bispecific antibodies produced here are shown in Table 2.

**[Table 2]**

| Anti-Allergin-1 bispecific antibody clones | Used humanized scFv clones | | Heavy chain SEQ ID No. | Light chain SEQ ID No. |
|---|---|---|---|---|
| | Fab | scFv | | |
| #11_h | Pro1586 | Pro1588 | 75 | 76 |
| #15_h | Pro1571 | Pro1588 | 77 | 78 |
| #16_h | Pro1571 | Pro1570 | 80 | 78 |
| #11p_h | Pro1588 | Pro1586 | 81 | 82 |
| #15p_h | Pro1588 | Pro1571 | 83 | 82 |
| #16p_h | Pro1570 | Pro1571 | 86 | 87 |
| #11_L | Pro1586 | Pro1588 | 88 | 89 |
| #15_L | Pro1571 | Pro1588 | 90 | 91 |
| #16_L | Pro1571 | Pro1570 | 90 | 92 |
| #11p_L | Pro1588 | Pro1586 | 93 | 94 |
| #15p_L | Pro1588 | Pro1571 | 93 | 95 |
| #16p_L | Pro1570 | Pro1571 | 96 | 97 |

### Example 7: Evaluation of the shielding degree of binding of the anti-Allergin-1 bispecific antibody to Allergin-1

Since antibodies covering extracellular portion more widely are preferred for receptors with unknown ligands, the shielding degree by binding to Allergin-1 of the anti-Allergin-1 bispecific antibody produced in Example 6 was evaluated.

Initially, the respective anti-Allergin-1 bispecific antibodies (antibody clone #1 to #20 in the table in Figure 16), which were prepared according to the same process as in Example 6, were added to human Allergin-1L-expressing CHO cell lines, respectively, which were incubated on ice. Furthermore, a biotin-labeled anti-Allergin-1 antibody (rabbit IgG obtained in Example 4: each antibody listed in the vertical column of "Rabbit IgG ID No." in the table in Figure 16) was added thereto, respectively, and which were incubated again on ice. After washing the CHO cells, to which APC-labeled streptavidin was added, and which were incubated again on ice. After washing said CHO cells again, the binding amount of the biotin-labeled anti-Allergin-1 antibody was evaluated in a flow cytometer. The results are shown in Figure 16.

The respective anti-Allergin-1 bispecific antibodies were confirmed to inhibit the binding of many kinds of anti-Allergin-1 antibodies to Allergin-1L and to widely shield the surface of Allergin-1L.

### Example 8: Generation of mutants of anti-Allergin-1 bispecific antibody

Using site-directed mutagenesis technique known to those skilled in the art, mutants keeping the ability of anti-Allergin-1 bispecific antibody to recognize its antigen were generated.

Anti-Allergin-1 bispecific antibody #15_h clone produced in Example 6 was subjected to site-directed mutagenesis so that aspartic acid at position 461 with alanine, serine at position 474 with arginine, aspartic acid at position 532 with glutamine, threonine at position 565 with cysteine and glycine at position 637 with cysteine in SEQ ID No. 77 representing the amino acid sequence of its heavy chain, were replaced, respectively, to generate anti-Allergin-1 bispecific antibody #15_h_opt clone having a heavy chain comprising the amino acid sequence of SEQ ID No. 79.

In addition, #15_h clone was separately subjected to site-directed mutagenesis so that aspartic acid at position 461 with alanine, serine at position 474 with arginine, and aspartic acid at position 532 with glutamine in SEQ ID No. 77 were replaced, respectively, to generate anti-Allergin-1 bispecific antibody #15_h_optv2 clone having a heavy chain comprising the amino acid sequence of SEQ ID No. 98.

Anti-Allergin-1 bispecific antibody #15p_h clone generated by replacing two antigen recognition sites (Fab side and scFv side) of #15_h clone each other was subjected to (i) site-directed mutagenesis so that threonine at position 562 with cysteine and glycine at positions 634 with cysteine in SEQ ID No 83 representing the amino acid sequence of its heavy chain, were replaced, respectively, to generate a heavy chain comprising the amino acid sequence of SEQ ID No 84, and (ii) site-directed mutagenesis so that aspartic acid at position 1 with alanine, serine at position 14 with arginine and aspartic acid at positions 72 with glutamine in SEQ ID No 82 representing the amino acid sequence of its light chain, were replaced, respectively, to generate a light chain comprising the amino acid sequence of SEQ ID No 85, and then anti-Allergin-1 bispecific antibody #15p_h_opt clone having the same heavy chain and light chain was generated (see Table 3).

**[Table 3]**

| Anti-Allergin-1 bispecific antibody clones | Used humanized scFv clones | | Heavy chain SEQ ID No. | Light chain SEQ ID No. |
|---|---|---|---|---|
| | Fab | scFv | | |
| #15_h_opt | Pro1571 | Pro2282 | 79 | 78 |
| #15_h_optv2 | Pro1571 | Pro2281 | 98 | 78 |
| #15p_h_opt | Pro2281 | Pro2285 | 84 | 85 |

As evaluated by flow cytometry, the binding activity (EC50 values) to Allergin-1L of the mutants produced here were all comparable to that of the original #15_h clone, as shown in Table 4.

**[Table 4]**

| Anti-Allergin-1 bispecific antibody clones | EC₅₀ |
|---|---|
| #15_h | 2.37 nM |
| #15_h_opt | 2.58 nM |
| #15_h_optv2 | 2.41 nM |
| #15p_h_opt | 1.49 nM |

### Example 9: Evaluation of the binding of anti-Allergin-1 bispecific antibody

Each binding site of the anti-Allergin-1 bispecific antibody produced in Example 8 was evaluated for its contribution to binding to Allergin-1L.

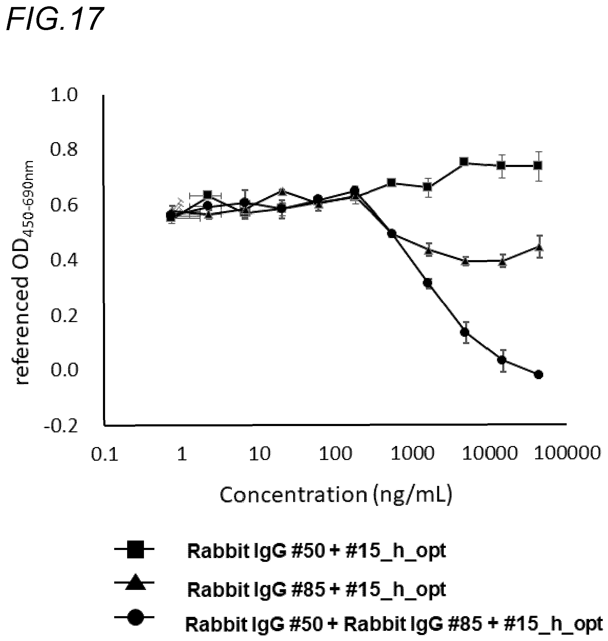

Initially, rabbit anti-Allergin-1S1 IgG antibody #85 clone as described in Example 4 having the same CDRs as those in a scFv of anti-Allergin-1 bispecific antibody #15_h_opt clone, rabbit anti-Allergin-1S1 IgG antibody #50 clone as described in Example 4 having the same CDRs as those in a Fab of anti-Allergin-1 bispecific antibody #15_h_opt clone, or both were added to CHO cells expressing human Allergin-1L, and which were incubated. After washing said CHO cells, to which 340 ng/mL of #15_h_opt clone was added, and which were incubated. After washing the CHO cells again, to which HRP-conjugated anti-human Fc antibody was added, and which were incubated. After further washing of the CHO cells, to which HRP chromogenic substrate was added, and which were incubated, and then of which absorbance was measured. The results are shown in Figure 17.

The binding of #15_h_opt clone to Allergin-1L was not completely inhibited by adding either #85 or #50 clone alone, but was completely inhibited by simultaneously adding both clones. Therefore, each binding site of anti-Allergin-1 bispecific antibody #15_h_opt clone was confirmed to contribute to binding to Allergin-1L.

### Example 10: Binding Characterization of anti-Allergin-1 bispecific antibody

Competitive binding assay was performed on the anti-Allergin-1 bispecific antibodies produced in Example 8 to evaluate their effects on the binding of ODN M362, known as TLR9 ligand, to Allergin-1.

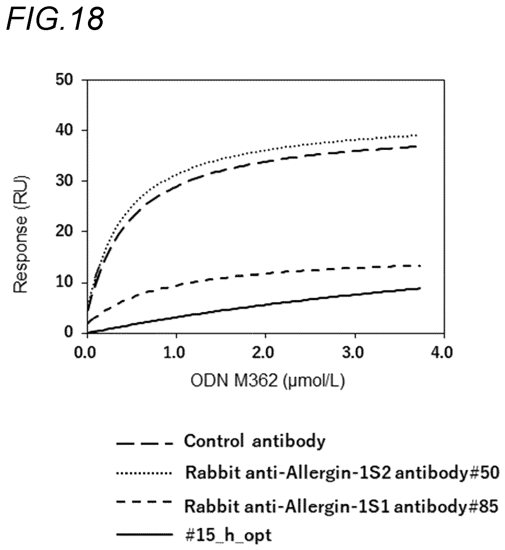

Initially, 6× His-tag fused human Allergin-1L protein was prepared by a method known to those skilled in the art and immobilized on Series S Sensor Chip CM5 (Cytiva, model number 29149603), using His capture Kit (Cytiva, model number 28995056). Anti-Allergin-1 bispecific antibody #15_h_opt clone, rabbit anti-Allergin-1S1 IgG antibody #85 clone and rabbit anti-Allergin-1S2 IgG antibody #50 clone were added to Series S Sensor Chip CM5, respectively, and then the binding of ODN M362 (InvivoGen, model number tlrl-m362-1) to immobilized 6×His-tag fused human Allergin-1L protein was evaluated by Biacore (Biacore T200, Cytiva). The results are shown in Figure 18.

#15_h_opt clone and #85 clone inhibited the binding to the 6×His-tag fused human Allergin-1L protein of ODN M362, but #15_h_opt clone was more strongly inhibited.

### Example 11: Evaluation of in vivo activity of anti-Allergin-1 bispecific antibody in tumor-bearing mice

Using methods known to those skilled in the art, in vivo activity of the anti-Allergin-1 bispecific antibody produced in Example 6 was evaluated in human Allergin-1L knock-in C57BL/6 mice, produced by replacing Allergin-1 gene with human Allergin-1L gene, bearing tumor.

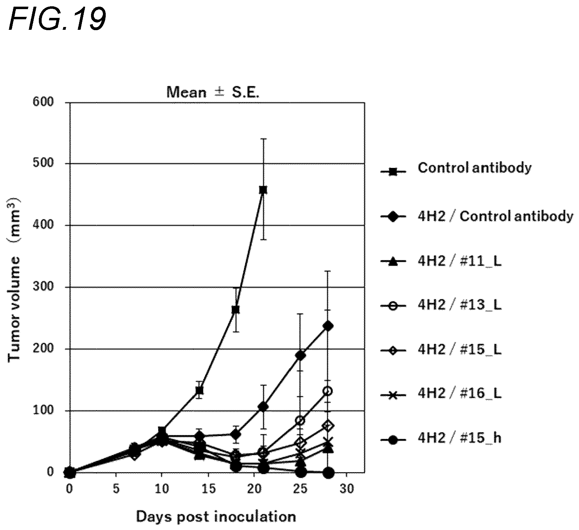

2 × 10⁵ of mouse colorectal cancer cell line MC38 were transplanted subcutaneously into right abdomen of human Allergin-1L knock-in C57BL/6 mice, and to which anti-mouse PD-1 antibody 4H2 (see WO2006/121168) was administered intraperitoneally on the day of transplantation, 6 days later, 12 days later, and 17 days later, respectively. On the other hand, anti-Allergin-1 bispecific antibodies (#11_L, #13_L, #15_L, #16_L and #15_h clones) were administered intraperitoneally on the day of transplantation, 4 days later, 7 days later, 10 days later, 14 days later, 18 days later and 21 days after transplantation, respectively, along with the anti-mouse PD-1 antibody 4H2 in the above schedule. Starting 7 days after transplantation, the long and short diameters of tumor were measured twice a week, and the tumor volume was calculated using the formula (long diameter) × (short diameter)² × 0.5. For individuals with multiple tumors, each tumor volume was calculated, and the sum thereof was used as the tumor volume for that individual. The results are shown in Figure 19.

All of the evaluated anti-Allergin-1 bispecific antibodies, when combined with anti-mouse PD-1 antibody 4H2, markedly enhanced the anti-tumor activity of 4H2 alone.

### Example 12: Evaluation of in vivo activity of anti-Allergin-1 bispecific antibody in tumor-bearing mice

*In vivo* activity of the anti-Allergin-1 bispecific antibody produced in Example 6 was evaluated in human Allergin-1L knock-in C57BL/6 mice bearing tumor of Example 11.

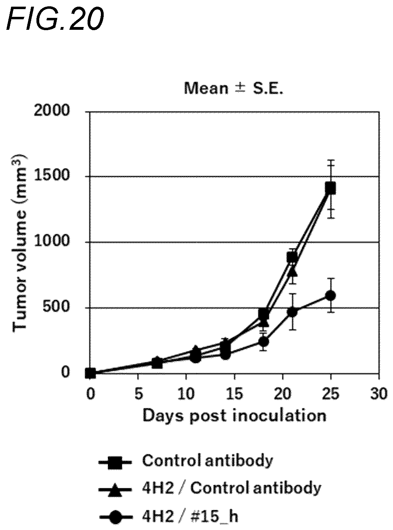

2 × 10⁵ of mouse lung cancer cell line 3LL were transplanted subcutaneously into right abdomen of human Allergin-1L knock-in C57BL/6 mice, and to which anti-mouse PD-1 antibody 4H2 was administered intraperitoneally on the day of transplantation, 6 days later, 12 days later, and 17 days later, respectively. On the other hand, anti-Allergin-1 bispecific antibody #15_h clone was administered intraperitoneally on the day of transplantation, 4 days later, 7 days later, 11 days later, 14 days later, 18 days later and 21 days after transplantation, respectively, along with the anti-mouse PD-1 antibody 4H2 in the above schedule. Starting 7 days after transplantation, the long and short diameters of tumor were measured twice a week, and the tumor volume was calculated using the formula (long diameter) × (short diameter)² × 0.5. For individuals with multiple tumors, each tumor volume was calculated, and the sum thereof was used as the tumor volume for that individual. The results are shown in Figure 20.

#15_h clone, in combination with anti-mouse PD-1 antibody 4H2, enhanced the anti-tumor effect that was hardly observed when 4H2 alone.

### Example 13: Evaluation of in vivo activity of anti-Allergin-1 bispecific antibody in tumor-bearing mice

*In vivo* activity of the anti-Allergin-1 bispecific antibody produced in Example 8 was evaluated in human Allergin-1L knock-in C57BL/6 mice bearing tumor of Example 11.

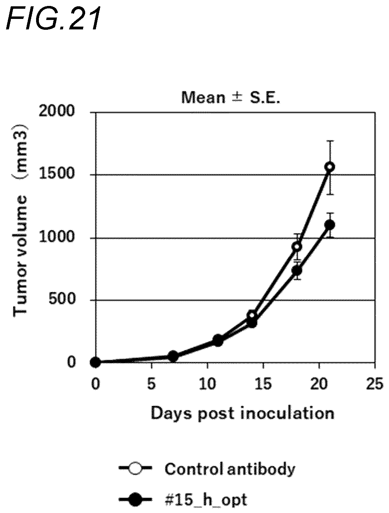

6 × 10⁴ of human PD-L1-forced expressing MC38 were transplanted subcutaneously into right abdomen of human Allergin-1L knock-in C57BL/6 mice, and to which anti-Allergin-1 bispecific antibody #15_h clone was administered intraperitoneally on the day of transplantation, 7 days later and 14 days later, respectively. Starting 7 days after transplantation, the long and short diameters of tumor were measured twice a week, and the tumor volume was calculated using the formula (long diameter) × (short diameter)² × 0.5. For individuals with multiple tumors, each tumor volume was calculated, and the sum thereof was used as the tumor volume for that individual. The results are shown in Figure 21.

The #15_h_opt clone showed the anti-tumor effect when administered alone.

### Example 14: Evaluation of in vivo activity of anti-Allergin-1 bispecific antibody on tumor-infiltrating CD45-positive cells in tumor-bearing mice

*In vivo* activity of the anti-Allergin-1 bispecific antibody produced in Example 6 and 8 on tumor-infiltrating CD45-positive cells was evaluated in human Allergin-1L knock-in C57BL/6 mice bearing tumor of Example 11.

2 × 10⁵ of mouse colorectal cancer cell line MC38 were transplanted subcutaneously into right abdomen of human Allergin-1L knock-in C57BL/6 mice, and to which the respective clones of anti-Allergin-1 bispecific antibodies, #15_h, #15_h_opt and #15p_h_opt were administered intraperitoneally on the day of transplantation, 4 days later and 7 days later, respectively, in combination with anti-mouse PD-1 antibody 4H2 (administered on the day of transplantation and 6 days after transplantation, respectively). Tumor tissues were removed 11 days after transplantation, and CD45-positive cells were purified. After mRNA was extracted from the purified cells and cDNA was synthesized by reverse transcription reaction, expression analysis of various genes was performed by quantitative PCR method. The results are shown in Table 5.

**[Table 5]**

| | 4H2 + #15_h | 4H2 + #15_h_opt | 4H2 + #15p_h_opt |
|---|---|---|---|
| *Gzmb* | 1.48 | 1.56 | 2.09 |
| *Prf1* | 1.19 | 1.89 | 2.71 |
| *Ifng* | 1.36 | 1.89 | 1.89 |
| *Ifna6* | 1.14 | 2.31 | 2.65 |

The numbers in the table represent the ratio of expression level to that of each gene in the anti-mouse PD-1 antibody 4H2 alone-administered group.

Each of the clones of anti-Allergin-1 bispecific antibody, when used in combination with anti-mouse PD-1 antibody 4H2, induced expression of anti-tumor immunity-related genes Gzmb, Prf1, Ifng, Ifnb1, Ifna2, Ifna4 and Ifna6.

### Example 15: Evaluation of in vivo activity of anti-Allergin-1 bispecific antibody on tumor-infiltrating CD45-positive cells in tumor-bearing mice

*In vivo* activity of the anti-Allergin-1 bispecific antibody produced in Example 8 on tumor-infiltrating CD45-positive cells was evaluated in human Allergin-1L knock-in C57BL/6 mice bearing tumor of Example 11.

6 × 10⁴ of human PD-L1-forced expressing MC38 were transplanted subcutaneously into right abdomen of human Allergin-1L knock-in C57BL/6 mice, and to which the anti-Allergin-1 bispecific antibody, #15_h_opt clone was administered intraperitoneally on the day of transplantation, 8 days later and 14 days later, respectively. Tumor tissues were removed 10 days and 15 days after transplantation, and CD45-positive cells were purified. After mRNA was extracted from the purified cells and cDNA was synthesized by reverse transcription reaction, expression analysis of various genes was performed by quantitative PCR method. The results are shown in Table 6.

**[Table 6]**

| | Day10 | Day15 |
|---|---|---|
| *Gzmb* | 2.2 | 1.81 |
| *Pre1* | 3.12 | 3.5 |
| *Ifng* | 3.53 | 1.64 |
| *Ifnb1* | 2.04 | 1 |
| *Ifna4* | 1.07 | 1.32 |

The numbers in the table represent the ratio of expression level to that of each gene in the control group.

#15_h_opt clone induced expression of anti-tumor immunity-related gene Gzmb, Prf1, Ifng, Ifnb1, and Ifna4 when administered alone.

### Example 16: Identification of epitopes for anti-Allergin-1 bispecific antibody #15_h_opt clone

6×His-tag fused human Allergin-1L (C38S) protein (but lacking amino acids at position 20 and 21 and amino acids at position 217 or after), Fv-clasp protein with the same CDRs as those of a scFv of #15_h_opt clone (S1-Fv-clasp) ( Structure, 2017, Vol. 25, No. 10, p. 1611-1622) and Fv-clasp protein with the same CDRs as those of a Fab of #15_h_opt clone (S2-Fv-clasp), respectively, and of which crystals of three-way complex were obtained. Crystals were frozen by FMS (free mounting system) method and X-ray diffraction data were obtained. The structure was refined using STARANISO-corrected data and determined at 2.62 Å resolution.

Based on the determined structure, it was found that the S1-A epitope is constituted by at least one or more, or all of phenylalanine at position 70, arginine at position 71, arginine at position 72, lysine at position 73, histidine at position 75, leucine at position 76, histidine at position 96, glutamic acid at position 97, lysine at position 102, lysine at position 104, and tyrosine at position 113 in Allergin-1 comprising the amino acid sequence set forth in SEQ ID No. 115, and that the S2-D epitope is constituted by at least one or more, or all of isoleucine at position 132, methionine at position 133, valine at position 134, isoleucine at position 135, glutamine at position 136, threonine at position 137, glutamic acid at position 138, 146th histidine at position 146 and proline at position 215 in said Allergin-1.

### Example 17: Evaluation of in vitro activity of anti-Allergin-1 bispecific antibody on cytokine release from human peripheral blood mononuclear cells

This experiment was performed on adding the anti-Allergin-1 bispecific antibody produced in Example 8 to human peripheral blood mononuclear cells ("human PBMCs").

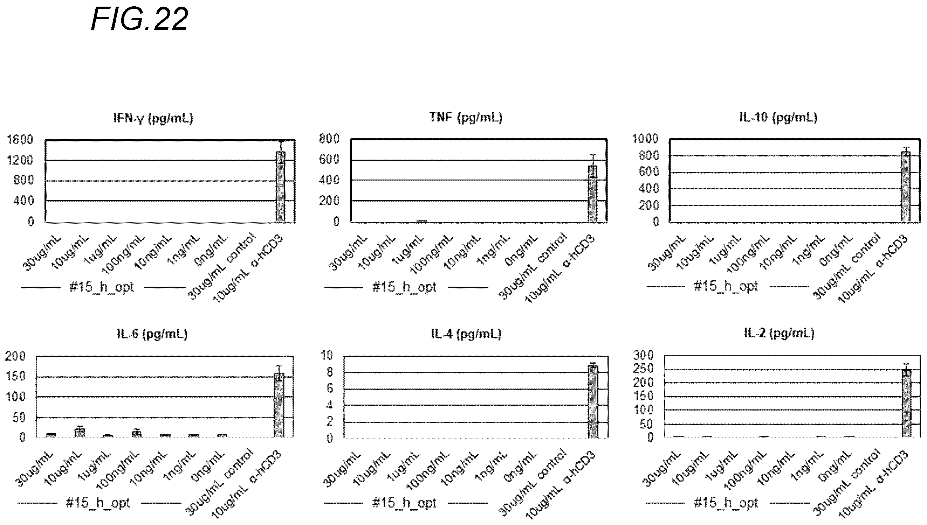

Anti-Allergin-1 bispecific antibody #15_h_opt clone at the final concentration of 1 ng/mL to 30 µg/mL and anti-human CD3 antibody as a positive control at that of 10 µg/mL were added to human PBMCs (Lonza, model number CC-2702), respectively, and which were incubated for 24 hours. IFN-γ, TNF, IL-10, IL-6, IL-4, and IL-2 in culture supernatant of 24 hours later were quantified by Cytometric Bead Array Human Th1/Th2 Cytokine Kit II (BD Biosciences, model number 551809), respectively. The results are shown in Figure 22. Cytokine production (pg/mL) in the figure is shown as mean ± standard error (N=3).

Anti-CD3 antibodies induced all cytokine production, whereas #15_h_opt clone did not significantly induce any cytokines.

### INDUSTRIAL AVAILABILITY

The anti-Allergin-1 bispecific antibody is useful for preventing, suppressing the progression of symptoms of, suppressing the recurrence of or treating cancer.

SEQUENCE LISTING

## Claims

1. A bispecific antibody to Allergin-1 or an antibody fragment thereof, comprising any two different antigen-binding sites selected from an S1-A binding site, an S1-B binding site, an S2-C binding site and an S2-D binding site,
wherein the S1-A binding site is any one selected from
(A) an antigen-binding site comprising
a VH having
(a) a VH-CDR1 comprising the amino acid sequence set forth in SEQ ID No. 25,
(b) a VH-CDR2 comprising the amino acid sequence set forth in SEQ ID No. 26, and
(c) a VH-CDR3 comprising the amino acid sequence set forth in SEQ ID No. 27, and a VL having
(d) a VL-CDR1 comprising the amino acid sequence set forth in SEQ ID No. 49,
(e) a VL-CDR2 comprising the amino acid sequence set forth in SEQ ID No. 50, and
(f) a VL-CDR3 comprising the amino acid sequence set forth in SEQ ID No. 51,
(B) an antigen-binding site comprising
a VH having
(a) a VH-CDR1 comprising the amino acid sequence set forth in SEQ ID No. 28,
(b) a VH-CDR2 comprising the amino acid sequence set forth in SEQ ID No. 29, and
(c) a VH-CDR3 comprising the amino acid sequence set forth in SEQ ID No. 30, and a VL having
(d) a VL-CDR1 comprising the amino acid sequence set forth in SEQ ID No. 52,
(e) a VL-CDR2 comprising the amino acid sequence set forth in SEQ ID No. 53, and
(f) a VL-CDR3 comprising the amino acid sequence set forth in SEQ ID No. 54,
(C) an antigen-binding site comprising
a VH having
(a) a VH-CDR1 comprising the amino acid sequence set forth in SEQ ID No. 31,
(b) a VH-CDR2 comprising the amino acid sequence set forth in SEQ ID No. 32, and
(c) a VH-CDR3 comprising the amino acid sequence set forth in SEQ ID No. 33, and a VL having
(d) a VL-CDR1 comprising the amino acid sequence set forth in SEQ ID No. 55,
(e) a VL-CDR2 comprising the amino acid sequence set forth in SEQ ID No. 56, and
(f) a VL-CDR3 comprising the amino acid sequence set forth in SEQ ID No. 57, and
(D) an antigen-binding site comprising
a VH having
(a) a VH-CDR1 comprising the amino acid sequence set forth in SEQ ID No. 34,
(b) a VH-CDR2 comprising the amino acid sequence set forth in SEQ ID No. 35, and
(c) a VH-CDR3 comprising the amino acid sequence set forth in SEQ ID No. 36, and a VL having
(d) a VL-CDR1 comprising the amino acid sequence set forth in SEQ ID No. 58,
(e) a VL-CDR2 comprising the amino acid sequence set forth in SEQ ID No. 59, and
(f) a VL-CDR3 comprising the amino acid sequence set forth in SEQ ID No. 60;
the S1-B binding site is
(E) an antigen-binding site comprising
a VH having
(a) a VH-CDR1 comprising the amino acid sequence set forth in SEQ ID No. 37,
(b) a VH-CDR2 comprising the amino acid sequence set forth in SEQ ID No. 38, and
(c) a VH-CDR3 comprising the amino acid sequence set forth in SEQ ID No. 39, and a VL having
(d) a VL-CDR1 comprising the amino acid sequence set forth in SEQ ID No. 61,
(e) a VL-CDR2 comprising the amino acid sequence set forth in SEQ ID No. 62, and
(f) a VL-CDR3 comprising the amino acid sequence set forth in SEQ ID No. 63, or
(F) an antigen-binding site comprising
a VH having
(a) a VH-CDR1 comprising the amino acid sequence set forth in SEQ ID No. 40,
(b) a VH-CDR2 comprising the amino acid sequence set forth in SEQ ID No. 41, and
(c) a VH-CDR3 comprising the amino acid sequence set forth in SEQ ID No. 42, and a VL having
(d) a VL-CDR1 comprising the amino acid sequence set forth in SEQ ID No. 64,
(e) a VL-CDR2 comprising the amino acid sequence set forth in SEQ ID No. 65, and
(f) a VL-CDR3 comprising the amino acid sequence set forth in SEQ ID No. 66;
the S2-C binding site is an antigen-binding site comprising
a VH having
(a) a VH-CDR1 comprising the amino acid sequence set forth in SEQ ID No. 43,
(b) a VH-CDR2 comprising the amino acid sequence set forth in SEQ ID No. 44, and
(c) a VH-CDR3 comprising the amino acid sequence set forth in SEQ ID No. 45, and a VL having
(d) a VL-CDR1 comprising the amino acid sequence set forth in SEQ ID No. 67,
(e) a VL-CDR2 comprising the amino acid sequence set forth in SEQ ID No. 68, and
(f) a VL-CDR3 comprising the amino acid sequence set forth in SEQ ID No. 69, and
the S2-D binding site is an antigen-binding site comprising
a VH having
(a) a VH-CDR1 comprising the amino acid sequence set forth in SEQ ID No. 46,
(b) a VH-CDR2 comprising the amino acid sequence set forth in SEQ ID No. 47, and
(c) a VH-CDR3 comprising the amino acid sequence set forth in SEQ ID No. 48, and a VL having
(d) a VL-CDR1 comprising the amino acid sequence set forth in SEQ ID No. 70,
(e) a VL-CDR2 comprising the amino acid sequence set forth in SEQ ID No. 71, and
(f) a VL-CDR3 comprising the amino acid sequence set forth in SEQ ID No. 72.

2. The bispecific antibody or antibody fragment thereof according to claim 1, wherein one to five arbitrary amino acid residues in any one or more CDRs of VH-CDR1, VH-CDR2, VH-CDR3, VL-CDR1, VL-CDR2 and VL-CDR3 constituting any two different antigen-binding sites selected from the S1-A binding site, the S1-B binding site, the S2-C binding site and the S2-D binding site, respectively, may be substituted with other amino acids, respectively.

3. The bispecific antibody or antibody fragment thereof according to claim 1, wherein the two different antigen-binding sites are the S1-A binding site and the S2-C binding site, wherein (i) the S1-A binding site is the antigen-binding site comprising
the VH having
(a) the VH-CDR1 comprising the amino acid sequence set forth in SEQ ID No. 25,
(b) the VH-CDR2 comprising the amino acid sequence set forth in SEQ ID No. 26, and
(c) the VH-CDR3 comprising the amino acid sequence set forth in SEQ ID No. 27, and
the VL having
(d) the VL-CDR1 comprising the amino acid sequence set forth in SEQ ID No. 49,
(e) the VL-CDR2 comprising the amino acid sequence set forth in SEQ ID No. 50, and
(f) the VL-CDR3 comprising the amino acid sequence set forth in SEQ ID No. 51, and
(ii) the S2-C binding site is the antigen-binding site comprising
the VH having
(a) the VH-CDR1 comprising the amino acid sequence set forth in SEQ ID No. 43,
(b) the VH-CDR2 comprising the amino acid sequence set forth in SEQ ID No. 44, and
(c) the VH-CDR3 comprising the amino acid sequence set forth in SEQ ID No. 45, and
the VL having
(d) the VL-CDR1 comprising the amino acid sequence set forth in SEQ ID No. 67,
(e) the VL-CDR2 comprising the amino acid sequence set forth in SEQ ID No. 68, and
(f) the VL-CDR3 comprising the amino acid sequence set forth in SEQ ID No. 69.

4. The bispecific antibody or antibody fragment thereof according to claim 1, wherein the two different antigen-binding sites are the S1-A binding site and the S2-C binding site, wherein (i) the S1-A binding site is the antigen-binding site comprising
the VH having
(a) the VH-CDR1 comprising the amino acid sequence set forth in SEQ ID No. 28,
(b) the VH-CDR2 comprising the amino acid sequence set forth in SEQ ID No. 29, and
(c) the VH-CDR3 comprising the amino acid sequence set forth in SEQ ID No. 30, and
the VL having
(d) the VL-CDR1 comprising the amino acid sequence set forth in SEQ ID No. 52,
(e) the VL-CDR2 comprising the amino acid sequence set forth in SEQ ID No. 53, and
(f) the VL-CDR3 comprising the amino acid sequence set forth in SEQ ID No. 54,
and
(ii) the S2-C binding site is the antigen-binding site comprising
the VH having
(a) the VH-CDR1 comprising the amino acid sequence set forth in SEQ ID No. 43,
(b) the VH-CDR2 comprising the amino acid sequence set forth in SEQ ID No. 44, and
(c) the VH-CDR3 comprising the amino acid sequence set forth in SEQ ID No. 45, and
the VL having
(d) the VL-CDR1 comprising the amino acid sequence set forth in SEQ ID No. 67,
(e) the VL-CDR2 comprising the amino acid sequence set forth in SEQ ID No. 68, and
(f) the VL-CDR3 comprising the amino acid sequence set forth in SEQ ID No. 69.

5. The bispecific antibody or antibody fragment thereof according to claim 1, wherein the two different antigen-binding sites are the S1-A binding site and the S2-C binding site, wherein (i) the S1-A binding site is the antigen-binding site comprising
the VH having
(a) the VH-CDR1 comprising the amino acid sequence set forth in SEQ ID No. 31,
(b) the VH-CDR2 comprising the amino acid sequence set forth in SEQ ID No. 32, and
(c) the VH-CDR3 comprising the amino acid sequence set forth in SEQ ID No. 33, and
the VL having:
(d) the VL-CDR1 comprising the amino acid sequence set forth in SEQ ID No. 55,
(e) the VL-CDR2 comprising the amino acid sequence set forth in SEQ ID No. 56, and
(f) the VL-CDR3 comprising the amino acid sequence set forth in SEQ ID No. 57,
and
(ii) the S2-C binding site is the antigen-binding site comprising
the VH having
(a) the VH-CDR1 comprising the amino acid sequence set forth in SEQ ID No. 43,
(b) the VH-CDR2 comprising the amino acid sequence set forth in SEQ ID No. 44, and
(c) the VH-CDR3 comprising the amino acid sequence set forth in SEQ ID No. 45, and
the VL having
(d) the VL-CDR1 comprising the amino acid sequence set forth in SEQ ID No. 67,
(e) the VL-CDR2 comprising the amino acid sequence set forth in SEQ ID No. 68, and
(f) the VL-CDR3 comprising the amino acid sequence set forth in SEQ ID No. 69.

6. The bispecific antibody or antibody fragment thereof according to claim 1, wherein the two different antigen-binding sites are the S1-A binding site and the S2-C binding site, wherein (i) the S1-A binding site is the antigen-binding site comprising
the VH having
(a) the VH-CDR1 comprising the amino acid sequence set forth in SEQ ID No. 34,
(b) the VH-CDR2 comprising the amino acid sequence set forth in SEQ ID No. 35, and
(c) the VH-CDR3 comprising the amino acid sequence set forth in SEQ ID No. 36, and
the VL having
(d) the VL-CDR1 comprising the amino acid sequence set forth in SEQ ID No. 58,
(e) the VL-CDR2 comprising the amino acid sequence set forth in SEQ ID No. 59, and
(f) the VL-CDR3 comprising the amino acid sequence set forth in SEQ ID No. 60,
and
(ii) the S2-C binding site is the antigen-binding site comprising
the VH having
(a) the VH-CDR1 comprising the amino acid sequence set forth in SEQ ID No. 43,
(b) the VH-CDR2 comprising the amino acid sequence set forth in SEQ ID No. 44, and
(c) the VH-CDR3 comprising the amino acid sequence set forth in SEQ ID No. 45, and
the VL having
(d) the VL-CDR1 comprising the amino acid sequence set forth in SEQ ID No. 67,
(e) the VL-CDR2 comprising the amino acid sequence set forth in SEQ ID No. 68, and
(f) the VL-CDR3 comprising the amino acid sequence set forth in SEQ ID No. 69.

7. The bispecific antibody or antibody fragment thereof according to claim 1, wherein the two different antigen-binding sites are the S1-A binding site and the S2-D binding site, wherein (i) the S1-A binding site is the antigen-binding site comprising
the VH having
(a) the VH-CDR1 comprising the amino acid sequence set forth in SEQ ID No. 25,
(b) the VH-CDR2 comprising the amino acid sequence set forth in SEQ ID No. 26, and
(c) the VH-CDR3 comprising the amino acid sequence set forth in SEQ ID No. 27, and
the VL having
(d) the VL-CDR1 comprising the amino acid sequence set forth in SEQ ID No. 49,
(e) the VL-CDR2 comprising the amino acid sequence set forth in SEQ ID No. 50, and
(f) the VL-CDR3 comprising the amino acid sequence set forth in SEQ ID No. 51,
and
(ii) the S2-D binding site is the antigen-binding site comprising
the VH having
(a) the VH-CDR1 comprising the amino acid sequence set forth in SEQ ID No. 46,
(b) the VH-CDR2 comprising the amino acid sequence set forth in SEQ ID No. 47, and
(c) the VH-CDR3 comprising the amino acid sequence set forth in SEQ ID No. 48, and
the VL having
(d) the VL-CDR1 comprising the amino acid sequence set forth in SEQ ID No. 70,
(e) the VL-CDR2 comprising the amino acid sequence set forth in SEQ ID No. 71, and
(f) the VL-CDR3 comprising the amino acid sequence set forth in SEQ ID No. 72.

8. The bispecific antibody or antibody fragment thereof according to claim 1, wherein the two different antigen-binding sites are the S1-A binding site and the S2-D binding site, wherein (i) the S1-A binding site is the antigen-binding site comprising
the VH having
(a) the VH-CDR1 comprising the amino acid sequence set forth in SEQ ID No. 28,
(b) the VH-CDR2 comprising the amino acid sequence set forth in SEQ ID No. 29, and
(c) the VH-CDR3 comprising the amino acid sequence set forth in SEQ ID No. 30, and
the VL having
(d) the VL-CDR1 comprising the amino acid sequence set forth in SEQ ID No. 52,
(e) the VL-CDR2 comprising the amino acid sequence set forth in SEQ ID No. 53, and
(f) the VL-CDR3 comprising the amino acid sequence set forth in SEQ ID No. 54,
and
(ii) the S2-D binding site is the antigen-binding site comprising
the VH having
(a) the VH-CDR1 comprising the amino acid sequence set forth in SEQ ID No. 46,
(b) the VH-CDR2 comprising the amino acid sequence set forth in SEQ ID No. 47, and
(c) the VH-CDR3 comprising the amino acid sequence set forth in SEQ ID No. 48, and
the VL having
(d) the VL-CDR1 comprising the amino acid sequence set forth in SEQ ID No. 70,
(e) the VL-CDR2 comprising the amino acid sequence set forth in SEQ ID No. 71, and
(f) the VL-CDR3 comprising the amino acid sequence set forth in SEQ ID No. 72.

9. The bispecific antibody or antibody fragment thereof according to claim 1, wherein the two different antigen-binding sites are the S1-A binding site and the S2-D binding site, wherein (i) the S1-A binding site is the antigen-binding site comprising
the VH having
(a) the VH-CDR1 comprising the amino acid sequence set forth in SEQ ID No. 31,
(b) the VH-CDR2 comprising the amino acid sequence set forth in SEQ ID No. 32, and
(c) the VH-CDR3 comprising the amino acid sequence set forth in SEQ ID No. 33, and
the VL having
(d) the VL-CDR1 comprising the amino acid sequence set forth in SEQ ID No. 55,
(e) the VL-CDR2 comprising the amino acid sequence set forth in SEQ ID No. 56, and
(f) the VL-CDR3 comprising the amino acid sequence set forth in SEQ ID No. 57,
and
(ii) the S2-D binding site is the antigen-binding site comprising
the VH having
(a) the VH-CDR1 comprising the amino acid sequence set forth in SEQ ID No. 46,
(b) the VH-CDR2 comprising the amino acid sequence set forth in SEQ ID No. 47, and
(c) the VH-CDR3 comprising the amino acid sequence set forth in SEQ ID No. 48, and
the VL having
(d) the VL-CDR1 comprising the amino acid sequence set forth in SEQ ID No. 70,
(e) the VL-CDR2 comprising the amino acid sequence set forth in SEQ ID No. 71, and
(f) the VL-CDR3 comprising the amino acid sequence set forth in SEQ ID No. 72.

10. The bispecific antibody or antibody fragment thereof according to claim 1, wherein the two different antigen-binding sites are the S1-A binding site and the S2-D binding site, wherein (i) the S1-A binding site is the antigen-binding site comprising
the VH having
(a) the VH-CDR1 comprising the amino acid sequence set forth in SEQ ID No. 34,
(b) the VH-CDR2 comprising the amino acid sequence set forth in SEQ ID No. 35, and
(c) the VH-CDR3 comprising the amino acid sequence set forth in SEQ ID No. 36, and
the VL having
(d) the VL-CDR1 comprising the amino acid sequence set forth in SEQ ID No. 58,
(e) the VL-CDR2 comprising the amino acid sequence set forth in SEQ ID No. 59, and
(f) the VL-CDR3 comprising the amino acid sequence set forth in SEQ ID No. 60,
and
(ii) the S2-D binding site is the antigen-binding site comprising
the VH having
(a) the VH-CDR1 comprising the amino acid sequence set forth in SEQ ID No. 46,
(b) the VH-CDR2 comprising the amino acid sequence set forth in SEQ ID No. 47, and
(c) the VH-CDR3 comprising the amino acid sequence set forth in SEQ ID No. 48, and
the VL having
(d) the VL-CDR1 comprising the amino acid sequence set forth in SEQ ID No. 70,
(e) the VL-CDR2 comprising the amino acid sequence set forth in SEQ ID No. 71, and
(f) the VL-CDR3 comprising the amino acid sequence set forth in SEQ ID No. 72.

11. The bispecific antibody or antibody fragment thereof according to any one of claims 1 to 10, wherein framework regions in the VHs constituting any two different antigen-binding sites selected from the S1-A binding site, the S1-B binding site, the S2-C binding site and the S2-D binding site, respectively, comprise the amino acid sequence encoded by human germ-line VH gene, IGHV3-66 or gene thereof with one or more somatic mutations and/or back mutations, and/or framework regions in the VLs constituting the same antigen-binding sites, respectively, comprise the amino acid sequence encoded by human germ-line VL gene, IGKV1-39 or gene thereof with one or more somatic mutations and/or back mutations.

12. The bispecific antibody or antibody fragment thereof according to any one of claims 1, 2, and 11, wherein the S1-A binding site is constituted by the VH and VL comprising any one pair of the amino acid sequences selected from
(a) SEQ ID No. 1 and SEQ ID No. 12 or 104,
(b) SEQ ID No. 2 and SEQ ID No. 13 or 105,
(c) SEQ ID No. 3 and SEQ ID No. 14 or 106,
(d) SEQ ID No. 4 and SEQ ID No. 15 or 107,
(e) SEQ ID No. 5 and SEQ ID No. 16 or 108, and
(f) SEQ ID No. 6 and SEQ ID No. 17 or 109, or a pair of amino acid sequences having at least 80%, 90%, 95%, 98% or 99% identity to the respective same amino acid sequences (excluding three CDRs, respectively), each independently.

13. The bispecific antibody or antibody fragment thereof according to any one of claims 1, 2, 11 and 12, wherein the S1-B binding site is constituted by the VH and VL comprising a pair of the amino acid sequences set forth in
(a) SEQ ID No. 7 and SEQ ID No. 18 or 110, or
(b) SEQ ID No. 8 and SEQ ID No. 19 or 111, or a pair of amino acid sequences having at least 80%, 90%, 95%, 98% or 99% identity to the respective same amino acid sequences (excluding three CDRs, respectively), each independently.

14. The bispecific antibody or antibody fragment thereof according to any one of claims 1, 2, and 11 to 13, wherein the S2-C binding site is constituted by the VH and VL comprising a pair of the amino acid sequences set forth in SEQ ID No. 9 and SEQ ID No. 20 or 112, or a pair of amino acid sequences having at least 80%, 90%, 95%, 98% or 99% identity to the respective same amino acid sequences (excluding three CDRs, respectively), each independently.

15. The bispecific antibody or antibody fragment thereof according to any one of claims 1, 2, and 11 to 14, wherein the S2-D binding site is constituted by the VH and VL comprising a pair of the amino acid sequences set forth in
(a) SEQ ID No. 10 and SEQ ID No. 21 or 113, or
(b) SEQ ID No. 11 and SEQ ID No. 22 or 114, or a pair of amino acid sequences having at least 80%, 90%, 95%, 98% or 99% identity to the respective same amino acid sequences (excluding three CDRs, respectively), each independently.

16. The bispecific antibody or antibody fragment thereof according to claim 1 or 11, wherein the S1-A binding site is constituted by the VH and VL comprising any one pair of the amino acid sequences selected from:
(a) SEQ ID No. 1 and SEQ ID No. 12 or 104,
(b) SEQ ID No. 2 and SEQ ID No. 13 or 105,
(c) SEQ ID No. 3 and SEQ ID No. 14 or 106,
(d) SEQ ID No. 4 and SEQ ID No. 15 or 107,
(e) SEQ ID No. 5 and SEQ ID No. 16 or 108, and
(f) SEQ ID No. 6 and SEQ ID No. 17 or 109.

17. The bispecific antibody or antibody fragment thereof according to any one of claims 1, 11, and 16, wherein the S1-B binding site is constituted by the VH and VL comprising a pair of the amino acid sequences set forth in
(a) SEQ ID No. 7 and SEQ ID No. 18 or 110, or
(b) SEQ ID No. 8 and SEQ ID No. 19 or 111.

18. The bispecific antibody or antibody fragment thereof according to any one of claims 1, 11, 16, and 17, wherein the S2-C binding site is constituted by the VH and VL comprising a pair of the amino acid sequences set forth in SEQ ID No. 9 and SEQ ID No. 20 or 112.

19. The bispecific antibody or antibody fragment thereof according to any one of claims 1, 11, and 16 to 18, wherein the S2-D binding site is constituted by the VH and VL comprising a pair of the amino acid sequences set forth in
(a) SEQ ID No. 10 and SEQ ID No. 21 or 113, or
(b) SEQ ID No. 11 and SEQ ID No. 22 or 114.

20. A bispecific antibody to Allergin-1 or an antibody fragment thereof, comprising any two different antigen-binding sites selected from an S1-A binding site, an S1-B binding site, an S2-C binding site and an S2-D binding site,
wherein (i) the S1-A binding site is constituted by a VH and VL comprising any one pair of the amino acid sequences selected from
(a) SEQ ID No. 1 and SEQ ID No. 12 or 104,
(b) SEQ ID No. 2 and SEQ ID No. 13 or 105,
(c) SEQ ID No. 3 and SEQ ID No. 14 or 106,
(d) SEQ ID No. 4 and SEQ ID No. 15 or 107,
(e) SEQ ID No. 5 and SEQ ID No. 16 or 108, and
(f) SEQ ID No. 6 and SEQ ID No. 17 or 109, or a pair of amino acid sequences having at least 80%, 90%, 95%, 98% or 99% identity to the respective same amino acid sequences (excluding three CDRs, respectively), each independently,
(ii) the S1-B binding site is constituted by a VH and VL comprising a pair of the amino acid sequences set forth in
(a) SEQ ID No. 7 and SEQ ID No. 18 or 110, or
(b) SEQ ID No. 8 and SEQ ID No. 19 or 111, or a pair of amino acid sequences having at least 80%, 90%, 95%, 98% or 99% identity to the respective same amino acid sequences (excluding three CDRs, respectively), each independently,
(iii) the S2-C binding site is constituted by a VH and VL comprising a pair of the amino acid sequences set forth in SEQ ID No. 9 and SEQ ID No. 20 or 112, or amino acid sequences having at least 80%, 90%, 95%, 98% or 99% identity to the respective same amino acid sequences (excluding three CDRs, respectively), each independently, and
(iv) the S2-D binding site is constituted by a VH and VL comprising a pair of the amino acid sequences set forth in
(a) SEQ ID No. 10 and SEQ ID No. 21 or 113, or
(b) SEQ ID No. 11 and SEQ ID No. 22 or 114, or a pair of amino acid sequences having at least 80%, 90%, 95%, 98% or 99% identity to the respective same amino acid sequences (excluding three CDRs, respectively), each independently.

21. The bispecific antibody or antibody fragment thereof according to claim 20,
wherein (i) the S1-A binding site is constituted by the VH and VL comprising any one pair of the amino acid sequences selected from
(a) SEQ ID No. 1 and SEQ ID No. 12 or 104,
(b) SEQ ID No. 2 and SEQ ID No. 13 or 105,
(c) SEQ ID No. 3 and SEQ ID No. 14 or 106,
(d) SEQ ID No. 4 and SEQ ID No. 15 or 107,
(e) SEQ ID No. 5 and SEQ ID No. 16 or 108, and
(f) SEQ ID No. 6 and SEQ ID No. 17 or 109,
(ii) the S1-B binding site is constituted by the VH and VL comprising a pair of the amino acid sequences set forth in
(a) SEQ ID No. 7 and SEQ ID No. 18 or 110, or
(b) SEQ ID No. 8 and SEQ ID No. 19 or 111,
(iii) the VH and VL constituting the S2-C binding site are constituted by the VH and VL comprising a pair of the amino acid sequences set forth in SEQ ID No. 9 and SEQ ID No. 20 or 112, and
(iv) the S2-D binding site is constituted by the VH and VL comprising a pair of the amino acid sequences set forth in
(a) SEQ ID No. 10 and SEQ ID No. 21 or 113, or
(b) SEQ ID No. 11 and SEQ ID No. 22 or 114.

22. The bispecific antibody or antibody fragment thereof according to any one of claims 1, 11, 20, and 21, wherein the two different antigen-binding sites are the S1-A binding site and the S2-C binding site, and wherein the S1-A binding site is constituted by the VH and VL comprising a pair of the amino acid sequences set forth in SEQ ID No. 1 and SEQ ID No. 12 or 104, and the S2-C binding site is constituted by the VH and VL comprising a pair of the amino acid sequences set forth in SEQ ID No. 9 and SEQ ID No. 20 or 112.

23. The bispecific antibody or antibody fragment thereof according to any one of claims 1, 11, 20, and 21, wherein the two different antigen-binding sites are the S1-A binding site and the S2-C binding site, and wherein the S1-A binding site is constituted by the VH and VL comprising a pair of the amino acid sequences set forth in SEQ ID No. 2 and SEQ ID No. 13 or 105, and the S2-C binding site is constituted by the VH and VL comprising a pair of the amino acid sequences set forth in SEQ ID No. 9 and SEQ ID No. 20 or 112.

24. The bispecific antibody or antibody fragment thereof according to any one of claims 1, 11, 20, and 21, wherein the two different antigen-binding sites are the S1-A binding site and the S2-C binding site, and wherein the S1-A binding site is constituted by the VH and VL comprising a pair of the amino acid sequences set forth in SEQ ID No. 3 and SEQ ID No. 14 or 106, and the S2-C binding site is constituted by the VH and VL comprising a pair of the amino acid sequences set forth in SEQ ID No. 9 and SEQ ID No. 20 or 112.

25. The bispecific antibody or antibody fragment thereof according to any one of claims 1, 11, 20, and 21, wherein the two different antigen-binding sites are the S1-A binding site and the S2-C binding site, and wherein the S1-A binding site is constituted by the VH and VL comprising a pair of the amino acid sequences set forth in SEQ ID No. 4 and SEQ ID No. 15 or 107, and the S2-C binding site is constituted by the VH and VL comprising a pair of the amino acid sequences set forth in SEQ ID No. 9 and SEQ ID No. 20 or 112.

26. The bispecific antibody or antibody fragment thereof according to any one of claims 1, 11, 20, and 21, wherein the two different antigen-binding sites are the S1-A binding site and the S2-C binding site, and wherein the S1-A binding site is constituted by the VH and VL comprising a pair of the amino acid sequences set forth in SEQ ID No. 5 and SEQ ID No. 16 or 108, and the S2-C binding site is constituted by the VH and VL comprising a pair of the amino acid sequences set forth in SEQ ID No. 9 and SEQ ID No. 20 or 112.

27. The bispecific antibody or antibody fragment thereof according to any one of claims 1, 11, 20, and 21, wherein the two different antigen-binding sites are the S1-A binding site and the S2-C binding site, and wherein the S1-A binding site is constituted by the VH and VL comprising a pair of the amino acid sequences set forth in SEQ ID No. 6 and SEQ ID No. 17 or 109, and the S2-C binding site is constituted by the VH and VL comprising a pair of the amino acid sequences set forth in SEQ ID No. 9 and SEQ ID No. 20 or 112.

28. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 1 and SEQ ID No. 12 or 104, and the S2-D binding site is constituted by the VH and VL comprising a pair of the amino acid sequences set forth in SEQ ID No. 10 and SEQ ID No. 21 or 113.

29. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 2 and SEQ ID No. 13 or 105, and the S2-D binding site is constituted by the VH and VL comprising a pair of the amino acid sequences set forth in SEQ ID No. 10 and SEQ ID No. 21 or 113.

30. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 3 and SEQ ID No. 14 or 106, and the S2-D binding site is constituted by the VH and VL comprising a pair of the amino acid sequences set forth in SEQ ID No. 10 and SEQ ID No. 21 or 113.

31. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 4 and SEQ ID No. 15 or 107, and the S2-D binding site is constituted by the VH and VL comprising a pair of the amino acid sequences set forth in SEQ ID No. 10 and SEQ ID No. 21 or 113.

32. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 5 and SEQ ID No. 16 or 108, and the S2-D binding site is constituted by the VH and VL comprising a pair of the amino acid sequences set forth in SEQ ID No. 10 and SEQ ID No. 21 or 113.

33. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 6 and SEQ ID No. 17 or 109, and the S2-D binding site is constituted by the VH and VL comprising a pair of the amino acid sequences set forth in SEQ ID No. 10 and SEQ ID No. 21 or 113.

34. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 1 and SEQ ID No. 12 or 104, and the S2-D binding site is constituted by the VH and VL comprising a pair of the amino acid sequences set forth in SEQ ID No. 11 and SEQ ID No. 22 or 114.

35. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 2 and SEQ ID No. 13 or 105, and the S2-D binding site is constituted by the VH and VL comprising a pair of the amino acid sequences set forth in SEQ ID No. 11 and SEQ ID No. 22 or 114.

36. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 3 and SEQ ID No. 14 or 106, and the S2-D binding site is constituted by the VH and VL comprising a pair of the amino acid sequences set forth in SEQ ID No. 11 and SEQ ID No. 22 or 114.

37. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 4 and SEQ ID No. 15 or 107, and the S2-D binding site is constituted by the VH and VL comprising a pair of the amino acid sequences set forth in SEQ ID No. 11 and SEQ ID No. 22 or 114.

38. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 5 and SEQ ID No. 16 or 108, and the S2-D binding site is constituted by the VH and VL comprising a pair of the amino acid sequences set forth in SEQ ID No. 11 and SEQ ID No. 22 or 114.

39. The bispecific antibody or antibody fragment thereof according to any one of claims 1, 11, 20, and 21, wherein the two different antigen-binding sites are the S1-A binding site and the S2-D binding site, and wherein the S1-A binding site is constituted by the VH and VL comprising a pair of the amino acid sequences set forth in SEQ ID No. 6 and SEQ ID No. 17 or 109, and the S2-D binding site is constituted by the VH and VL comprising a pair of the amino acid sequences set forth in SEQ ID No. 11 and SEQ ID No. 22 or 114.

40. A bispecific antibody or an antibody fragment thereof, having antigen-binding sites specifically binding to two different epitopes on extracellular domains of Allergin-1, respectively,
wherein the antigen-binding sites do not mutually compete for Allergin-1, and are any two different antigen-binding sites selected from an S1-A binding site, an S1-B binding site, an S2-C binding site and an S2-D binding site, and
wherein (i) the S1-A binding site can cross-compete for the binding to an S1 domain on Allergin-1 with an antibody or an antigen-binding site thereof having a VH and VL comprising any one pair of the amino acid sequences selected from
(a) SEQ ID No. 1 and SEQ ID No. 104,
(b) SEQ ID No. 2 and SEQ ID No. 105,
(c) SEQ ID No. 3 and SEQ ID No. 106,
(d) SEQ ID No. 4 and SEQ ID No. 107,
(e) SEQ ID No. 5 and SEQ ID No. 108, and
(f) SEQ ID No. 6 and SEQ ID No. 109,
(ii) the S1-B binding site can cross-compete for the binding to the S1 domain on Allergin-1 with an antibody or an antigen-binding site thereof having a VH and VL comprising a pair of the amino acid sequences set forth in
(a) SEQ ID No. 7 and SEQ ID No. 110, or
(b) SEQ ID No. 8 and SEQ ID No. 111,
(iii) the S2-C binding site can cross-compete for the binding to an S2 domain on Allergin-1 with an antibody or an antigen-binding site thereof having a VH and VL comprising a pair of the amino acid sequences set forth in SEQ ID No. 9 and SEQ ID No. 112, and
(iv) the S2-D binding site can cross-compete for the binding to the S2 domain on Allergin-1 with an antibody or an antigen-binding site thereof having a VH and VL comprising a pair of the amino acid sequences set forth in
(a) SEQ ID No. 10 and SEQ ID No. 113, or
(b) SEQ ID No. 11 and SEQ ID No. 114.

41. A bispecific antibody or an antibody fragment thereof, having antigen-binding sites specifically binding to two different epitopes on extracellular domains of Allergin-1, respectively,
wherein the antigen-binding sites do not mutually compete for Allergin-1, and are any two different antigen-binding sites selected from an S1-A binding site, an S1-B binding site, an S2-C binding site and an S2-D binding site, and
wherein (i) the S1-A binding site is an antigen-binding site of which the binding to an S1 domain on Allergin-1 is cross-competed by an antibody or an antigen-binding site thereof having a VH and VL comprising any one pair of the amino acid sequences selected from
(a) SEQ ID No. 1 and SEQ ID No. 104,
(b) SEQ ID No. 2 and SEQ ID No. 105,
(c) SEQ ID No. 3 and SEQ ID No. 106,
(d) SEQ ID No. 4 and SEQ ID No. 107,
(e) SEQ ID No. 5 and SEQ ID No. 108, and
(f) SEQ ID No. 6 and SEQ ID No. 109,
(ii) the S1-B binding site is an antigen-binding site of which the binding to the S1 domain on Allergin-1 is cross-competed by an antibody or an antigen-binding site thereof having a VH and VL comprising a pair of the amino acid sequences set forth in (a) SEQ ID No. 7 and SEQ ID No. 110, or (b) SEQ ID No. 8 and SEQ ID No. 111,
(iii) the S2-C binding site is an antigen-binding site of which the binding to an S2 domain on Allergin-1 is cross-competed by an antibody or an antigen-binding site thereof having a VH and VL comprising a pair of the amino acid sequences set forth in SEQ ID No. 9 and SEQ ID No. 112, and
(iv) the S2-D binding site is an antigen-binding site of which the binding to the S2 domain on Allergin-1 is cross-competed by an antibody or an antigen-binding site thereof having a VH and VL comprising a pair of the amino acid sequences set forth in (a) SEQ ID No. 10 and SEQ ID No. 113, or (b) SEQ ID No. 11 and SEQ ID No. 114.

42. The bispecific antibody or antibody fragment thereof according to any one of claims 1 to 41, of which one antigen-binding site forms a part of a heavy chain/light chain complex constituting the anti-Allergin-1 bispecific antibody.

43. The bispecific antibody or antibody fragment thereof according to claim 42, wherein the heavy chain/light chain complex is formed by any one selected from
(A) a combination of (a) a heavy chain having the VH constituting the S1-A binding site and (b) a light chain having the VL constituting the S1-A binding site,
(B) a combination of (a) a heavy chain having the VH constituting the S1-B binding site and (b) a light chain having the VL constituting the S1-B binding site,
(C) a combination of (a) a heavy chain having the VH constituting the S2-C binding site and (b) a light chain having the VL constituting the S2-C binding site, and
(D) a combination of (a) a heavy chain having the VH constituting the S2-D binding site and (b) a light chain having the VL constituting the S2-D binding site.

44. The bispecific antibody or antibody fragment thereof according to any one of claims 1 to 43, wherein another antigen-binding site is in a form of scFv comprising a VH and VL constituting it.

45. The bispecific antibody or antibody fragment thereof according to claim 44, wherein the scFv links to a C-terminus of the heavy chain or light chain.

46. The bispecific antibody or antibody fragment thereof according to claim 44, wherein the scFv links to a C-terminus of the heavy chain.

47. The bispecific antibody or antibody fragment thereof according to claim 44, wherein the scFv links to a C-terminus of the light chain.

48. The bispecific antibody or antibody fragment thereof according to claim 44 or 46, (i) having (a) the heavy chain of which the C-terminus is linked with the scFv and (b) the light chain, or (ii) consisting of two same complexes of heavy chain/light chain constituted by the heavy chain and light chain of the preceding item (i).

49. The bispecific antibody or antibody fragment thereof according to claim 44 or 47, (i) having (a) the heavy chain and (b) the light chain of which the C-terminus is linked with the scFv, or (ii) consisting of two same complexes of heavy chain/light chain constituted by the heavy chain and light chain of the preceding item (i).

50. The bispecific antibody or antibody fragment thereof according to any one of claims 44 to 49, wherein the antigen-binding site in the heavy chain/light chain complex is the S1-A binding site and the antigen-binding site in the form of scFv is any one selected from the S1-B binding site, the S2-C binding site and the S2-D binding site.

51. The bispecific antibody or antibody fragment thereof according to any one of claims 44 to 49, wherein the antigen-binding site in the heavy chain/light chain complex is the S2-C binding site and the antigen-binding site in the form of scFv is any one selected from the S1-A binding site, the S1-B binding site and the S2-D binding site.

52. The bispecific antibody or antibody fragment thereof according to any one of claims 44 to 49, wherein the antigen-binding site in the heavy chain/light chain complex is the S2-D binding site and the antigen-binding site in the form of scFv is any one selected from the S1-A binding site, the S1-B binding site and the S2-C binding site.

53. The bispecific antibody or antibody fragment thereof according to any one of claims 44 to 49, wherein the antigen-binding site in the heavy chain/light chain complex is the S1-A binding site and the antigen-binding site in the form of scFv is the S2-C binding site.

54. The bispecific antibody or antibody fragment thereof according to any one of claims 44 to 49, wherein the antigen-binding site in the heavy chain/light chain complex is the S1-A binding site and the antigen-binding site in the form of scFv is the S2-D binding site.

55. The bispecific antibody or antibody fragment thereof according to any one of claims 44 to 49, wherein the antigen-binding site in the heavy chain/light chain complex is the S2-C binding site and the antigen-binding site in the form of scFv is the S1-A binding site.

56. The bispecific antibody or antibody fragment thereof according to any one of claims 44 to 49, wherein the antigen-binding site in the heavy chain/light chain complex is the S2-D binding site and the antigen-binding site in the form of scFv is the S1-A binding site.

57. The bispecific antibody or antibody fragment thereof according to any one of claims 44 to 56, wherein the scFv links to the C-terminus of the heavy chain or light chain through a direct amide bond or a peptide linker.

58. The bispecific antibody or antibody fragment thereof according to claim 57, wherein an N-terminus of the VL constituting the scFv links to the C-terminus of the heavy chain or light chain through a direct amide bond or a peptide linker.

59. The bispecific antibody or antibody fragment thereof according to any one of claims 44 to 58, wherein the VH and VL constituting the scFv mutually links in order from the VL to the VH from the N-terminus thereof through a direct amide bond or a peptide linker.

60. The bispecific antibody or antibody fragment thereof according to claim 57 or 58, wherein the peptide linker for linking the scFv to the C-terminus of the heavy chain or light chain comprises the amino acid sequence set forth in SEQ ID No. 73.

61. The bispecific antibody or antibody fragment thereof according to claim 59, wherein the peptide linker for mutually linking the VH and VL constituting the scFv comprises the amino acid sequence set forth in SEQ ID No. 74.

62. A bispecific antibody to Allergin-1 or an antibody fragment thereof, comprising any two different antigen-binding sites selected from an S1-A binding site, an S1-B binding site, an S2-C binding site and an S2-D binding site,
(I) wherein the S1-A binding site is any one selected from
(A) an antigen-binding site comprising
a VH having
(a) a VH-CDR1 comprising the amino acid sequence set forth in SEQ ID No. 25,
(b) a VH-CDR2 comprising the amino acid sequence set forth in SEQ ID No. 26, and
(c) a VH-CDR3 comprising the amino acid sequence set forth in SEQ ID No. 27, and a VL having
(d) a VL-CDR1 comprising the amino acid sequence set forth in SEQ ID No. 49,
(e) a VL-CDR2 comprising the amino acid sequence set forth in SEQ ID No. 50, and
(f) a VL-CDR3 comprising the amino acid sequence set forth in SEQ ID No. 51,
(B) an antigen-binding site comprising
a VH having
(a) a VH-CDR1 comprising the amino acid sequence set forth in SEQ ID No. 28,
(b) a VH-CDR2 comprising the amino acid sequence set forth in SEQ ID No. 29, and
(c) a VH-CDR3 comprising the amino acid sequence set forth in SEQ ID No. 30, and a VL having
(d) a VL-CDR1 comprising the amino acid sequence set forth in SEQ ID No. 52,
(e) a VL-CDR2 comprising the amino acid sequence set forth in SEQ ID No. 53, and
(f) a VL-CDR3 comprising the amino acid sequence set forth in SEQ ID No. 54,
(C) an antigen-binding site comprising
a VH having
(a) a VH-CDR1 comprising the amino acid sequence set forth in SEQ ID No. 31,
(b) a VH-CDR2 comprising the amino acid sequence set forth in SEQ ID No. 32, and
(c) a VH-CDR3 comprising the amino acid sequence set forth in SEQ ID No. 33, and a VL having
(d) a VL-CDR1 comprising the amino acid sequence set forth in SEQ ID No. 55,
(e) a VL-CDR2 comprising the amino acid sequence set forth in SEQ ID No. 56, and
(f) a VL-CDR3 comprising the amino acid sequence set forth in SEQ ID No. 57, and
(D) an antigen-binding site comprising
a VH having
(a) a VH-CDR1 comprising the amino acid sequence set forth in SEQ ID No. 34,
(b) a VH-CDR2 comprising the amino acid sequence set forth in SEQ ID No. 35, and
(c) a VH-CDR3 comprising the amino acid sequence set forth in SEQ ID No. 36, and a VL having
(d) a VL-CDR1 comprising the amino acid sequence set forth in SEQ ID No. 58,
(e) a VL-CDR2 comprising the amino acid sequence set forth in SEQ ID No. 59, and
(f) a VL-CDR3 comprising the amino acid sequence set forth in SEQ ID No. 60;
the S2-C binding site is an antigen-binding site comprising
a VH having
(a) a VH-CDR1 comprising the amino acid sequence set forth in SEQ ID No. 43,
(b) a VH-CDR2 comprising the amino acid sequence set forth in SEQ ID No. 44, and
(c) a VH-CDR3 comprising the amino acid sequence set forth in SEQ ID No. 45, and a VL having
(d) a VL-CDR1 comprising the amino acid sequence set forth in SEQ ID No. 67,
(e) a VL-CDR2 comprising the amino acid sequence set forth in SEQ ID No. 68, and
(f) a VL-CDR3 comprising the amino acid sequence set forth in SEQ ID No. 69, and
the S2-D binding site is an antigen-binding site comprising
a VH having
(a) a VH-CDR1 comprising the amino acid sequence set forth in SEQ ID No. 46,
(b) a VH-CDR2 comprising the amino acid sequence set forth in SEQ ID No. 47, and
(c) a VH-CDR3 comprising the amino acid sequence set forth in SEQ ID No. 48, and a VL having
(d) a VL-CDR1 comprising the amino acid sequence set forth in SEQ ID No. 70,
(e) a VL-CDR2 comprising the amino acid sequence set forth in SEQ ID No. 71, and
(f) a VL-CDR3 comprising the amino acid sequence set forth in SEQ ID No. 72;
(II) wherein one of the two different antigen-binding sites is a part of a heavy chain/light chain complex constituting the bispecific antibody, and another is an antigen-binding site in a form of scFv, and wherein
(i) the antigen-binding site in the heavy chain/light chain complex is the S1-A binding site, and the antigen-binding site in the form of scFv is the S2-C binding site or the S2-D binding site,
(ii) the antigen-binding site in the heavy chain/light chain complex is the S2-C binding site, and the antigen-binding site in the form of scFv is the S1-A binding site, or
(iii) the antigen-binding site in the heavy chain/light chain complex is the S2-D binding site, and the antigen-binding site in the form of scFv is the S1-A binding site,
(III) wherein an N-terminus of the VL constituting the scFv links to a C-terminus of the heavy chain or light chain through a peptide linker, and
(IV) (i) having the heavy chain and light chain in the form described in the preceding items (I) to (III), or (ii) consisting of two same complexes of heavy chain/light chain constituted by the heavy chain and light chain in the form described in the preceding items (I) to (III).

63. The bispecific antibody or antibody fragment thereof according to claim 62, wherein the two different antigen-binding sites are any two selected from
(i) the S1-A binding site constituted by the VH and VL comprising any one pair of the amino acid sequences selected from
(a) SEQ ID No. 1 and 12,
(b) SEQ ID No. 2 and 13,
(c) SEQ ID No. 3 and SEQ ID No. 14 or 106,
(d) SEQ ID No. 4 and SEQ ID No. 15 or 107,
(e) SEQ ID No. 5 and 16, and
(f) SEQ ID No. 6 and SEQ ID No. 17 or 109,
(ii) the S2-C binding site constituted by the VH and VL comprising a pair of the amino acid sequences set forth in SEQ ID No. 9 and SEQ ID No. 20 or 112, and
(iii) the S2-D binding site constituted by the VH and VL comprising a pair of the amino acid sequences set forth in
(a) SEQ ID No. 10 and SEQ ID No. 21 or 113, or
(b) SEQ ID No. 11 and 22.

64. The bispecific antibody or antibody fragment thereof according to claim 62, wherein the two different antigen-binding sites are any two selected from
(i) the S1-A binding site constituted by the VH and VL comprising any one pair of the amino acid sequences selected from
(a) SEQ ID No. 3 and SEQ ID No. 14 or 106,
(b) SEQ ID No. 4 and SEQ ID No. 15 or 107,
(c) SEQ ID No. 5 and 16, and
(d) SEQ ID No. 6 and SEQ ID No. 17 or 109,
(ii) the S2-C binding site constituted by the VH and VL comprising a pair of the amino acid sequences set forth in SEQ ID No. 9 and SEQ ID No. 20 or 112, and
(iii) the S2-D binding site constituted by the VH and VL comprising a pair of the amino acid sequences set forth in
(a) SEQ ID No. 10 and SEQ ID No. 21 or 113, or
(b) SEQ ID No. 11 and 22.

65. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 3 and SEQ ID No. 14 or 106, and
(b) the S2-C binding site constituted by the VH and VL comprising a pair of the amino acid sequences set forth in SEQ ID No. 9 and SEQ ID No. 20 or 112.

66. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 6 and SEQ ID No. 17 or 109, and
(b) the S2-D binding site constituted by the VH and VL comprising a pair of the amino acid sequences set forth in SEQ ID No. 10 and SEQ ID No. 21 or 113.

67. The bispecific antibody or antibody fragment thereof according to claim 62, wherein the two different antigen-binding sites are
the S1-A binding site constituted by the VH and VL comprising any one pair of the amino acid sequences selected from
(a) SEQ ID No. 3 and SEQ ID No. 14 or 106,
(b) SEQ ID No. 4 and SEQ ID No. 15 or 107, and
(c) SEQ ID No. 5 and 16, and
the S2-D binding site constituted by the VH and VL comprising a pair of the amino acid sequences set forth in
(d) SEQ ID No. 10 and SEQ ID No. 21 or 113, or
(e) SEQ ID No. 11 and 22.

68. The bispecific antibody or antibody fragment thereof according to any one of claims 63 to 67,
wherein (a) (i) if the S1-A binding site constitutes a scFv, the VL constituting the scFv comprises the amino acid sequence selected from SEQ ID No. 12 to 17, and
(ii) if the S1-A binding site constitutes a part of a heavy chain/light chain complex, the VL constituting the light chain comprises the amino acid sequence selected from SEQ ID No. 106, 107 and 109,
(b) (i) if the S2-C binding site constitutes a scFv, the VL constituting the scFv comprises the amino acid sequence set forth in SEQ ID No. 20, and
(ii) if the S2-C binding site constitutes a part of a heavy chain/light chain complex, the VL constituting the light chain comprises the amino acid sequence set forth in SEQ ID No. 112, and
(c) (i) if the S2-D binding site constitutes a scFv, the VL constituting the scFv comprises the amino acid sequence set forth in SEQ ID No. 21 or 22, and
(ii) if the S2-D binding site constitutes a part of a heavy chain/light chain complex, the VL constituting the light chain comprises the amino acid sequence set forth in SEQ ID No. 113.

69. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 3 and 14, and
(b) the S2-C binding site constituted by the VH and VL comprising a pair of the amino acid sequences set forth in SEQ ID No. 9 and 112.

70. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 3 and 106, and
(b) the S2-C binding site constituted by the VH and VL comprising a pair of the amino acid sequences set forth in SEQ ID No. 9 and 20.

71. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 6 and 17, and
(b) the S2-D binding site constituted by the VH and VL comprising a pair of the amino acid sequences set forth in SEQ ID No. 10 and 113.

72. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 6 and 109, and
(b) the S2-D binding site constituted by the VH and VL comprising a pair of the amino acid sequences set forth in SEQ ID No. 10 and 21.

73. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 3 and 14, and
(b) the S2-D binding site constituted by the VH and VL comprising a pair of the amino acid sequences set forth in SEQ ID No. 10 and 113.

74. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 4 and 15, and
(b) the S2-D binding site constituted by the VH and VL comprising a pair of the amino acid sequences set forth in SEQ ID No. 10 and 113.

75. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 5 and 16, and
(b) the S2-D binding site constituted by the VH and VL comprising a pair of the amino acid sequences set forth in SEQ ID No. 10 and 113.

76. The bispecific antibody or antibody fragment thereof according to claim 62, wherein the two different antigen-binding sites are
(a) the S1-A binding site constituted by the VH and VL comprising a pair of the amino acid sequences set forth in SEQ ID No. 3 and 106, and
(b) the S2-D binding site constituted by the VH and VL comprising a pair of the amino acid sequences set forth in SEQ ID No. 10 and 21.

77. The bispecific antibody or antibody fragment thereof according to claim 62 wherein the two different antigen-binding sites are
(a) the S1-A binding site constituted by the VH and VL comprising a pair of the amino acid sequences set forth in SEQ ID No. 4 and 107, and
(b) the S2-D binding site constituted by the VH and VL comprising a pair of the amino acid sequences set forth in SEQ ID No. 11 and 22.

78. The bispecific antibody or antibody fragment thereof according to any one of claims 62 to 77, wherein the VH and VL constituting the scFv are mutually linked in order from the VL to the VH from the N-terminus thereof through a peptide linker comprising the amino acid sequence set forth in SEQ ID No. 74.

79. The bispecific antibody or antibody fragment thereof according to any one of claims 62 to 78, wherein the scFv is linked to the C-terminus of the heavy chain through a peptide linker comprising the amino acid sequence set forth in SEQ ID No. 73.

80. The bispecific antibody or antibody fragment thereof according to any one of claims 1 to 79, wherein an epitope to which the S1-A binding site specifically binds is constituted by at least any one or more, or all of phenylalanine at position 70, arginine at position 71, arginine at position 72, lysine at position 73, histidine at position 75, leucine at position 76, histidine at position 96, glutamic acid at position 97, lysine at position 102, lysine at position 104 and tyrosine at position 113 in Allergin-1 comprising the amino acid sequence set forth in SEQ ID No. 115,
wherein an epitope to which the S2-D binding site specifically binds is constituted by at least any one or more, or all of isoleucine at position 132, methionine at position 133, valine at position 134, isoleucine at position 135, glutamine at position 136, threonine at position 137, glutamic acid at position 138, histidine at position 146 and proline at position 215 in said Allergin-1,
wherein an epitope to which the S1-B binding site specifically binds is an epitope different from the epitope to which the S1-A binding site specifically binds, represented by at least the preceding amino acids, and
wherein an epitope to which the S2-C binding site specifically binds is an epitope different from the epitope to which the S2-D binding site specifically binds, represented by at least the preceding amino acids.

81. A bispecific antibody to Allergin-1 or an antibody fragment thereof, comprising any two different antigen-binding sites selected from an S1-A binding site, an S1-B binding site, an S2-C binding site and an S2-D binding site, wherein
(a) an epitope to which the S1-A binding site specifically binds (an S1-A epitope) is constituted by at least any one or more, or all of phenylalanine at position 70, arginine at position 71, arginine at position 72, lysine at position 73, histidine at position 75, leucine at position 76, histidine at position 96, glutamic acid at position 97, lysine at position 102, lysine at position 104 and tyrosine at position 113 in Allergin-1 comprising the amino acid sequence set forth in SEQ ID No.115,
(b) an epitope to which the S2-D binding site specifically binds (an S2-D epitope) is constituted by at least any one or more, or all of isoleucine at position 132, methionine at position 133, valine at position 134, isoleucine at position 135, glutamine at position 136, threonine at position 137, glutamic acid at position 138, histidine at position 146 and proline at position 215 in the same Allergin-1,
(c) an epitope to which the S1-B binding site specifically bind is an epitope different from the S1-A epitope represented by at least the preceding amino acids, and
(d) an epitope to which the S2-C binding site specifically binds is an epitope different from the S2-D epitope represented by at least the preceding amino acids.

82. The bispecific antibody or antibody fragment thereof according to any one of claims 42 to 81, wherein both of the heavy chain and light chain are derived from an IgG antibody.

83. The bispecific antibody or antibody fragment thereof according to claim 82, wherein the IgG antibody is an IgG₁ antibody.

84. The bispecific antibody or antibody fragment thereof according to claim 83, wherein lysines at position 447 according to the EU numbering system in two heavy chain constant regions of the IgG₁ antibody are deleted, respectively.

85. The bispecific antibody or antibody fragment thereof according to any one of claims 42 to 84, wherein a heavy chain constant region of the heavy chain comprises the amino acid sequence set forth in SEQ ID No. 23 or 99.

86. The bispecific antibody or antibody fragment thereof according to any one of claims 42 to 85, wherein a light chain constant region of the light chain comprises the amino acid sequence set forth in SEQ ID No. 24.

87. The bispecific antibody or antibody fragment thereof according to any one of claims 42 to 83, wherein a heavy chain constant region of the heavy chain and light chain constant region of the light chain comprise the amino acid sequences set forth in SEQ ID No. 23 or 99, and SEQ ID No. 24, respectively.

88. A bispecific antibody to Allergin-1, (i) having a heavy chain and light chain comprising any one pair of the amino acid sequences selected from
(a) SEQ ID No. 75 and 76,
(b) SEQ ID No. 77 and 78,
(c) SEQ ID No. 79 and 78,
(d) SEQ ID No. 80 and 78,
(e) SEQ ID No. 81 and 82,
(f) SEQ ID No. 83 and 82,
(g) SEQ ID No. 84 and 85,
(h) SEQ ID No. 86 and 87,
(i) SEQ ID No. 88 and 89,
(j) SEQ ID No. 90 and 91,
(k) SEQ ID No. 90 and 92,
(l) SEQ ID No. 93 and 94,
(m) SEQ ID No. 93 and 95,
(n) SEQ ID No. 96 and 97,
(o) SEQ ID No. 98 and 78,
(p) SEQ ID No. 100 and 78,
(q) SEQ ID No. 101 and 78,
(r) SEQ ID No. 102 and 78, and
(s) SEQ ID No. 103 and 85, or
(ii) consisting of two same complexes of heavy chain/light chain constituted by the heavy chain and light chain of the preceding item (i).

89. A bispecific antibody to Allergin-1, (i) having a heavy chain and light chain comprising any one pair of the amino acid sequences selected from
(a) SEQ ID No. 77 and 78,
(b) SEQ ID No. 79 and 78,
(c) SEQ ID No. 83 and 82,
(d) SEQ ID No. 84 and 85,
(e) SEQ ID No. 98 and 78,
(f) SEQ ID No. 100 and 78,
(g) SEQ ID No. 101 and 78,
(h) SEQ ID No. 102 and 78, and
(j) SEQ ID No. 103 and 85, or
(ii) consisting of two same complexes of heavy chain/light chain constituted by the heavy chain and light chain of the preceding item (i).

90. The bispecific antibody to Allergin-1 according to claim 88 or 89, wherein alanine at position 299 in SEQ ID No. 75, 81, 83, 86, 88, 93 and 96 and at position 301 in SEQ ID No. 80 and 90 (corresponding to position 297 according to the EU numbering system in a constant region of the heavy chain) is substituted by asparagine, each independently.

91. The bispecific antibody or antibody fragment thereof according to any one of claims 1 to 90, wherein cytokine production is sufficiently reduced during administration thereof or within 24 hours after administration thereof.

92. The bispecific antibody or antibody fragment thereof according to claim 91, wherein the cytokine includes at least one or more selected from IL-2, IL-4, IL-6, IL-10, IFN-γ and TNF-α.

93. The bispecific antibody or antibody fragment thereof according to any one of claims 1 to 92, wherein Allergin-1 is human Allergin-1.

94. The bispecific antibody or antibody fragment thereof according to claim 93, wherein human Allergin-1 is/are one or more selected from human Allergin-1L, human Allergin-1S1 and human Allergin-1S2.

95. A pharmaceutical composition containing the anti-Allergin-1 bispecific antibody or the antibody fragment thereof according to any one of claims 1 to 94, as an active ingredient.

96. The pharmaceutical composition according to claim 95, further containing at least a pharmaceutically acceptable carrier.

97. An agent for suppressing a progression of symptoms of, suppressing a recurrence of, and/or treating cancer, containing the anti-Allergin-1 bispecific antibody or the antibody fragment thereof of any one selected from claims 1 to 94, as an active ingredient.

98. The agent according to claim 97, wherein the cancer is solid cancer or hematological cancer.

99. The agent according to claim 98, wherein the cancer is solid cancer, and wherein the solid cancer is one or more selected from malignant melanoma, non-small cell lung cancer, small cell lung cancer, head and neck cancer, renal cell cancer, breast cancer, ovarian cancer, anal cancer, colorectal cancer, rectal cancer, colon cancer, hepatocellular carcinoma, esophageal cancer, gastric cancer, esophagogastric junction cancer, pancreatic cancer, urine urothelial cancer, prostate cancer, fallopian tube cancer, primary peritoneal cancer, malignant pleural mesothelium tumor, gallbladder cancer, bile duct cancer, biliary tract cancer, skin cancer, testicular cancer, vagina cancer, vulvar cancer, penile cancer, small intestine cancer, endocrine system cancer, thyroid cancer, parathyroid cancer, adrenal cancer, spinal tumor, neuroblastoma, medulloblastoma, retinoblastoma, neuroendocrine tumor, brain tumor and squamous cell carcinomas.

100. The agent according to any one of claims 97 to 99, which is further prescribed in combination with other antineoplastic agents.

101. The agent according to claim 100, wherein other antineoplastic agent is a tumor immunotherapeutic drug, wherein the tumor immunotherapeutic drug is an anti-PD-1 antibody, an anti-PD-L1 antibody, or an anti-CTLA-4 antibody.

102. The agent according to claim 101, wherein the anti-PD-1 antibody is an antibody selected from Nivolumab, Cemiplimab-rwlc, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, Geptanolimab, AMP-514, STI-A1110, ENUM 388D4, ENUM 244C8, Zimberelimab, CS1003, BAT-1306, Penpulimab, AK103, Ezabenlimab, LZM009, CMAB819, Sym021, SSI-361, JY034, Pucotenlimab, ISU106 and CX-188.

103. The agent according to claim 101, wherein the anti-PD-L1 antibody is an antibody selected from Atezolizumab, Avelumab, Durvalumab, Manelimab, Pacmilimab, Envafolimab, Cosibelimab, Sugemalimab, BMS-936559, STI-1014, HLX20, Adebrelimab, MSB2311, BGB-A333, KL-A167, AK106, Socazolimab, FAZ053, CBT-502 and JS003.

104. The agent according to claim 101, wherein the anti-CTLA-4 antibody is an antibody selected from Ipilimumab, Zalifrelimab, Nurulimab and Tremelimumab.
